# EUROPEAN PATENT APPLICATION

(11) **EP 4 644 392 A1**
(43) Date of publication of application: **05.11.2025**
(21) Application number: 23910627.1
(22) Date of filing: 26.12.2023
(51) Int. Cl.: C07D 487/04, A61K 31/53, A61P 29/00

(54) **PYRIDAZINE FUSED ARYL RING COMPOUND AND USE THEREOF**

(30) Priority: 27.12.2022 CN 202211682289; 07.04.2023 CN 202310369110; 18.09.2023 CN 202311205493; 19.12.2023 CN 202311744437
(71) Applicant: Chia Tai Tianqing Pharmaceutical Group Co., Ltd., Lianyungang, Jiangsu 222062 (CN)
(72) Inventor: AO, Wangwei, Lianyungang, Jiangsu 222062 (CN); LI, Yuan, Lianyungang, Jiangsu 222062 (CN); WANG, Hui, Lianyungang, Jiangsu 222062 (CN); ZHU, Wen, Lianyungang, Jiangsu 222062 (CN); WU, Jie, Lianyungang, Jiangsu 222062 (CN); ZHANG, Yinsheng, Lianyungang, Jiangsu 222062 (CN)
(74) Representative: Grünecker Patent- und Rechtsanwälte PartG mbB
(86) International application number: PCT/CN2023/142013
(87) International publication number: WO 2024/140704

(57) **Abstract**

The present disclosure relates to the technical field of medicines, and relates to a pyridazine fused aryl ring compound and a use thereof, and in particular, to a compound of formula (I), an isomer thereof, a pharmaceutically acceptable salt thereof, and a use thereof. The compound in the present disclosure is used as an NLRP3 inflammasome inhibitor having a brand-new structure, and has a good inhibition effect on signal transduction induced by an NLRP3 inflammasome.

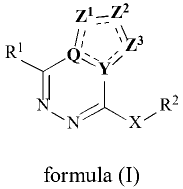

## Description

### CROSS-REFERENCE TO RELATED APPLICATIONS

The present disclosure claims the priority to and benefits of the Chinese Patent Application No. 202211682289.0 filed with the China National Intellectual Property Administration on December 27, 2022, the Chinese Patent Application No. 202310369110.4 filed with the China National Intellectual Property Administration on April 7, 2023, and the Chinese Patent Application No. 202311205493.8 filed with the China National Intellectual Property Administration on September 18, 2023, the contents of which are incorporated herein by reference in their entireties.

### TECHNICAL FIELD

The present disclosure pertains to the technical field of medicines, and relates to a pyridazine fused aromatic ring compound and use thereof, and particularly, to a compound of formula (I), an isomer thereof, a pharmaceutically acceptable salt thereof, and use thereof.

### BACKGROUND

Inflammasomes, as a component of the innate immune system, play a crucial role in immune regulation. It is known that intracellular NOD-like receptors and AIM2-like receptors can assemble into inflammasomes with structural commonalities. Among these, the NLRP3 inflammasome is currently the most well-characterized type of inflammasome. The NLRP3 inflammasome is a multi-protein macromolecular complex consisting of the NLRP3 protein, the adaptor protein ASC, and the effector caspase-1, and is usually distributed in macrophages, dendritic cells, microglia, and endothelial cells. The content of the NLRP3 protein in normal cells is relatively low. When cells are stimulated by pathogen-associated molecular patterns (PAMPs) or danger-associated molecular patterns (DAMPs), the gene expression of NLRP3 and proinflammatory factors is up-regulated through the NF-κB signaling pathway. Then, the NLRP3 protein undergoes oligomerization, followed by recruitment of pro-caspase-1 by ASC, thereby assembling the NLRP3 inflammasome. Caspase-1 is converted from a precursor form to an active form, which promotes the release of inflammatory factors while simultaneously inducing inflammatory pyroptosis.

The activation of the NLRP3 inflammasome is accompanied by severe inflammatory responses and is involved in the pathology of various diseases, such as inflammasome-related diseases/disorders or inflammatory diseases. Therefore, the rational design and synthesis of compounds with NLRP3 inflammasome inhibitory activity hold important value for the treatment of inflammatory diseases.

### SUMMARY

The present disclosure provides a compound of formula (1) or a pharmaceutically acceptable salt thereof: 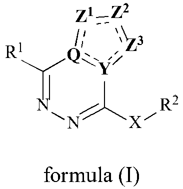 wherein
the structural unit 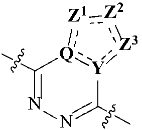 comprises fused bicyclic heteroaryl;
Q and Y are each independently selected from the group consisting of C, Si, and N, and one of Q and Y is N;
Z¹, Z², and Z³ are each independently selected from the group consisting of C(R³), Si(R³), and N;
X is selected from the group consisting of -N(R⁴)-(C(R⁵)(R⁶))ₘ-, -O-(C(R⁵)(R⁶))ₘ-, -S-(C(R⁵)(R⁶))ₘ-, and -(C(R⁵)(R⁶))ₘ-
R¹ is selected from the group consisting of C₃₋₈ cycloalkyl, 3- to 8-membered heterocycloalkyl, C₆₋₁₀ aryl, and 5-to 9-membered heteroaryl, the C₃₋₈ cycloalkyl, 3- to 8-membered heterocycloalkyl, C₆₋₁₀ aryl, or 5- to 9-membered heteroaryl being optionally and independently substituted with 1, 2, or 3 R^{a};
R² is selected from the group consisting of C₁₋₆ alkyl, C₂₋₆ alkenyl, C₂₋₆ alkynyl, -(CH₂)ᵢC₃₋₈ cycloalkyl, -(CH₂)ᵢ-(3- to 8-membered heterocycloalkyl), -(CH₂)ᵢ-(C₆₋₁₀ aryl), and -(CH₂)ᵢ-(5- to 9-membered heteroaryl), the C₁₋₆ alkyl, C₂₋₆ alkenyl, or C₂₋₆ alkynyl being optionally and independently substituted with 1, 2, or 3 halogens, OH, NH₂, or CN, and the C₃₋₈ cycloalkyl, 3- to 8-membered heterocycloalkyl, C₆₋₁₀ aryl, or 5- to 9-membered heteroaryl being optionally and independently substituted with 1, 2, or 3 R^{b};
each R³ is independently selected from the group consisting of H, OH, CN, NH₂, halogen, C₁₋₃ alkyl, C₁₋₃ alkoxy, C₁₋₃ alkylthio, -NH(C₁₋₃ alkyl), -N(C₁₋₃ alkyl)₂, C₃₋₆ cycloalkyl, and 3- to 6-membered heterocycloalkyl, the C₁₋₃ alkyl, C₁₋₃ alkoxy, C₁₋₃ alkylthio, -NH(C₁₋₃ alkyl), or -N(C₁₋₃ alkyl)₂ being optionally and independently substituted with 1, 2, or 3 halogens, OH, NH₂, or CN, and the C₃₋₆ cycloalkyl or 3- to 6-membered heterocycloalkyl being optionally and independently substituted with 1, 2, or 3 R^{c};
or, Z¹ and Z² are each independently selected from the group consisting of C(R³) and Si(R³), and the R³ groups on Z¹ and Z², together with the atoms to which they are attached, form C₃₋₈ partially saturated cycloalkyl, 3- to 8-membered partially saturated heterocycloalkyl, C₆₋₁₀ aryl, or 5- to 9-membered heteroaryl, the C₃₋₈ partially saturated cycloalkyl, 3- to 8-membered partially saturated heterocycloalkyl, C₆₋₁₀ aryl, or 5- to 9-membered heteroaryl being optionally and independently substituted with 1, 2, or 3 R^{a};
or, Z² and Z³ are each independently selected from the group consisting of C(R³) and Si(R³), and the R³ groups on Z² and Z³, together with the atoms to which they are attached, form C₃₋₈ partially saturated cycloalkyl, 3- to 8-membered partially saturated heterocycloalkyl, C₆₋₁₀ aryl, or 5- to 9-membered heteroaryl, the C₃₋₈ partially saturated cycloalkyl, 3- to 8-membered partially saturated heterocycloalkyl, C₆₋₁₀ aryl, or 5- to 9-membered heteroaryl being optionally and independently substituted with 1, 2, or 3 R^{a};
R⁴ is selected from the group consisting of H, C₁₋₃ alkyl, halogenated C₁₋₃ alkyl, C₃₋₆ cycloalkyl, and 3- to 6-membered heterocycloalkyl, wherein the C₃₋₆ cycloalkyl or 3- to 6-membered heterocycloalkyl may be optionally and independently substituted with 1, 2, or 3 halogens, OH, NH₂, or CN;
R⁵ and R⁶ are each independently selected from the group consisting of H, OH, NH₂, CN, C₁₋₃ alkyl, and halogenated C₁₋₃ alkyl;
each R^{a} is independently selected from the group consisting of OH, CN, NH₂, halogen, =O, C₁₋₃ alkyl, C₁₋₃ alkoxy, C₁₋₃ alkylthio, -NH(C₁₋₃ alkyl), -N(C₁₋₃ alkyl)₂, C₂₋₆ alkenyl, C₂₋₆ alkynyl, -(CH₂)ᵢC₂₋₆ alkenyl, -(CH₂)ᵢC₂₋₆ alkynyl, C₃₋₆ cycloalkyl, and 3- to 6-membered heterocycloalkyl, the C₁₋₃ alkyl, C₁₋₃ alkoxy, C₁₋₃ alkylthio, -NH(C₁₋₃ alkyl), -N(C₁₋₃ alkyl)₂, C₂₋₆ alkenyl, C₂₋₆ alkynyl, -(CH₂)ᵢC₂₋₆ alkenyl, or -(CH₂)ᵢC₂₋₆ alkynyl being optionally and independently substituted with 1, 2, or 3 halogens, OH, NH₂, or CN, and the C₃₋₆ cycloalkyl or 3- to 6-membered heterocycloalkyl being optionally and independently substituted with 1, 2, or 3 R^{d};
each R^{b} is independently selected from the group consisting of OH, CN, NH₂, halogen, =O, C₁₋₃ alkyl, C₁₋₃ alkoxy, C₁₋₃ alkylthio, -NH(C₁₋₃ alkyl), -N(C₁₋₃ alkyl)₂, -S(=O)₂-C₁₋₃ alkyl, -S(=O)₂-NH₂, -S(=O)₂-NH-C₁₋₃ alkyl, -S(=O)₂-C₃₋₆ cycloalkyl, -S(=O)₂-NH-C₃₋₆ cycloalkyl, -S(=O)(NH)-C₁₋₃ alkyl, -S(=O)(NH)-C₃₋₆ cycloalkyl, C₃₋₆ cycloalkyl, and 3- to 6-membered heterocycloalkyl, the C₁₋₃ alkyl, C₁₋₃ alkoxy, C₁₋₃ alkylthio, -NH(C₁₋₃ alkyl), -N(C₁₋₃ alkyl)₂, -S(=O)₂-C₁₋₃ alkyl, -S(=O)₂-NH-C₁₋₃ alkyl, or -S(=O)(NH)-C₁₋₃ alkyl being optionally and independently substituted with 1, 2, or 3 halogens, OH, NH₂, or CN, and the -S(=O)₂-C₃₋₆ cycloalkyl, -S(=O)₂-NH-C₃₋₆ cycloalkyl, -S(=O)(NH)-C₃₋₆ cycloalkyl, C₃₋₆ cycloalkyl, or 3- to 6-membered heterocycloalkyl being optionally and independently substituted with 1, 2, or 3 R^{d};
each R^{c} is independently selected from the group consisting of halogen, OH, CN, NH₂, C₁₋₃ alkyl, and C₁₋₃ halogenated alkyl;
each R^{d} is independently selected from the group consisting of halogen, OH, CN, NH₂, C₁₋₃ alkyl, and C₁₋₃ halogenated alkyl;
each R^{e} is independently selected from the group consisting of halogen, OH, CN, NH₂, C₁₋₃ alkyl, and C₁₋₃ halogenated alkyl;
m is selected from the group consisting of 0, 1, 2, and 3;
i is selected from the group consisting of 0, 1, and 2;
each R⁴, R⁵, R⁶, R^{c}, R^{d}, or R^{e} is optionally and independently substituted with one or more substituents; optionally, each R¹, R², R³, R^{a}, or R^{b} is independently substituted with one or more additional substituents.

In some embodiments of the present disclosure, each R⁴, R⁵, R^{6,} R^{c}, R^{d}, or R^{e} is independently and optionally substituted with 1, 2, or 3 substituents.

In some embodiments of the present disclosure, each R¹, R², R³, R^{a}, or R^{b} is independently and optionally substituted with one or more additional substituents.

In some embodiments of the present disclosure, the "1, 2, or 3" described in the present disclosure may also be "one or more", for example, it may be "1, 2, 3, 4, 5, or 6".

In some embodiments of the present disclosure, in the compound of formula (I) or the pharmaceutically acceptable salt thereof,
the structural unit 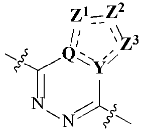 is fused bicyclic heteroaryl;
Q and Y are each independently selected from the group consisting of C, Si, and N, and one of Q and Y is N;
Z¹, Z², and Z³ are each independently selected from the group consisting of C(R³), Si(R³), and N;
X is selected from the group consisting of -N(R⁴)-(C(R⁵)(R⁶))ₘ-, -O-(C(R⁵)(R⁶))ₘ-, -S-(C(R⁵)(R⁶))ₘ-, and -(C(R⁵)(R⁶))ₘ-;
R¹ is selected from the group consisting of C₃₋₈ cycloalkyl, 3- to 8-membered heterocycloalkyl, C₆₋₁₀ aryl, and 5-to 9-membered heteroaryl, the C₃₋₈ cycloalkyl, 3- to 8-membered heterocycloalkyl, C₆₋₁₀ aryl, or 5- to 9-membered heteroaryl being optionally and independently substituted with 1, 2, or 3 R^{a};
R² is selected from the group consisting of C₁₋₆ alkyl, C₂₋₆ alkenyl, C₂₋₆ alkynyl, -(CH₂)ᵢC₃₋₈ cycloalkyl, -(CH₂)ᵢ-(3- to 8-membered heterocycloalkyl), -(CH₂)ᵢ-(C₆₋₁₀ aryl), and -(CH₂)ᵢ-(5- to 9-membered heteroaryl), the C₁₋₆ alkyl, C₂₋₆ alkenyl, or C₂₋₆ alkynyl being optionally and independently substituted with 1, 2, or 3 halogens, OH, NH₂, or CN, and the C₃₋₈ cycloalkyl, 3- to 8-membered heterocycloalkyl, C₆₋₁₀ aryl, or 5- to 9-membered heteroaryl being optionally and independently substituted with 1, 2, or 3 R^{b};
each R³ is independently selected from the group consisting of H, OH, CN, NH₂, halogen, C₁₋₃ alkyl, C₁₋₃ alkoxy, C₁₋₃ alkylthio, -NH(C₁₋₃ alkyl), -N(C₁₋₃ alkyl)₂, C₃₋₆ cycloalkyl, and 3- to 6-membered heterocycloalkyl, the C₁₋₃ alkyl, C₁₋₃ alkoxy, C₁₋₃ alkylthio, -NH(C₁₋₃ alkyl), or -N(C₁₋₃ alkyl)₂ being optionally and independently substituted with 1, 2, or 3 halogens, OH, NH₂, or CN, and the C₃₋₆ cycloalkyl or 3- to 6-membered heterocycloalkyl being optionally and independently substituted with 1, 2, or 3 R^{c};
R⁴ is selected from the group consisting of H, C₁₋₃ alkyl, halogenated C₁₋₃ alkyl, C₃₋₆ cycloalkyl, and 3- to 6-membered heterocycloalkyl, wherein the C₃₋₆ cycloalkyl or 3- to 6-membered heterocycloalkyl may be optionally and independently substituted with 1, 2, or 3 halogens, OH, NH₂, or CN;
R⁵ and R⁶ are each independently selected from the group consisting of H, OH, NH₂, CN, C₁₋₃ alkyl, and halogenated C₁₋₃ alkyl;
each R^{a} is independently selected from the group consisting of OH, CN, NH₂, halogen, =O, C₁₋₃ alkyl, C₁₋₃ alkoxy, C₁₋₃ alkylthio, -NH(C₁₋₃ alkyl), -N(C₁₋₃ alkyl)₂, C₂₋₆ alkenyl, C₂₋₆ alkynyl, -(CH₂)ᵢC₂₋₆ alkenyl, -(CH₂)ᵢC₂₋₆ alkynyl, C₃₋₆ cycloalkyl, and 3- to 6-membered heterocycloalkyl, the C₁₋₃ alkyl, C₁₋₃ alkoxy, C₁₋₃ alkylthio, -NH(C₁₋₃ alkyl), -N(C₁₋₃ alkyl)₂, C₂₋₆ alkenyl, C₂₋₆ alkynyl, -(CH₂)ᵢC₂₋₆ alkenyl, or -(CH₂)ᵢC₂₋₆ alkynyl being optionally and independently substituted with 1, 2, or 3 halogens, OH, NH₂, or CN, and the C₃₋₆ cycloalkyl or 3- to 6-membered heterocycloalkyl being optionally and independently substituted with 1, 2, or 3 R^{d};
each R^{b} is independently selected from the group consisting of OH, CN, NH₂, halogen, =O, C₁₋₃ alkyl, C₁₋₃ alkoxy, C₁₋₃ alkylthio, -NH(C₁₋₃ alkyl), -N(C₁₋₃ alkyl)₂, -S(=O)₂-C₁₋₃ alkyl, -S(=O)₂-NH₂, -S(=O)₂-NH-C₁₋₃ alkyl, -S(=O)₂-C₃₋₆ cycloalkyl, -S(=O)₂-NH-C₃₋₆ cycloalkyl, -S(=O)(NH)-C₁₋₃ alkyl, -S(=O)(NH)-C₃₋₆ cycloalkyl, C₃₋₆ cycloalkyl, and 3- to 6-membered heterocycloalkyl, the C₁₋₃ alkyl, C₁₋₃ alkoxy, C₁₋₃ alkylthio, -NH(C₁₋₃ alkyl), -N(C₁₋₃ alkyl)₂, -S(=O)₂-C₁₋₃ alkyl, -S(=O)₂-NH-C₁₋₃ alkyl, or -S(=O)(NH)-C₁₋₃ alkyl being optionally and independently substituted with 1, 2, or 3 halogens, OH, NH₂, or CN, and the -S(=O)₂-C₃₋₆ cycloalkyl, -S(=O)₂-NH-C₃₋₆ cycloalkyl, -S(=O)(NH)-C₃₋₆ cycloalkyl, C₃₋₆ cycloalkyl, or 3- to 6-membered heterocycloalkyl being optionally and independently substituted with 1, 2, or 3 R^{d};
each R^{c} is independently selected from the group consisting of halogen, OH, CN, NH₂, C₁₋₃ alkyl, and C₁₋₃ halogenated alkyl;
each R^{d} is independently selected from the group consisting of halogen, OH, CN, NH₂, C₁₋₃ alkyl, and C₁₋₃ halogenated alkyl;
each R^{e} is independently selected from the group consisting of halogen, OH, CN, NH₂, C₁₋₃ alkyl, and C₁₋₃ halogenated alkyl;
m is selected from the group consisting of 0, 1, 2, and 3;
i is selected from the group consisting of 0, 1, and 2.

In some embodiments of the present disclosure, the structural unit 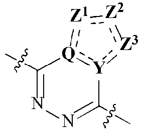 has one and only one fused bicyclic heteroaryl.

In some embodiments of the present disclosure, the structural unit 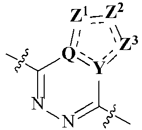 is fused bicyclic heteroaryl.

In some embodiments of the present disclosure, the structural unit 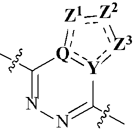 is fused tricyclic heteroaryl.

In some embodiments of the present disclosure, the structural unit 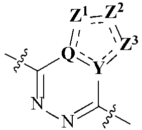 is a fused tricyclic structure, but has one and only one fused bicyclic heteroaryl.

In some embodiments of the present disclosure, the heteroatom in the "heterocycloalkyl" or "partially saturated heterocycloalkyl" described in the present disclosure is selected from the group consisting of N, O, S, Si, P, and Se.

In some embodiments of the present disclosure, the heteroatom in the "heterocycloalkyl" or "heteroaryl" described in the present disclosure is selected from the group consisting of N, O, and S.

In some embodiments of the present disclosure, the "heterocycloalkyl", "partially saturated heterocycloalkyl", or "heteroaryl" described in the present disclosure contains 1, 2, or 3 ring heteroatoms selected from the group consisting of N, O, S, Si, P, and Se, or contains 1, 2, or 3 ring heteroatoms selected from the group consisting of N, O, and S, or contains 1 or 2 ring heteroatoms selected from the group consisting of N, O, and S.

In some embodiments of the present disclosure, Q and Y are each independently selected from the group consisting of C and N, and one of Q and Y is N.

In some embodiments of the present disclosure, one and only one of Q and Y is N, and the other is Si or C.

In some embodiments of the present disclosure, one and only one of Q and Y is N, and the other is C.

In some embodiments of the present disclosure, Q is selected from N, and Y is selected from C.

In some embodiments of the present disclosure, Q is selected from C, and Y is selected from N.

In some embodiments of the present disclosure, Z¹, Z², and Z³ are each independently selected from the group consisting of C(R³) and N.

In some embodiments of the present disclosure, Q and Y are each independently selected from the group consisting of C and N, and one of Q and Y is N; Z¹, Z², and Z³ are each independently selected from the group consisting of C(R³) and N.

In some embodiments of the present disclosure, Z¹ is selected from N, and Z² and Z³ are selected from C(R³).

In some embodiments of the present disclosure, Z¹ and Z² are selected from N, and Z³ is selected from C(R³).

In some embodiments of the present disclosure, Z¹ and Z³ are selected from N, and Z² is selected from C(R³).

In some embodiments of the present disclosure, Z² is selected from N, and Z¹ and Z³ are selected from C(R³).

In some embodiments of the present disclosure, Z² and Z³ are selected from N, and Z¹ is selected from C(R³).

In some embodiments of the present disclosure, Z³ is selected from N, and Z¹ and Z² are selected from C(R³).

In some embodiments of the present disclosure, Z¹, Z², and Z³ are all selected from C(R³).

In some embodiments of the present disclosure, Z¹, Z², and Z³ are all selected from N.

In some embodiments of the present disclosure, Q is selected from N, Y is selected from C, Z¹ is selected from N, and Z² and Z³ are selected from C(R³).

In some embodiments of the present disclosure, Q is selected from N, Y is selected from C, Z¹ and Z² are selected from N, and Z³ is selected from C(R³).

In some embodiments of the present disclosure, Q is selected from N, Y is selected from C, Z¹ and Z³ are selected from N, and Z² is selected from C(R³).

In some embodiments of the present disclosure, Q is selected from N, Y is selected from C, Z² is selected from N, and Z¹ and Z³ are selected from C(R³).

In some embodiments of the present disclosure, Q is selected from N, Y is selected from C, Z² and Z³ are selected from N, and Z¹ is selected from C(R³).

In some embodiments of the present disclosure, Q is selected from N, Y is selected from C, Z³ is selected from N, and Z¹ and Z² are selected from C(R³).

In some embodiments of the present disclosure, Q is selected from N, Y is selected from C, and Z¹, Z², and Z³ are all selected from C(R³).

In some embodiments of the present disclosure, Q is selected from N, Y is selected from C, and Z¹, Z², and Z³ are all selected from N.

In some embodiments of the present disclosure, Q is selected from C, Y is selected from N, Z¹ is selected from N, and Z² and Z³ are selected from C(R³).

In some embodiments of the present disclosure, Q is selected from C, Y is selected from N, Z¹ and Z² are selected from N, and Z³ is selected from C(R³).

In some embodiments of the present disclosure, Q is selected from C, Y is selected from N, Z¹ and Z³ are selected from N, and Z² is selected from C(R³).

In some embodiments of the present disclosure, Q is selected from C, Y is selected from N, Z² is selected from N, and Z¹ and Z³ are selected from C(R³).

In some embodiments of the present disclosure, Q is selected from C, Y is selected from N, Z² and Z³ are selected from N, and Z¹ is selected from C(R³).

In some embodiments of the present disclosure, Q is selected from C, Y is selected from N, Z³ is selected from N, and Z¹ and Z² are selected from C(R³).

In some embodiments of the present disclosure, Q is selected from C, Y is selected from N, and Z¹, Z², and Z³ are all selected from C(R³).

In some embodiments of the present disclosure, Q is selected from C, Y is selected from N, and Z¹, Z², and Z³ are all selected from N.

In some embodiments of the present disclosure, each R³ is independently selected from the group consisting of H, OH, CN, NH₂, halogen, methyl, ethyl, isopropyl, methoxy, ethoxy, methylthio, ethylthio, -NHCH₃, -N(CH₃)₂, cyclopropyl, cyclobutyl, oxiranyl, thiiranyl, aziridinyl, oxetanyl, thietanyl, and azetidinyl, the methyl, ethyl, isopropyl, methoxy, ethoxy, methylthio, ethylthio, -NHCH₃, or -N(CH₃)₂ being optionally and independently substituted with 1, 2, or 3 halogens, OH, NH₂, or CN, and the cyclopropyl, cyclobutyl, oxiranyl, thiiranyl, aziridinyl, oxetanyl, thietanyl, or azetidinyl being optionally and independently substituted with 1, 2, or 3 R^{c}.

In some embodiments of the present disclosure, each R^{c} is independently selected from the group consisting of F, Cl, OH, methyl, and trifluoromethyl.

In some embodiments of the present disclosure, each R^{c} is independently selected from the group consisting of OH and methyl.

In some embodiments of the present disclosure, each R³ is independently selected from the group consisting of H, OH, CN, NH₂, halogen, methyl, ethyl, isopropyl, methoxy, ethoxy, methylthio, ethylthio, -NHCH₃, and -N(CH₃)₂, the methyl, ethyl, isopropyl, methoxy, ethoxy, methylthio, ethylthio, -NHCH₃, or -N(CH₃)₂ being optionally and independently substituted with 1, 2, or 3 halogens, OH, NH₂, or CN.

In some embodiments of the present disclosure, each R³ is independently selected from the group consisting of H, OH, CN, NH₂, F, Cl, Br, I, methyl, ethyl, isopropyl, difluoromethyl, trifluoromethyl, methoxy, ethoxy, difluoromethoxy, and trifluoromethoxy.

In some embodiments of the present disclosure, each R³ is independently selected from the group consisting of H, F, Cl, methyl, and trifluoromethyl.

In some embodiments of the present disclosure, each R³ is independently selected from H.

In some embodiments of the present disclosure, Z¹ and Z² are each independently selected from C(R³), and the R³ groups on Z¹ and Z², together with the atoms to which they are attached, form C₃₋₈ partially saturated cycloalkyl, 3- to 8-membered partially saturated heterocycloalkyl, C₆₋₁₀ aryl, or 5- to 9-membered heteroaryl, the C₃₋₈ partially saturated cycloalkyl, 3- to 8-membered partially saturated heterocycloalkyl, C₆₋₁₀ aryl, or 5- to 9-membered heteroaryl being optionally and independently substituted with 1, 2, or 3 R^{a}.

In some embodiments of the present disclosure, Z¹ and Z² are each independently selected from C(R³), and the R³ groups on Z¹ and Z², together with the atoms to which they are attached, form C₅₋₆ partially saturated cycloalkyl, 5- to 6-membered partially saturated heterocycloalkyl, phenyl, or 5- to 6-membered heteroaryl, the C₅₋₆ partially saturated cycloalkyl, 5- to 6-membered partially saturated heterocycloalkyl, phenyl, or 5- to 6-membered heteroaryl being optionally and independently substituted with 1, 2, or 3 R^{a}.

In some embodiments of the present disclosure, Z¹ and Z² are each independently selected from C(R³), and the R³ groups on Z¹ and Z², together with the atoms to which they are attached, form C₅₋₆ partially saturated cycloalkyl, 5- to 6-membered partially saturated heterocycloalkyl containing 1, 2, or 3 ring heteroatoms selected from the group consisting of N, O, and S, phenyl, or 5- to 6-membered heteroaryl containing 1, 2, or 3 ring heteroatoms selected from the group consisting of N, O, and S, the C₅₋₆ partially saturated cycloalkyl, 5- to 6-membered partially saturated heterocycloalkyl, phenyl, or 5- to 6-membered heteroaryl being optionally and independently substituted with 1, 2, or 3 R^{a}.

In some embodiments of the present disclosure, Z¹ and Z² are each independently selected from C(R³), and the R³ groups on Z¹ and Z², together with the atoms to which they are attached, form C₅₋₆ partially saturated cycloalkyl, 5- to 6-membered partially saturated heterocycloalkyl containing 1 or 2 ring heteroatoms selected from the group consisting of N, O, and S, phenyl, or 5- to 6-membered heteroaryl containing 1 or 2 ring heteroatoms selected from the group consisting of N, O, and S, the C₅₋₆ partially saturated cycloalkyl, 5- to 6-membered partially saturated heterocycloalkyl, phenyl, or 5- to 6-membered heteroaryl being optionally and independently substituted with 1, 2, or 3 R^{a}.

In some embodiments of the present disclosure, Z¹ and Z² are each independently selected from C(R³), and the R³ groups on Z¹ and Z², together with the atoms to which they are attached, form C₅₋₆ partially saturated cycloalkyl, 5- to 6-membered partially saturated heterocycloalkyl containing 1 or 2 ring heteroatoms selected from the group consisting of N, O, and S, phenyl, or 5- to 6-membered heteroaryl containing 1 or 2 ring heteroatoms selected from the group consisting of N, O, and S, the C₅₋₆ partially saturated cycloalkyl, 5- to 6-membered partially saturated heterocycloalkyl, phenyl, or 5- to 6-membered heteroaryl being optionally and independently substituted with 1, 2, or 3 R^{a}.

In some embodiments of the present disclosure, Z¹ and Z² are each independently selected from C(R³), and the R³ groups on Z¹ and Z², together with the atoms to which they are attached, form C₅₋₆ partially saturated cycloalkyl, 5- to 6-membered partially saturated heterocycloalkyl containing 1 or 2 ring heteroatoms selected from the group consisting of N, O, and S, or 5-membered heteroaryl containing 1 or 2 ring heteroatoms selected from the group consisting of N, O, and S, the C₅₋₆ partially saturated cycloalkyl, 5- to 6-membered partially saturated heterocycloalkyl, or 5-membered heteroaryl being optionally and independently substituted with 1, 2, or 3 R^{a}.

In some embodiments of the present disclosure, Z¹ and Z² are each independently selected from C(R³), and the R³ groups on Z¹ and Z², together with the atoms to which they are attached, form 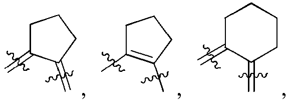 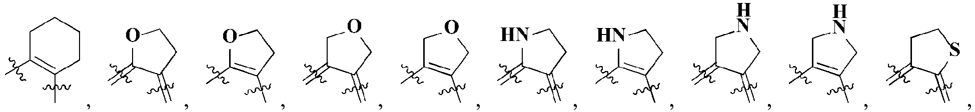 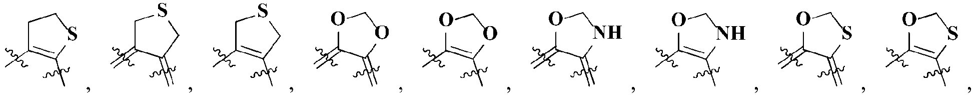 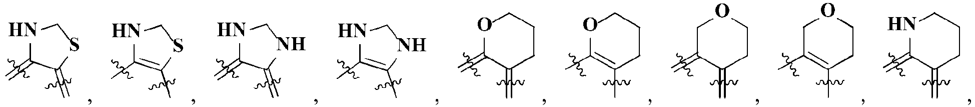 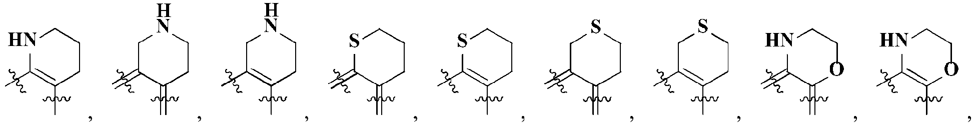 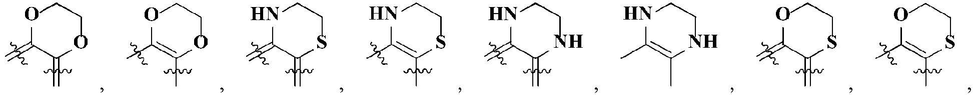 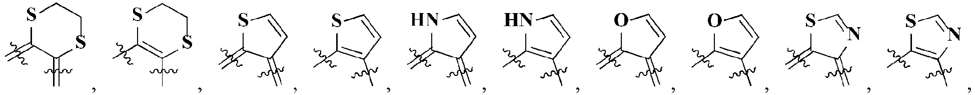 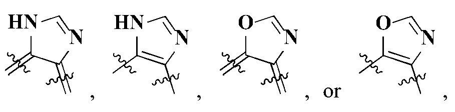 the 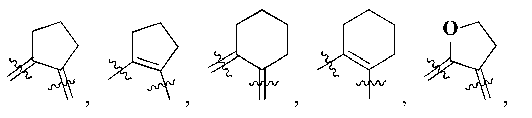 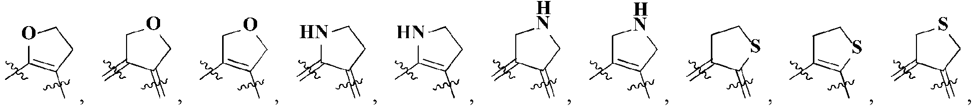 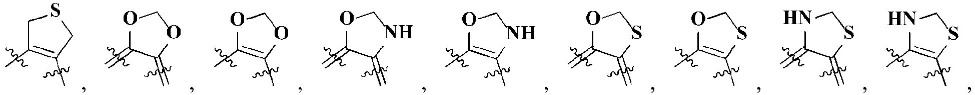 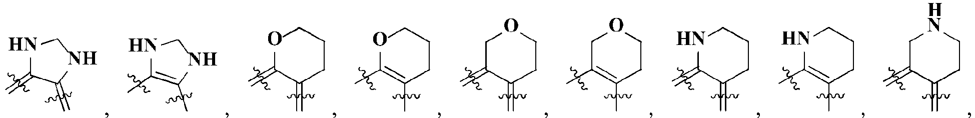 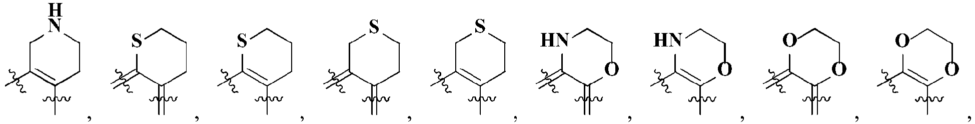 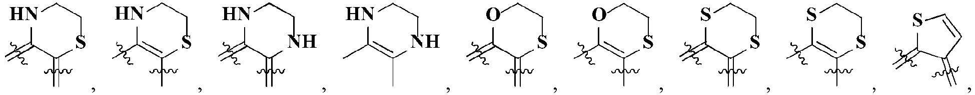 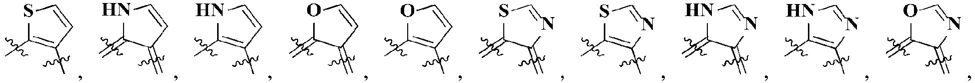 or 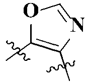 being optionally and independently substituted with 1, 2, or 3 R^{a}.

In some embodiments of the present disclosure, Z¹ and Z² are each independently selected from C(R³), and the R³ groups on Z¹ and Z², together with the atoms to which they are attached, form 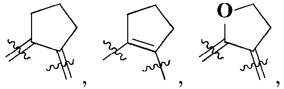 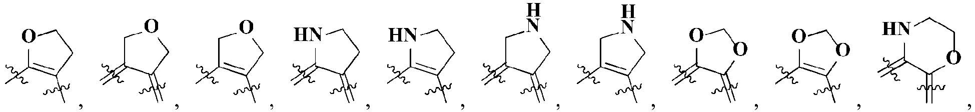 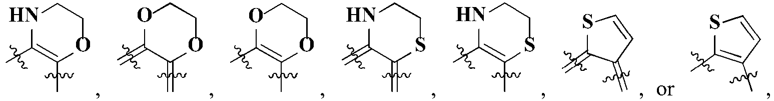 the 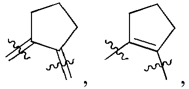 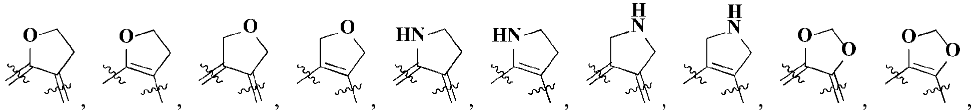 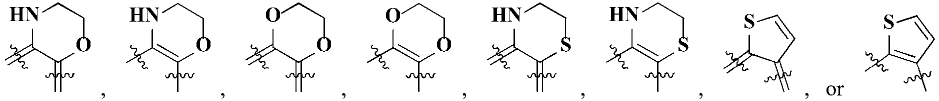 being optionally and independently substituted with 1, 2, or 3 R^{a}.

In some embodiments of the present disclosure, Z¹ and Z² are each independently selected from C(R³), and the R³ groups on Z¹ and Z², together with the atoms to which they are attached, form 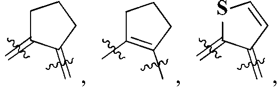 or 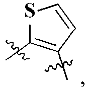 the 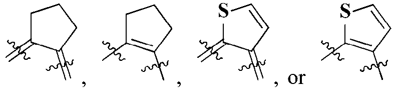 being optionally and independently substituted with 1, 2, or 3 R^{a}.

In some embodiments of the present disclosure, Z² and Z³ are each independently selected from C(R³), and the R³ groups on Z² and Z³, together with the atoms to which they are attached, form C₃₋₈ partially saturated cycloalkyl, 3- to 8-membered partially saturated heterocycloalkyl, C₆₋₁₀ aryl, or 5- to 9-membered heteroaryl, the C₃₋₈ partially saturated cycloalkyl, 3- to 8-membered partially saturated heterocycloalkyl, C₆₋₁₀ aryl, or 5- to 9-membered heteroaryl being optionally and independently substituted with 1, 2, or 3 R^{a}.

In some embodiments of the present disclosure, Z² and Z³ are each independently selected from C(R³), and the R³ groups on Z² and Z³, together with the atoms to which they are attached, form C₅₋₆ partially saturated cycloalkyl, 5- to 6-membered partially saturated heterocycloalkyl, phenyl, or 5- to 6-membered heteroaryl, the C₅₋₆ partially saturated cycloalkyl, 5- to 6-membered partially saturated heterocycloalkyl, phenyl, or 5- to 6-membered heteroaryl being optionally and independently substituted with 1, 2, or 3 R^{a}.

In some embodiments of the present disclosure, Z² and Z³ are each independently selected from C(R³), and the R³ groups on Z² and Z³, together with the atoms to which they are attached, form C₅₋₆ partially saturated cycloalkyl, 5- to 6-membered partially saturated heterocycloalkyl containing 1, 2, or 3 ring heteroatoms selected from the group consisting of N, O, and S, phenyl, or 5- to 6-membered heteroaryl containing 1, 2, or 3 heteroatoms selected from the group consisting of N, O, and S, the C₅₋₆ partially saturated cycloalkyl, 5- to 6-membered partially saturated heterocycloalkyl, phenyl, or 5- to 6-membered heteroaryl being optionally and independently substituted with 1, 2, or 3 R^{a}.

In some embodiments of the present disclosure, Z² and Z³ are each independently selected from C(R³), and the R³ groups on Z² and Z³, together with the atoms to which they are attached, form C₅₋₆ partially saturated cycloalkyl, 5- to 6-membered partially saturated heterocycloalkyl containing 1 or 2 ring heteroatoms selected from the group consisting of N, O, and S, phenyl, or 5- to 6-membered heteroaryl containing 1 or 2 heteroatoms selected from the group consisting of N, O, and S, the C₅₋₆ partially saturated cycloalkyl, 5- to 6-membered partially saturated heterocycloalkyl, phenyl, or 5- to 6-membered heteroaryl being optionally and independently substituted with 1, 2, or 3 R^{a}.

In some embodiments of the present disclosure, Z² and Z³ are each independently selected from C(R³), and the R³ groups on Z² and Z³, together with the atoms to which they are attached, form C₅₋₆ partially saturated cycloalkyl, 5- to 6-membered partially saturated heterocycloalkyl containing 1 or 2 ring heteroatoms selected from the group consisting of N, O, and S, phenyl, or 5- to 6-membered heteroaryl containing 1 or 2 heteroatoms selected from the group consisting of N, O, and S, the C₅₋₆ partially saturated cycloalkyl, 5- to 6-membered partially saturated heterocycloalkyl, phenyl, or 5- to 6-membered heteroaryl being optionally and independently substituted with 1, 2, or 3 R^{a}.

In some embodiments of the present disclosure, Z² and Z³ are each independently selected from C(R³), and the R³ groups on Z² and Z³, together with the atoms to which they are attached, form C₅₋₆ partially saturated cycloalkyl, 5- to 6-membered partially saturated heterocycloalkyl containing 1 or 2 ring heteroatoms selected from the group consisting of N, O, and S, or 5-membered heteroaryl containing 1 or 2 heteroatoms selected from the group consisting of N, O, and S, the C₅₋₆ partially saturated cycloalkyl, 5- to 6-membered partially saturated heterocycloalkyl, or 5-membered heteroaryl being optionally and independently substituted with 1, 2, or 3 R^{a}.

In some embodiments of the present disclosure, Z² and Z³ are each independently selected from C(R³), and the R³ groups on Z² and Z³, together with the atoms to which they are attached, form 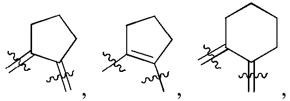 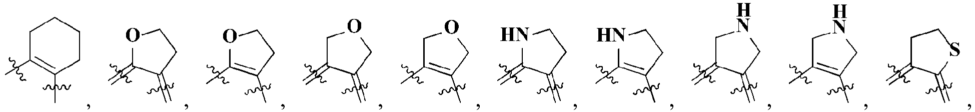 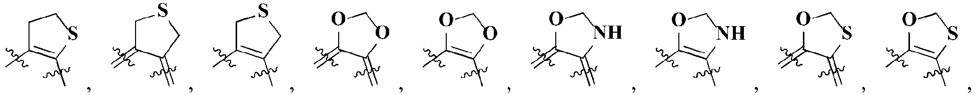 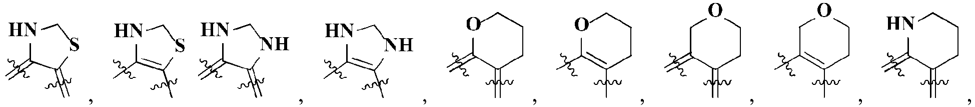 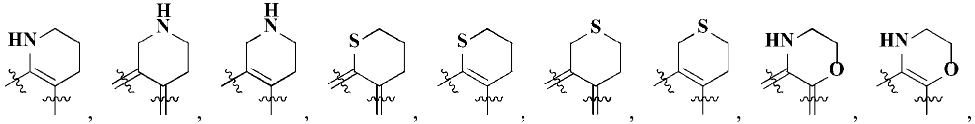 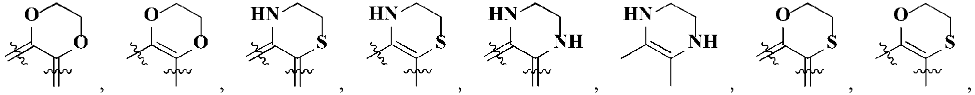 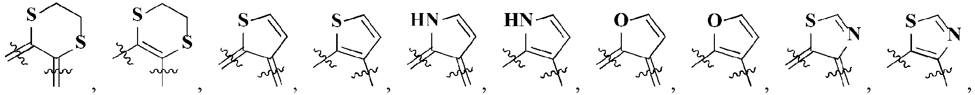 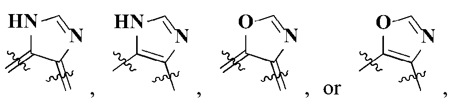 the 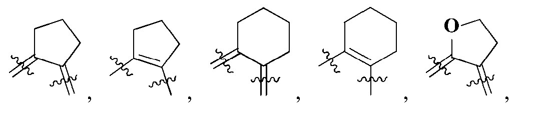 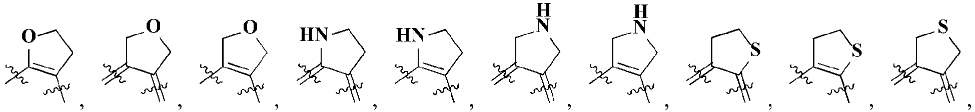 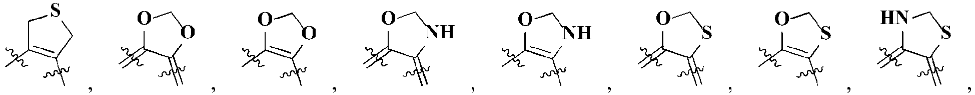 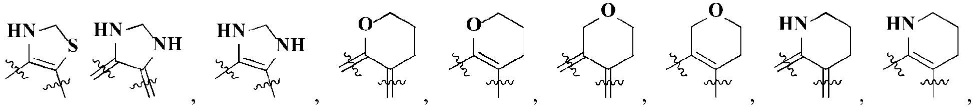 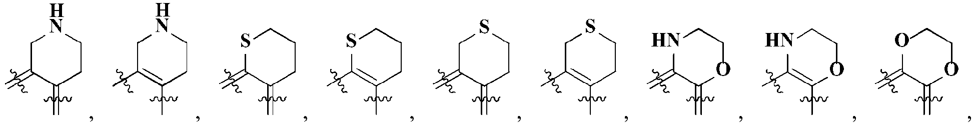 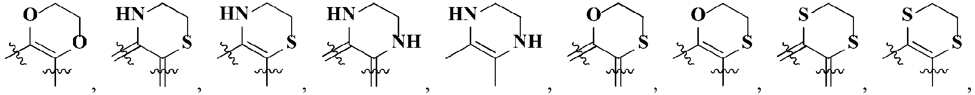 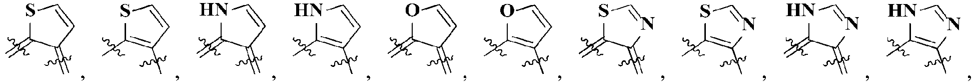 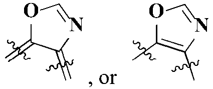 being optionally and independently substituted with 1, 2, or 3 R^{a}.

In some embodiments of the present disclosure, Z² and Z³ are each independently selected from C(R³), and the R³ groups on Z² and Z³, together with the atoms to which they are attached, form 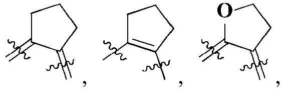 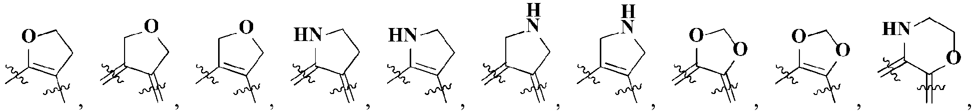 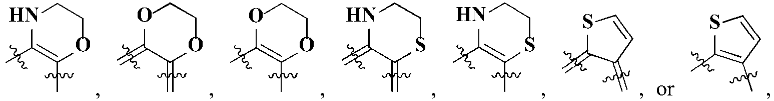 the 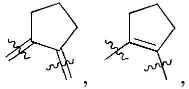 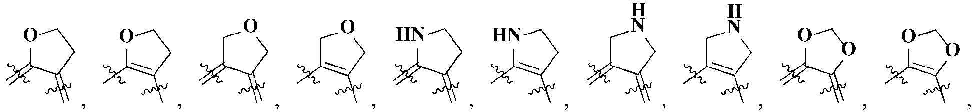 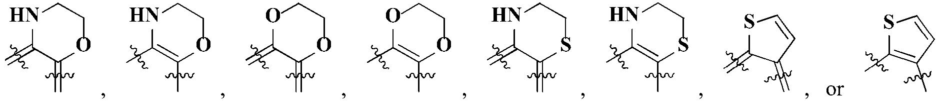 being optionally and independently substituted with 1, 2, or 3 R^{a}.

In some embodiments of the present disclosure, Z² and Z³ are each independently selected from C(R³), and the R³ groups on Z² and Z³, together with the atoms to which they are attached, form 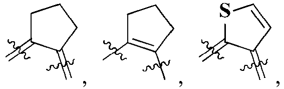 or 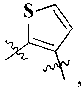 the 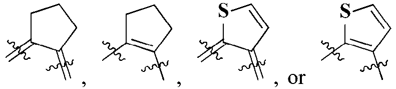 being optionally and independently substituted with 1, 2, or 3 R^{a}.

In some embodiments of the present disclosure, the structural unit 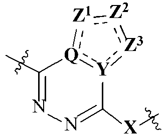 is selected from the group consisting of 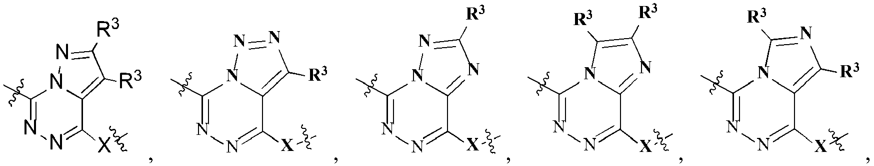 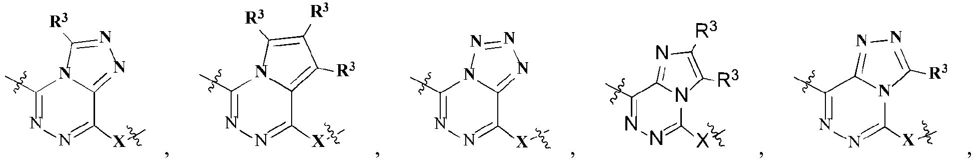 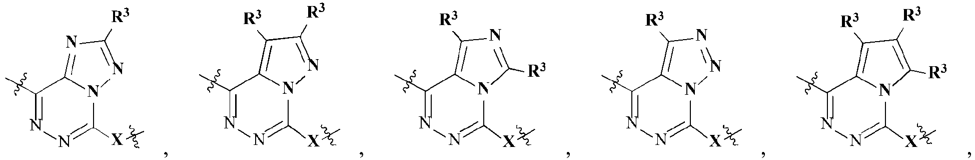 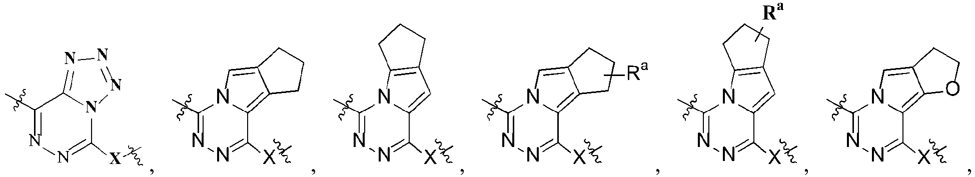 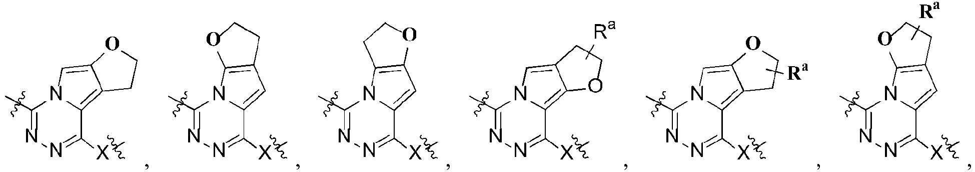 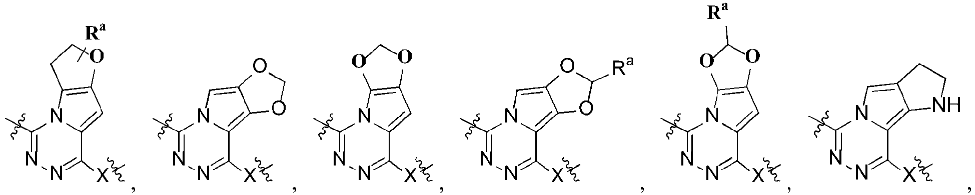 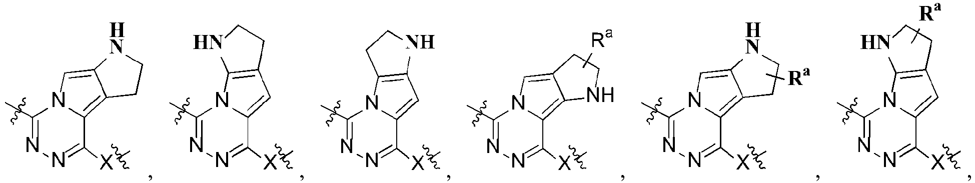 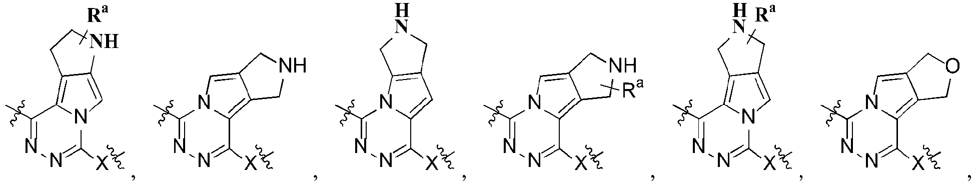 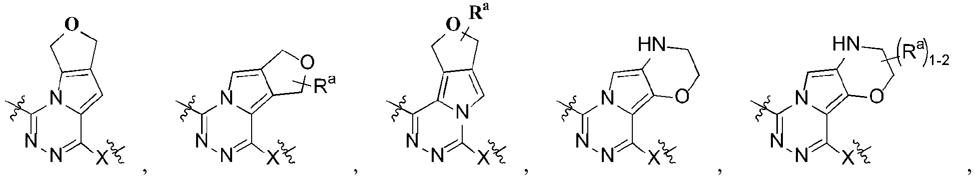 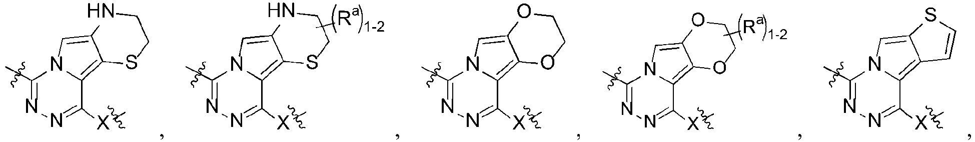 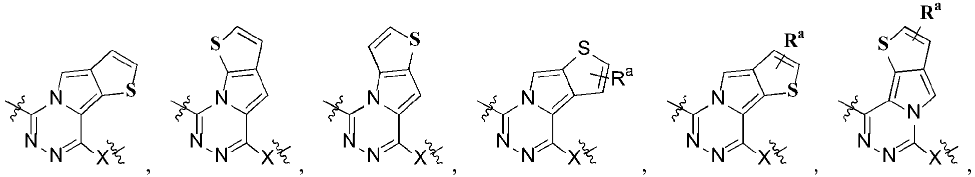 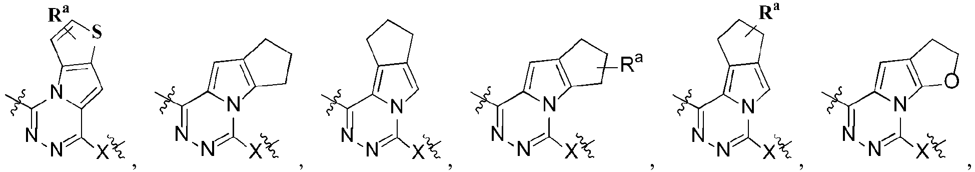 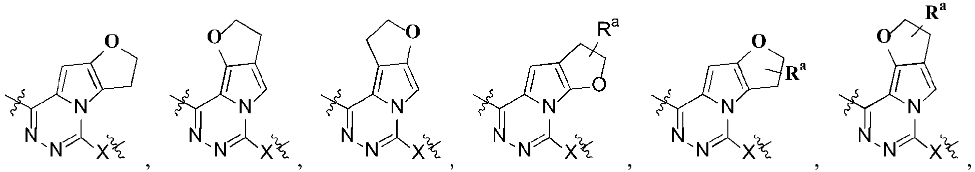 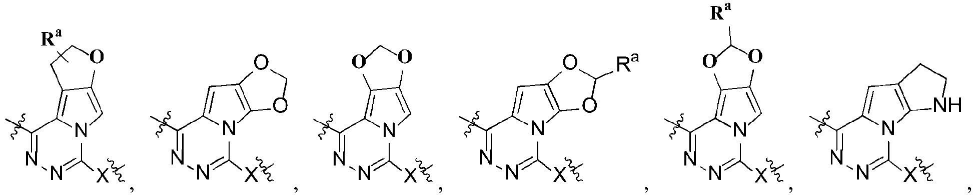 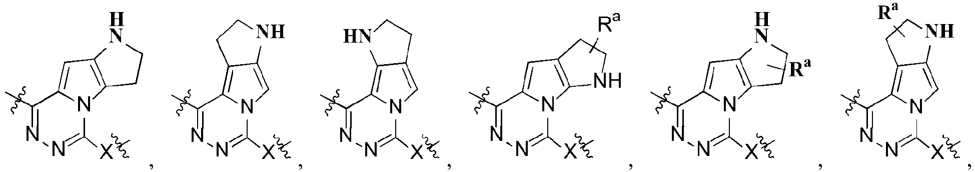 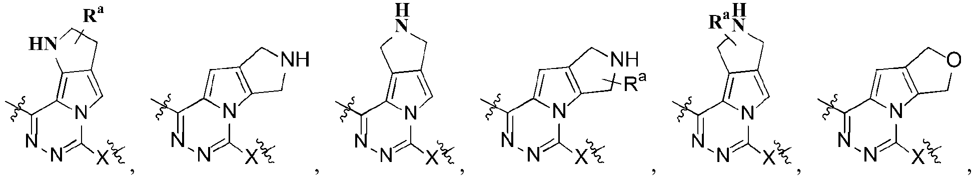 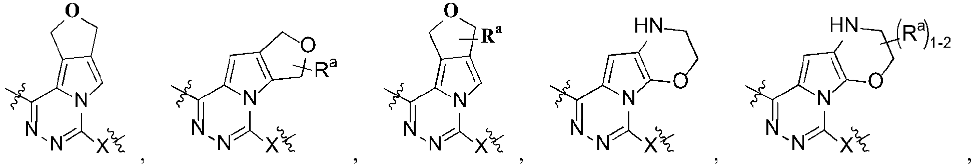 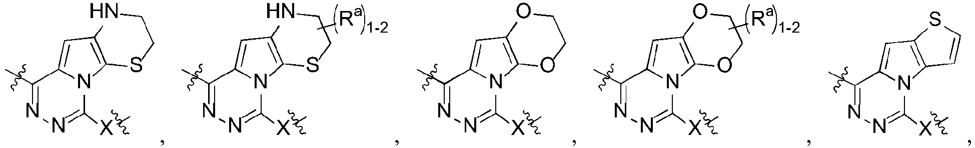 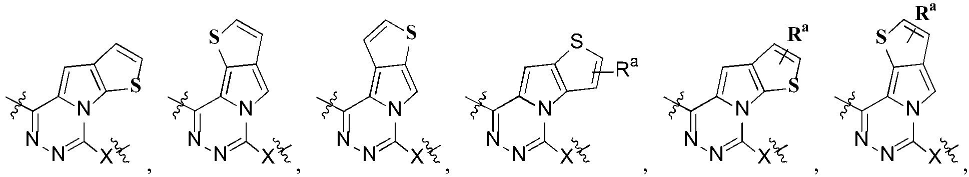 and

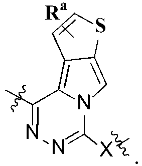

In some embodiments of the present disclosure, the structural unit 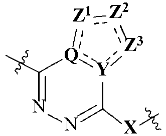 is selected from the group consisting of 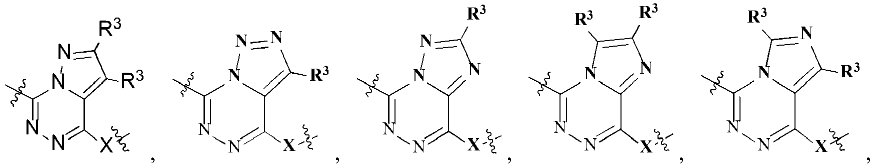 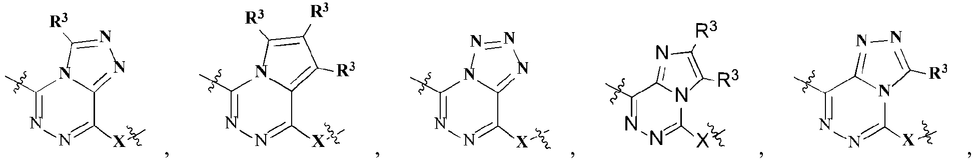 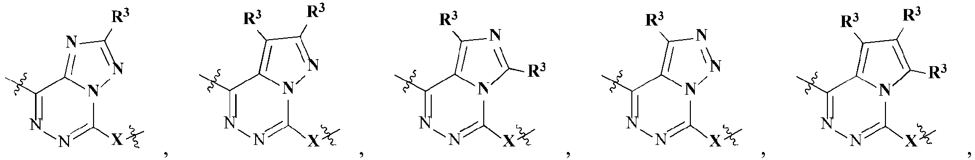 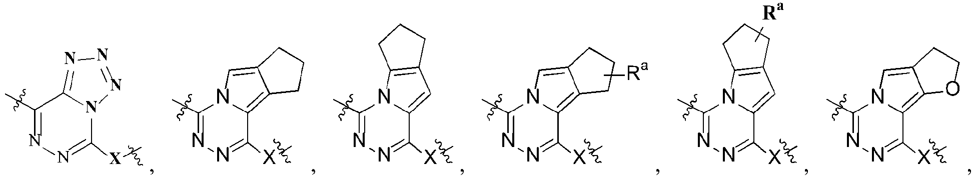 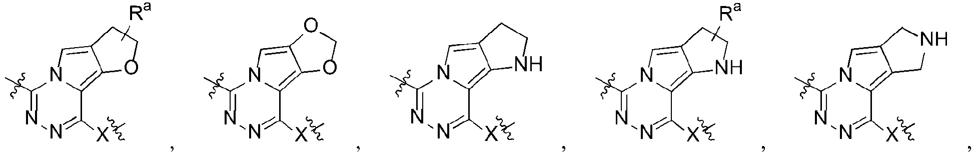 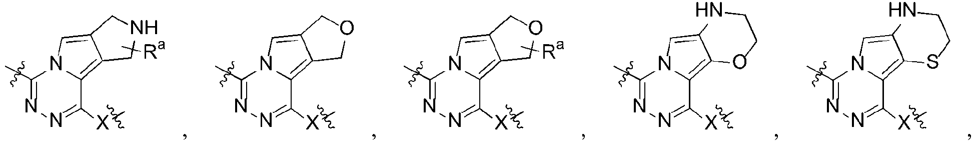 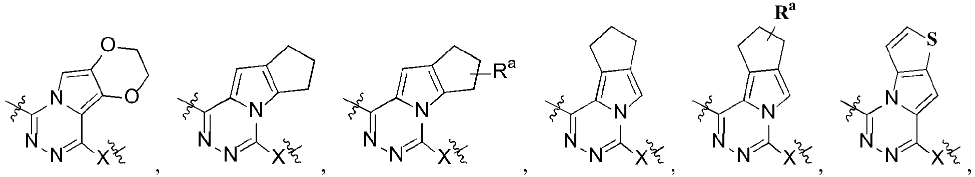 and

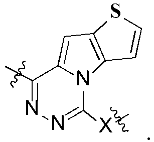

In some embodiments of the present disclosure, the structural unit 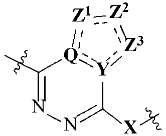 is selected from the group consisting of 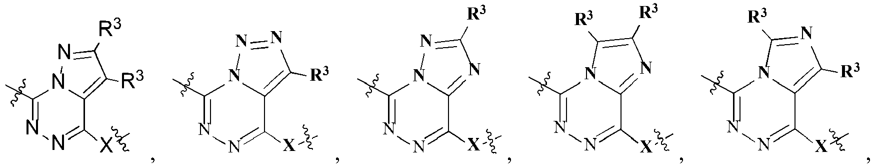 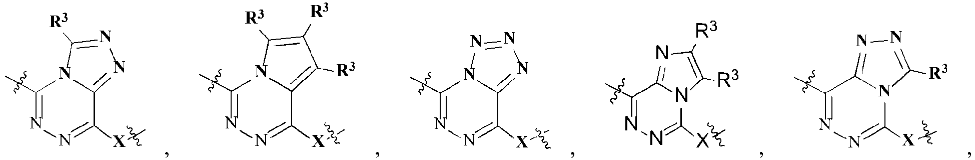 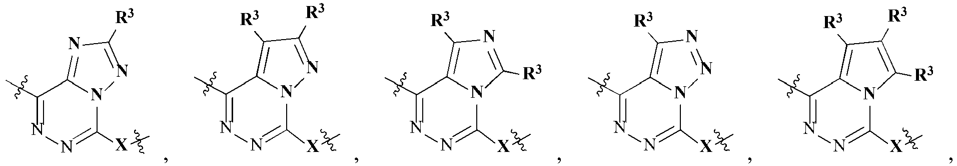 and

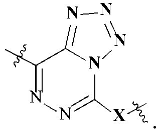

In some embodiments of the present disclosure, the structural unit 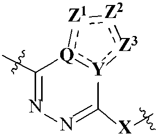 is selected from the group consisting of

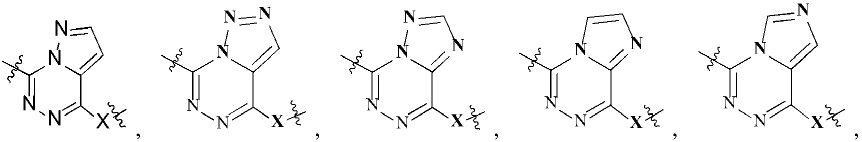

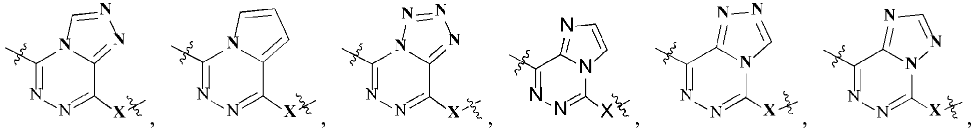

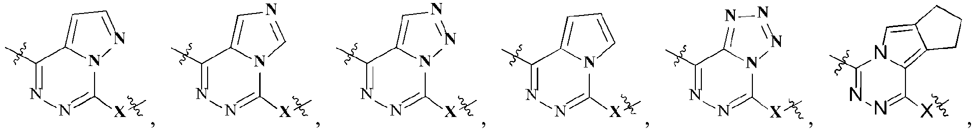

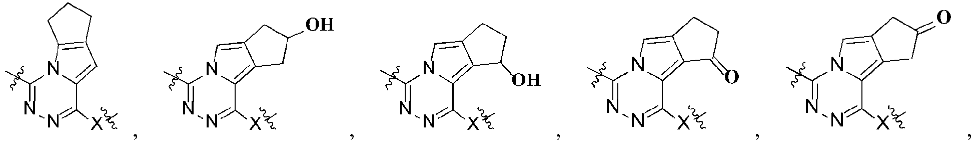

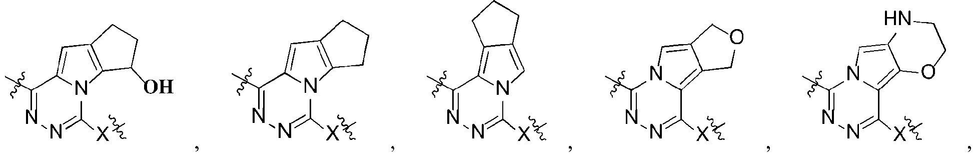

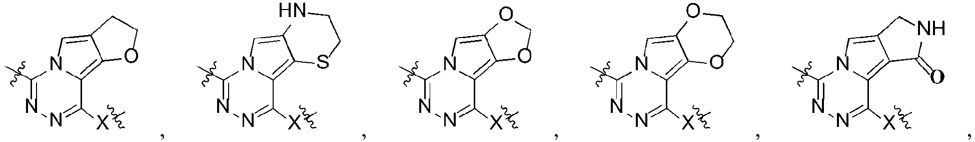

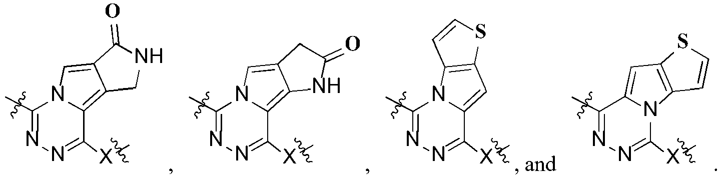

In some embodiments of the present disclosure, the structural unit 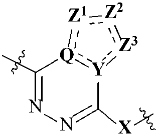 is selected from the group consisting of 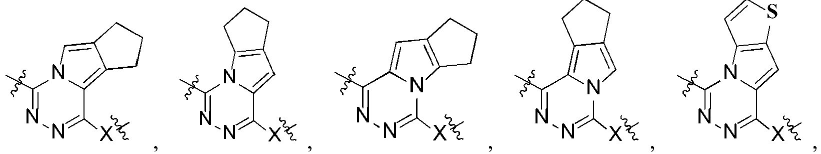 and

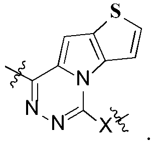

In some embodiments of the present disclosure, the structural unit 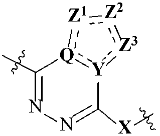 is selected from the group consisting of

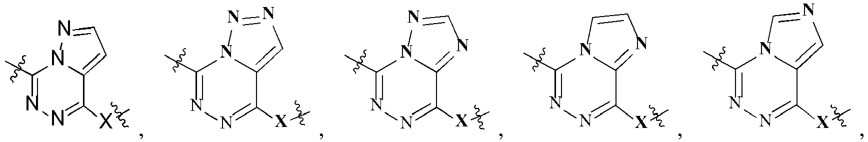

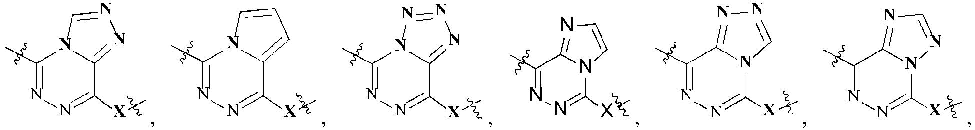

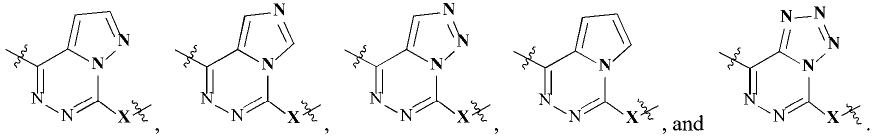

In some embodiments of the present disclosure, the structural unit 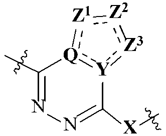 is selected from the group consisting of

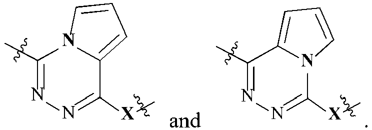

In some embodiments of the present disclosure, X is selected from the group consisting of -N(R⁴)-, -O-, -S-, and -(C(R⁵)(R⁶))ₘ-.

In some embodiments of the present disclosure, R⁴ is selected from the group consisting of H, methyl, ethyl, and isopropyl.

In some embodiments of the present disclosure, R⁴ is selected from H.

In some embodiments of the present disclosure, R⁵ and R⁶ are each independently selected from the group consisting of H and OH.

In some embodiments of the present disclosure, R⁵ and R⁶ are each independently selected from H.

In some embodiments of the present disclosure, X is selected from the group consisting of -NH-, -O-, -S-, -CH₂-, and -CH₂CH₂-.

In some embodiments of the present disclosure, X is selected from -NH-.

In some embodiments of the present disclosure, R¹ is selected from the group consisting of C₃₋₆ cycloalkyl, 3- to 6-membered heterocycloalkyl, phenyl, and 5- to 6-membered heteroaryl, the C₃₋₆ cycloalkyl, 3- to 6-membered heterocycloalkyl, phenyl, or 5- to 6-membered heteroaryl being optionally and independently substituted with 1, 2, or 3 R^{a}.

In some embodiments of the present disclosure, R¹ is selected from the group consisting of C₃₋₆ cycloalkyl, 3- to 6-membered heterocycloalkyl containing 1, 2, or 3 heteroatoms selected from the group consisting of N, O, and S, phenyl, and 5- to 6-membered heteroaryl containing 1, 2, or 3 heteroatoms selected from the group consisting of N, O, and S, the C₃₋₆ cycloalkyl, 3- to 6-membered heterocycloalkyl, phenyl, or 5- to 6-membered heteroaryl being optionally and independently substituted with 1, 2, or 3 R^{a}.

In some embodiments of the present disclosure, R¹ is selected from the group consisting of phenyl and 5- to 6-membered heteroaryl containing 1, 2, or 3 heteroatoms selected from the group consisting of N, O, and S, the phenyl or 5- to 6-membered heteroaryl being optionally and independently substituted with 1, 2, or 3 R^{a}.

In some embodiments of the present disclosure, R¹ is selected from the group consisting of phenyl, pyrrolyl, pyrazolyl, imidazolyl, oxazolyl, isoxazolyl, thiazole ring, isothiazolyl, triazolyl, furanyl, thienyl, pyridinyl, pyrazinyl, pyrimidinyl, and pyridazinyl, the phenyl, pyrrolyl, pyrazolyl, imidazolyl, oxazolyl, isoxazolyl, thiazole ring, isothiazolyl, triazolyl, furanyl, thienyl, pyridinyl, pyrazinyl, pyrimidinyl, or pyridazinyl being optionally and independently substituted with 1, 2, or 3 R^{a}.

In some embodiments of the present disclosure, R¹ is selected from the group consisting of phenyl, pyridinyl, pyrazinyl, pyrimidinyl, and pyridazinyl, the phenyl, pyridinyl, pyrazinyl, pyrimidinyl, or pyridazinyl being optionally and independently substituted with 1, 2, or 3 R^{a}.

In some embodiments of the present disclosure, R¹ is selected from phenyl, the phenyl being optionally and independently substituted with 1, 2, or 3 R^{a}.

In some embodiments of the present disclosure, R¹ is selected from

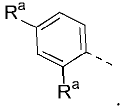

In some other embodiments of the present disclosure, each R^{a} is independently selected from the group consisting of OH, CN, NH₂, halogen, =O, C₁₋₃ alkyl, C₁₋₃ alkoxy, C₁₋₃ alkylthio, -NH(C₁₋₃ alkyl), -N(C₁₋₃ alkyl)₂, C₃₋₆ cycloalkyl, and 3- to 6-membered heterocycloalkyl, the C₁₋₃ alkyl, C₁₋₃ alkoxy, C₁₋₃ alkylthio, -NH(C₁₋₃ alkyl), or -N(C₁₋₃ alkyl)₂ being optionally and independently substituted with 1, 2, or 3 halogens, OH, NH₂, or CN, and the C₃₋₆ cycloalkyl or 3- to 6-membered heterocycloalkyl being optionally and independently substituted with 1, 2, or 3 R^{d}.

In some other embodiments of the present disclosure, each R^{a} is independently selected from the group consisting of OH, CN, halogen, C₁₋₃ alkyl, C₁₋₃ alkoxy, C₂₋₄ alkenyl, C₂₋₄ alkynyl, and -(CH₂)ᵢC₂₋₄ alkynyl, the C₁₋₃ alkyl, C₁₋₃ alkoxy, C₂₋₄ alkenyl, C₂₋₄ alkynyl, or -(CH₂)ᵢC₂₋₄ alkynyl being optionally and independently substituted with 1, 2, or 3 halogens, OH, NH₂, or CN.

In some other embodiments of the present disclosure, each R^{a} is independently selected from the group consisting of C₂₋₄ alkenyl, C₂₋₄ alkynyl, and -(CH₂)ᵢC₂₋₄ alkynyl, the C₂₋₄ alkenyl, C₂₋₄ alkynyl, or -(CH₂)ᵢC₂₋₄ alkynyl being optionally and independently substituted with 1, 2, or 3 halogens, OH, NH₂, or CN.

In some embodiments of the present disclosure, each R^{a} is independently selected from the group consisting of OH, CN, NH₂, halogen, =O, methyl, ethyl, isopropyl, methoxy, ethoxy, methylthio, ethylthio, -NHCH₃, -N(CH₃)₂, cyclopropyl, cyclobutyl, oxiranyl, thiiranyl, aziridinyl, oxetanyl, thietanyl, and azetidinyl, the methyl, ethyl, isopropyl, methoxy, ethoxy, methylthio, ethylthio, -NHCH₃, or -N(CH₃)₂ being optionally and independently substituted with 1, 2, or 3 halogens, OH, NH₂, or CN, and the cyclopropyl, cyclobutyl, oxiranyl, thiiranyl, aziridinyl, oxetanyl, thietanyl, or azetidinyl being optionally and independently substituted with 1, 2, or 3 R^{d}. In some other embodiments of the present disclosure, each R^{a} is independently selected from the group consisting of 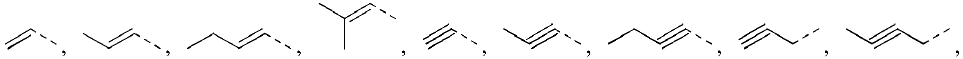 and 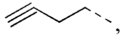 and R^{a} is optionally and independently substituted with 1, 2, or 3 halogens, OH, NH₂, or CN.

In some embodiments of the present disclosure, each R^{a} is independently selected from the group consisting of F, Cl, Br, I, =O, OH, CN, NH₂, methyl, and methoxy, the methyl or methoxy being optionally and independently substituted with 1, 2, or 3 halogens, OH, NH₂, or CN. In some other embodiments of the present disclosure, each R^{a} is independently selected from the group consisting of ethyl, 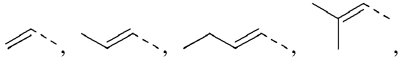 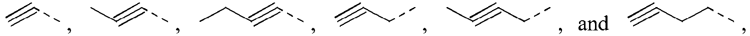 and R^{a} is optionally and independently substituted with 1, 2, or 3 halogens, OH, NH₂, or CN.

In some embodiments of the present disclosure, each R^{a} is independently selected from the group consisting of F, Cl, Br, I, OH, CN, NH₂, methyl, and methoxy, the methyl or methoxy being optionally and independently substituted with 1, 2, or 3 halogens, OH, NH₂, or CN. In some other embodiments of the present disclosure, each R^{a} is independently selected from the group consisting of ethyl, 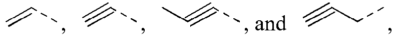 and R^{a} is optionally and independently substituted with 1, 2, or 3 halogens, OH, NH₂, or CN.

In some embodiments of the present disclosure, each R^{a} is independently selected from the group consisting of F, Cl, =O, OH, and trifluoromethyl. In some embodiments of the present disclosure, each R^{a} is independently selected from the group consisting of F, Cl, OH, and trifluoromethyl. In some other embodiments of the present disclosure, each R^{a} is independently selected from the group consisting of trifluoromethoxy, difluoromethoxy, CF₃CH₂-, CN,

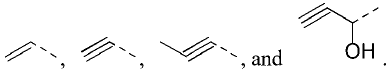

In some embodiments of the present disclosure, each R^{a} is independently selected from the group consisting of OH and trifluoromethyl.

In some other embodiments of the present disclosure, each R^{a} is independently selected from the group consisting of

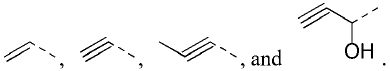

In some embodiments of the present disclosure, each R^{d} is independently selected from the group consisting of F, Cl, OH, methyl, and trifluoromethyl.

In some embodiments of the present disclosure, R¹ is selected from the group consisting of 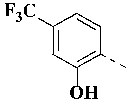 and 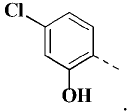 In some other embodiments of the present disclosure, R¹ is selected from the group consisting of

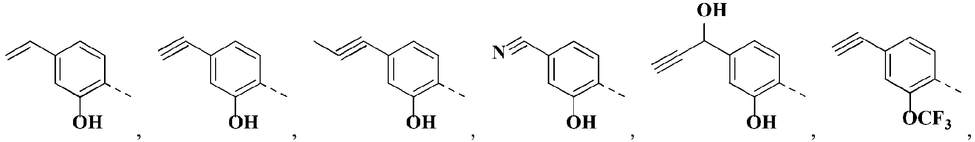

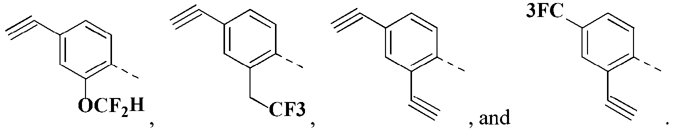

In some embodiments of the present disclosure, R¹ is selected from

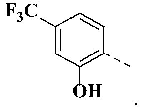

In some other embodiments of the present disclosure, R¹ is selected from the group consisting of 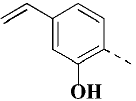 and

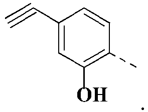

In some embodiments of the present disclosure, R² is selected from the group consisting of C₁₋₄ alkyl, C₂₋₄ alkenyl, C₂₋₄ alkynyl, -(CH₂)ᵢC₃₋₆ cycloalkyl, -(CH₂)ᵢ-(3- to 6-membered heterocycloalkyl), -(CH₂)ᵢ-phenyl, and -(CH₂)ᵢ-(5- to 6-membered heteroaryl), the C₁₋₄ alkyl, C₂₋₄ alkenyl, C₂₋₄ alkynyl, C₃₋₆ cycloalkyl, 3- to 6-membered heterocycloalkyl, phenyl, or 5- to 6-membered heteroaryl being optionally and independently substituted with 1, 2, or 3 R^{b}.

In some embodiments of the present disclosure, R² is selected from the group consisting of C₁₋₄ alkyl, -(CH₂)ᵢC₃₋₆ cycloalkyl, -(CH₂)ᵢ-(3- to 6-membered heterocycloalkyl), -(CH₂)ᵢ-phenyl, and -(CH₂)ᵢ-(5- to 6-membered heteroaryl), the C₁₋₄ alkyl, C₃₋₆ cycloalkyl, 3- to 6-membered heterocycloalkyl, phenyl, or 5- to 6-membered heteroaryl being optionally and independently substituted with 1, 2, or 3 R^{b}.

In some embodiments of the present disclosure, R² is selected from the group consisting of C₁₋₄ alkyl, C₃₋₆ cycloalkyl, 3- to 6-membered heterocycloalkyl, phenyl, and 5- to 6-membered heteroaryl, the C₁₋₄ alkyl, C₃₋₆ cycloalkyl, 3- to 6-membered heterocycloalkyl, phenyl, or 5- to 6-membered heteroaryl being optionally and independently substituted with 1, 2, or 3 R^{b}.

In some embodiments of the present disclosure, R² is selected from the group consisting of C₁₋₄ alkyl, C₃₋₆ cycloalkyl, 3- to 6-membered heterocycloalkyl containing 1, 2, or 3 heteroatoms selected from the group consisting of N, O, and S, phenyl, and 5- to 6-membered heteroaryl containing 1, 2, or 3 heteroatoms selected from the group consisting of N, O, and S, the C₁₋₄ alkyl, C₃₋₆ cycloalkyl, 3- to 6-membered heterocycloalkyl, phenyl, or 5- to 6-membered heteroaryl being optionally and independently substituted with 1, 2, or 3 R^{b}.

In some embodiments of the present disclosure, R² is selected from the group consisting of methyl, ethyl, *n*-propyl, isopropyl, *n*-butyl, isobutyl, *sec*-butyl, *tert*-butyl, cyclopropyl, cyclobutyl, cyclopentyl, cyclohexyl, oxiranyl, thiiranyl, aziridinyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, isoxazolidinyl, piperidinyl, piperazinyl, morpholinyl, phenyl, pyrrolyl, pyrazolyl, imidazolyl, oxazolyl, isoxazolyl, thiazole ring, isothiazolyl, triazolyl, furanyl, thienyl, pyridinyl, pyrazinyl, pyrimidinyl, and pyridazinyl, and R² is optionally and independently substituted with 1, 2, or 3 R^{b}.

In some embodiments of the present disclosure, R² is selected from the group consisting of *n*-propyl, isobutyl, cyclobutyl, cyclopentyl, cyclohexyl, tetrahydrofuranyl, and piperidinyl, and R² is optionally and independently substituted with 1, 2, or 3 R^{b}. In some other embodiments of the present disclosure, R² is selected from the group consisting of cyclobutyl, cyclohexyl, and piperidinyl, and R² is optionally and independently substituted with 1, 2, or 3 R^{b}.

In some embodiments of the present disclosure, R² is selected from piperidinyl, the piperidinyl being optionally and independently substituted with 1, 2, or 3 R^{b}.

In some embodiments of the present disclosure, each R^{b} is independently selected from the group consisting of OH, CN, NH₂, halogen, =O, C₁₋₃ alkyl, C₁₋₃ alkoxy, C₁₋₃ alkylthio, -NH(C₁₋₃ alkyl), -N(C₁₋₃ alkyl)₂, C₃₋₆ cycloalkyl, and 3- to 6-membered heterocycloalkyl, the C₁₋₃ alkyl, C₁₋₃ alkoxy, C₁₋₃ alkylthio, -NH(C₁₋₃ alkyl), or -N(C₁₋₃ alkyl)₂ being optionally and independently substituted with 1, 2, or 3 halogens, OH, NH₂, or CN, and the C₃₋₆ cycloalkyl or 3- to 6-membered heterocycloalkyl being optionally and independently substituted with 1, 2, or 3 R^{d}.

In some embodiments of the present disclosure, each R^{b} is independently selected from the group consisting of OH, CN, NH₂, halogen, =O, methyl, ethyl, isopropyl, methoxy, ethoxy, methylthio, ethylthio, -NHCH₃, -N(CH₃)₂, cyclopropyl, cyclobutyl, oxiranyl, thiiranyl, aziridinyl, oxetanyl, thietanyl, and azetidinyl, the methyl, ethyl, isopropyl, methoxy, ethoxy, methylthio, ethylthio, -NHCH₃, or -N(CH₃)₂ being optionally and independently substituted with 1, 2, or 3 halogens, OH, NH₂, or CN, and the cyclopropyl, cyclobutyl, oxiranyl, thiiranyl, aziridinyl, oxetanyl, thietanyl, or azetidinyl being optionally and independently substituted with 1, 2, or 3 R^{e}. In some other embodiments of the present disclosure, each R^{b} is independently selected from the group consisting of -S(=O)₂-CH₃, -S(=O)₂-CH₂CH₃, -S(=O)₂-NH₂, -S(=O)₂-NH-CH₃, -S(=O)₂-NH-CH₂CH₃, -S(=O)₂-cyclopropyl, -S(=O)₂-NH-cyclopropyl, -S(=O)(NH)-CH₃, -S(=O)(NH)-CH₂CH₃, and -S(=O)(NH)-cyclopropyl, the -S(=O)₂-CH₃, -S(=O)₂-CH₂CH₃, -S(=O)₂-NH-CH₃, -S(=O)₂-NH-CH₂CH₃, -S(=O)(NH)-CH₃, or -S(=O)(NH)-CH₂CH₃ being optionally and independently substituted with 1, 2, or 3 halogens, OH, NH₂, or CN, and the -S(=O)₂-cyclopropyl, -S(=O)₂-NH-cyclopropyl, or -S(=O)(NH)-cyclopropyl being optionally and independently substituted with 1, 2, or 3 R^{e}.

In some embodiments of the present disclosure, each R^{b} is independently selected from the group consisting of F, Cl, Br, I, OH, CN, NH₂, methyl, and methoxy, the methyl or methoxy being optionally and independently substituted with 1, 2, or 3 halogens, OH, NH₂, or CN. In some other embodiments of the present disclosure, each R^{b} is independently selected from the group consisting of ethyl, -S(=O)₂-CH₃, -S(=O)₂-NH₂, -S(=O)₂-cyclopropyl, and -S(=O)(NH)-CH₃, the ethyl, -S(=O)₂-CH₃, -S(=O)(NH)-CH₃, or -S(=O)₂-cyclopropyl being optionally and independently substituted with 1, 2, or 3 halogens, OH, NH₂, or CN.

In some embodiments of the present disclosure, each R^{b} is independently selected from the group consisting of F, Cl, Br, I, OH, CN, NH₂, methyl, and trifluoromethyl. In some other embodiments of the present disclosure, each R^{b} is independently selected from the group consisting of ethyl, 2-hydroxyethyl, -S(=O)₂-CH₃, -S(=O)₂-NH₂, -S(=O)₂-cyclopropyl, and -S(=O)(NH)-CH₃.

In some embodiments of the present disclosure, each R^{b} is independently selected from the group consisting of F, Cl, OH, methyl, and trifluoromethyl.

In some embodiments of the present disclosure, each R^{b} is independently selected from the group consisting of OH and methyl.

In some other embodiments of the present disclosure, each R^{b} is independently selected from the group consisting of OH, methyl, ethyl, 2-hydroxyethyl, -S(=O)₂-CH₃, -S(=O)₂-NH₂, -S(=O)₂-cyclopropyl, and -S(=O)(NH)-CH₃.

In some other embodiments of the present disclosure, each R^{b} is independently selected from the group consisting of OH, methyl, and ethyl.

In some embodiments of the present disclosure, each R^{e} is independently selected from the group consisting of F, Cl, OH, methyl, and trifluoromethyl.

In some embodiments of the present disclosure, R² is selected from the group consisting of 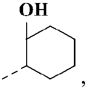 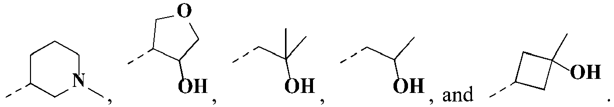 In some other embodiments of the present disclosure, R² is selected from the group consisting of

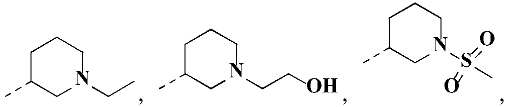

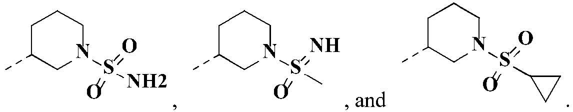

In some embodiments of the present disclosure, R² is selected from

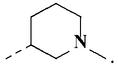

In some embodiments of the present disclosure, R² is a group in a single enantiomeric form, in a form enriched in one enantiomer, or in a racemic form.

In some embodiments of the present disclosure, R² is selected from

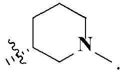

In some embodiments of the present disclosure, m is selected from the group consisting of 0 and 1.

In some embodiments of the present disclosure, i is selected from the group consisting of 0 and 1.

In some embodiments of the present disclosure, the compound of formula (I) or the pharmaceutically acceptable salt thereof is selected from the group consisting of a compound of formula (I-1a) and a compound of formula (I-1b), and pharmaceutically acceptable salts thereof: 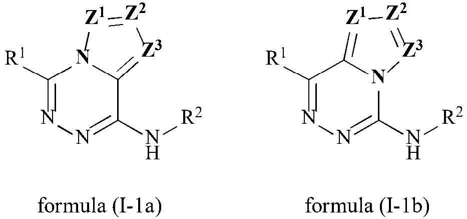 wherein
R¹, R², Z¹, Z², and Z³ are as defined in the compound of formula (I).

In some embodiments of the present disclosure, the compound of formula (I) or the pharmaceutically acceptable salt thereof is selected from the group consisting of a compound of formula (I-2a), a compound of formula (I-2b), a compound of formula (I-2c), and a compound of formula (I-2c), and pharmaceutically acceptable salts thereof: 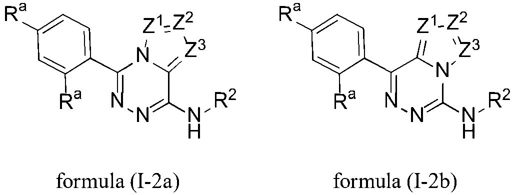 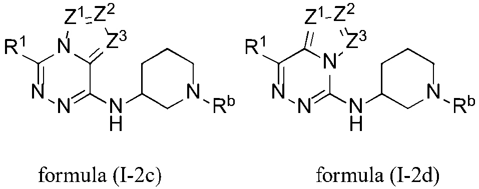 wherein
R¹, R², R^{a}, R^{b}, Z¹, Z², and Z³ are as defined in the compound of formula (I).

In some embodiments of the present disclosure, the compound of formula (I) or the pharmaceutically acceptable salt thereof is selected from the group consisting of a compound of formula (I-2c-1), a compound of formula (I-2c-2), a compound of formula (I-2d-1), and a compound of formula (I-2d-2), and pharmaceutically acceptable salts thereof: 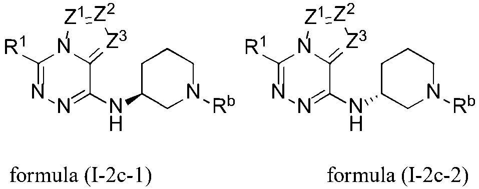 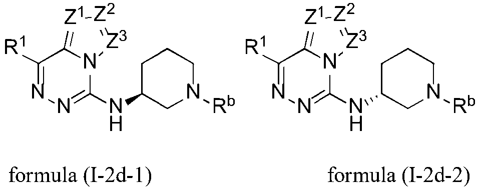 wherein
R¹, R^{b}, Z¹, Z², and Z³ are as defined in the compound of formula (I).

In some embodiments of the present disclosure, the compound of formula (I) or the pharmaceutically acceptable salt thereof is selected from the group consisting of a compound of formula (I-3a) and a compound of formula (I-3b), and pharmaceutically acceptable salts thereof: 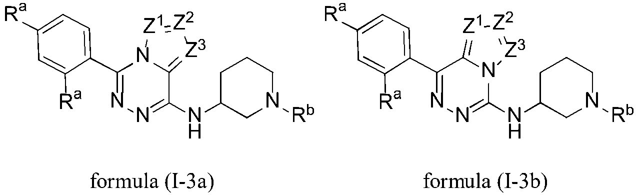 wherein
R^{a}, R^{b}, Z¹, Z², and Z³ are as defined in the compound of formula (I).

In some embodiments of the present disclosure, the compound of formula (I) or the pharmaceutically acceptable salt thereof is selected from the group consisting of a compound of formula (I-3a-1), a compound of formula (I-3a-2), a compound of formula (I-3b-1), and a compound of formula (I-3b-2), and pharmaceutically acceptable salts thereof: 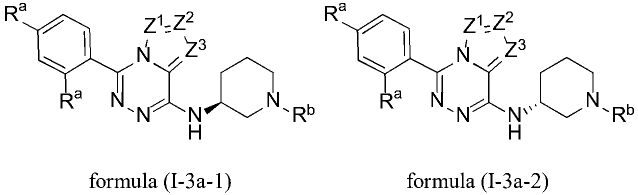 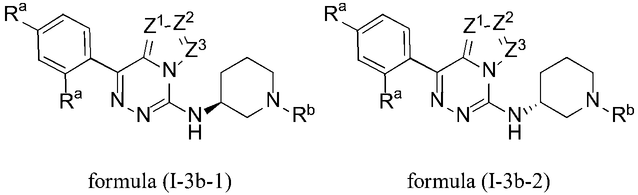 wherein
R^{a}, R^{b}, Z¹, Z², and Z³ are as defined in the compound of formula (I).

Some other embodiments of the present disclosure are derived from any combination of the variables.

The present disclosure further provides a compound of a formula selected from the group consisting of the following or a pharmaceutically acceptable salt thereof:

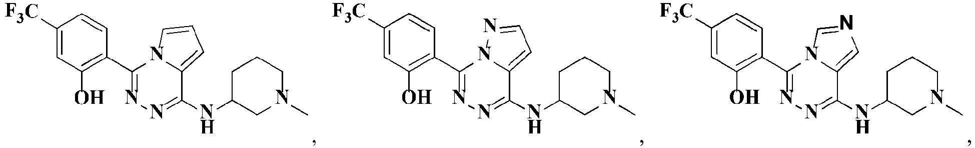

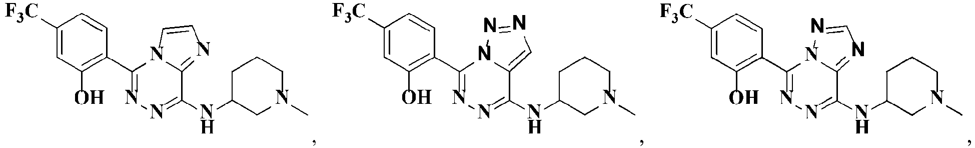

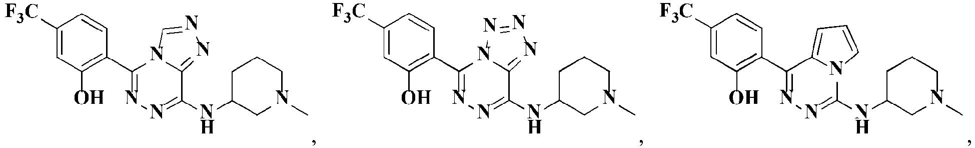

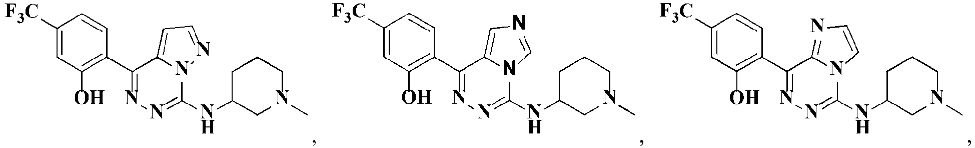

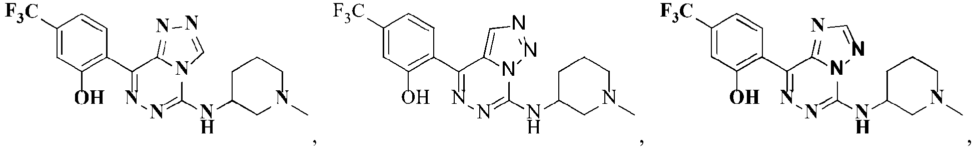

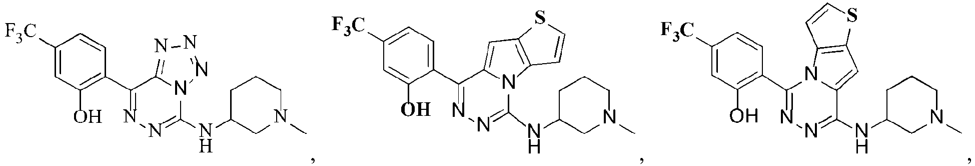

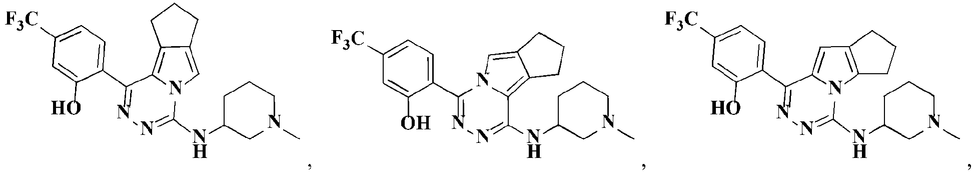

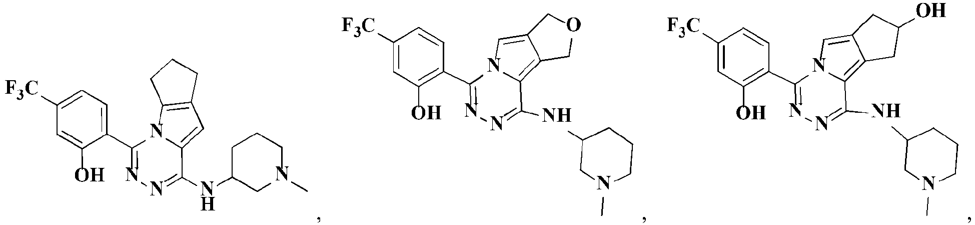

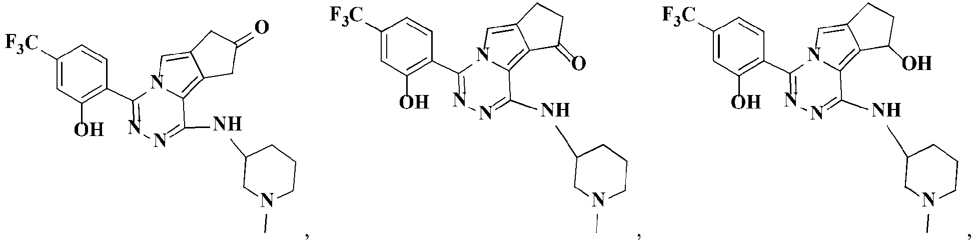

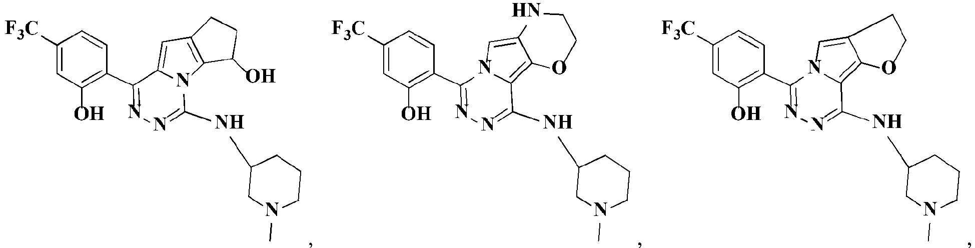

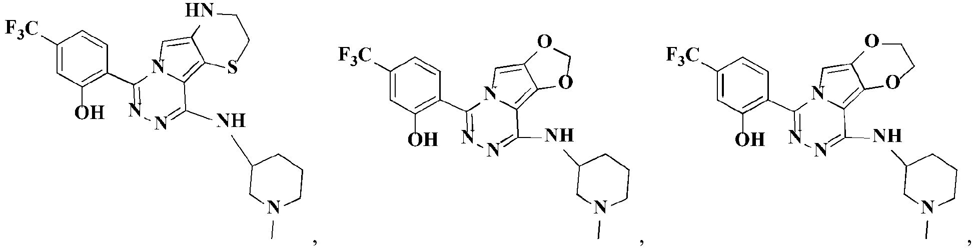

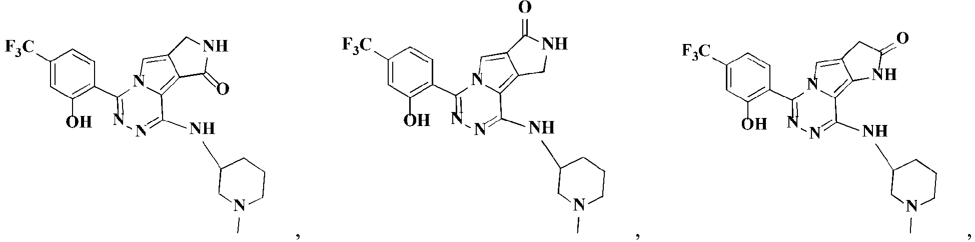

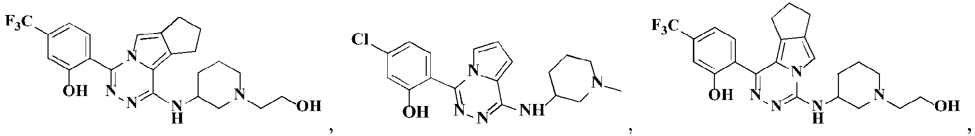

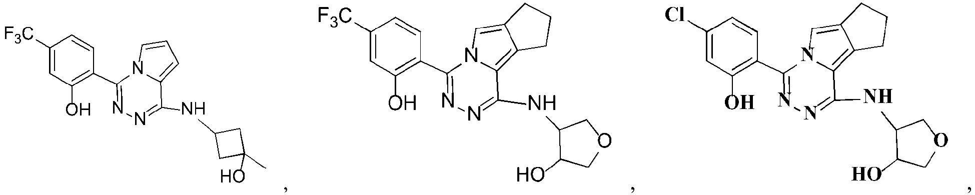

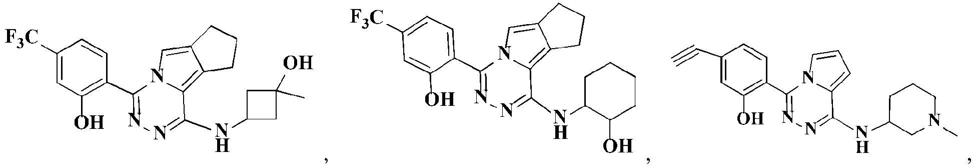

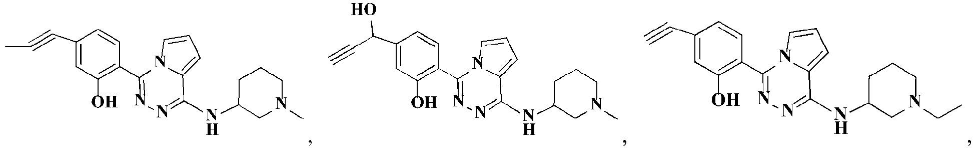

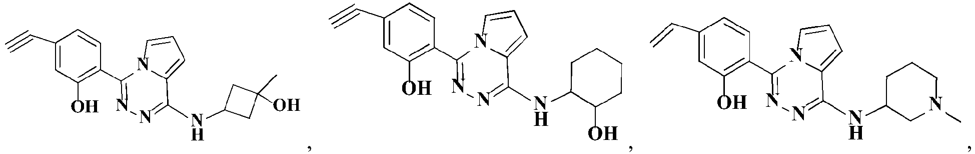

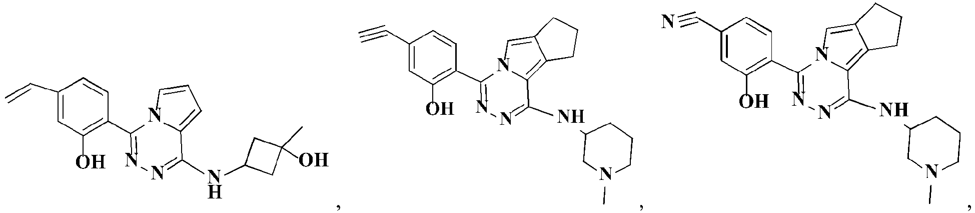

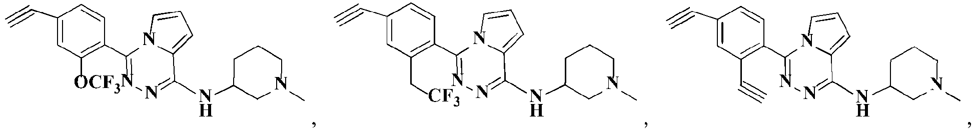

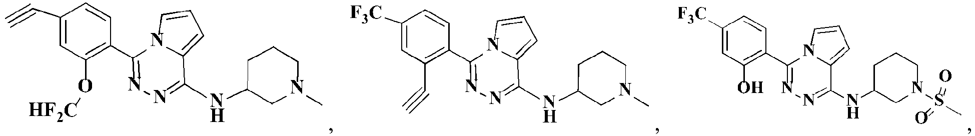

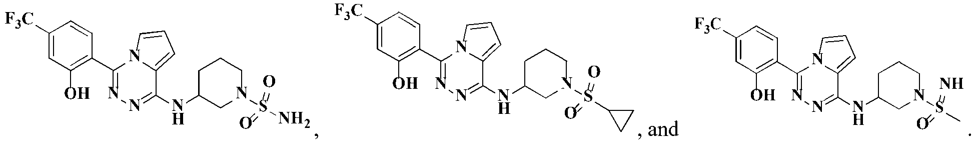

In some embodiments of the present disclosure, the compound described in the present disclosure is a compound in a single enantiomeric form, in a form enriched in one enantiomer, or in a racemic form.

The present disclosure further provides a compound of a formula selected from the group consisting of the following or a pharmaceutically acceptable salt thereof:

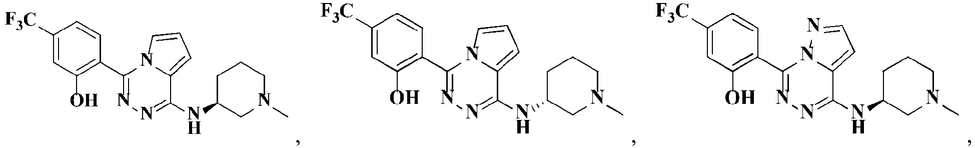

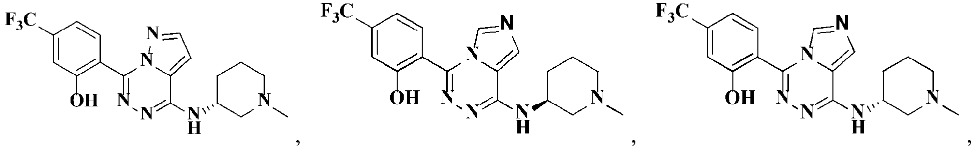

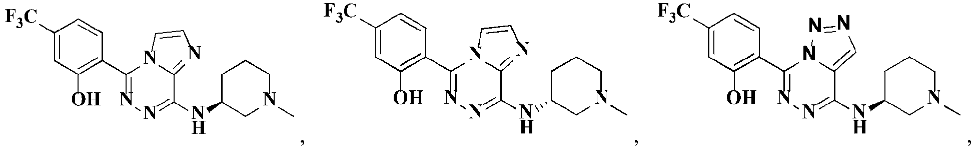

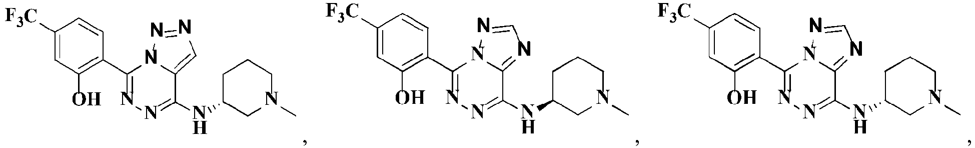

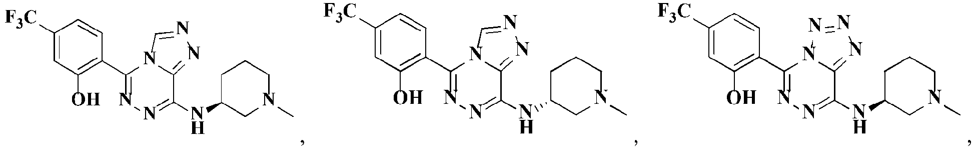

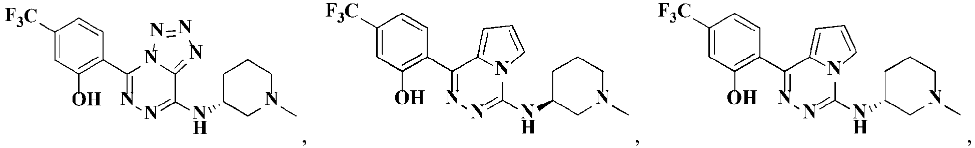

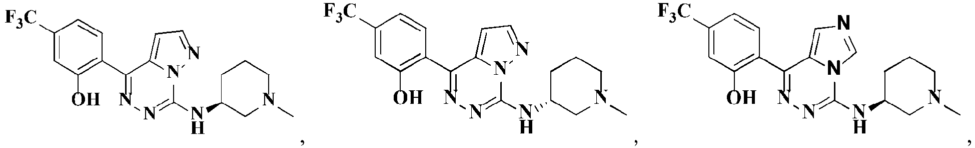

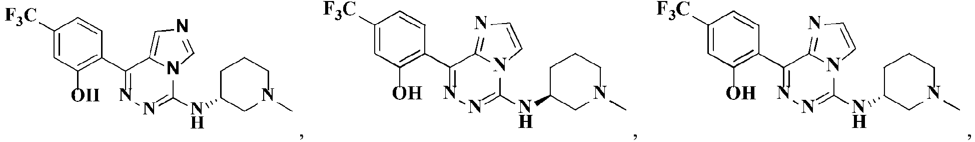

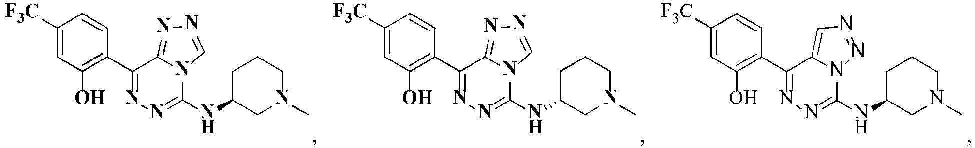

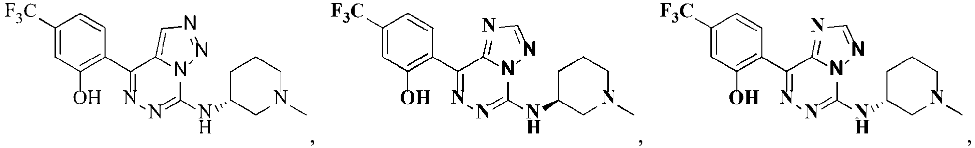

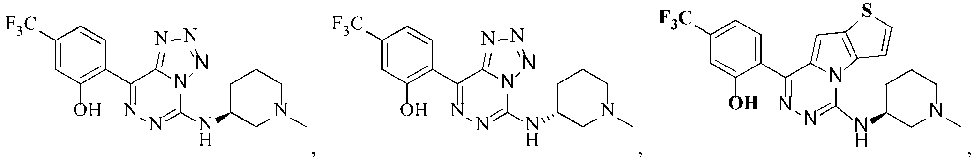

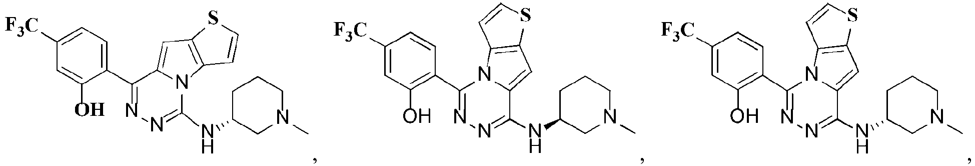

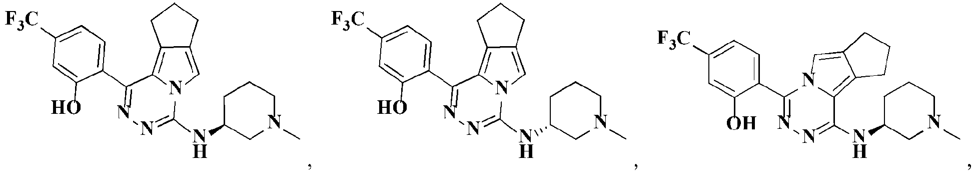

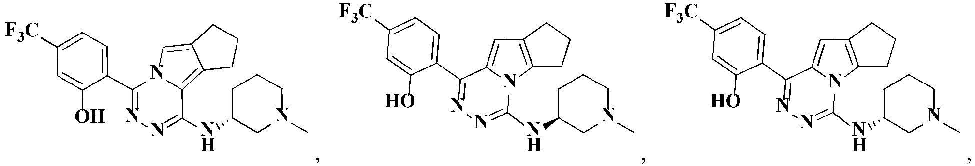

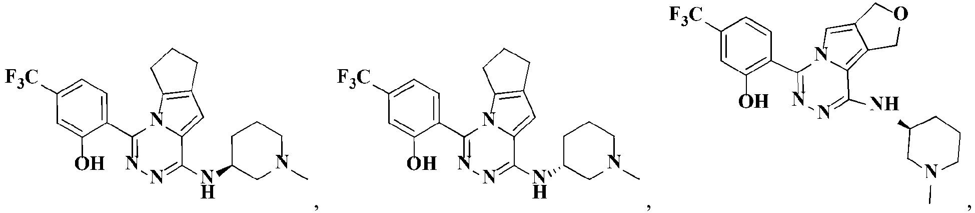

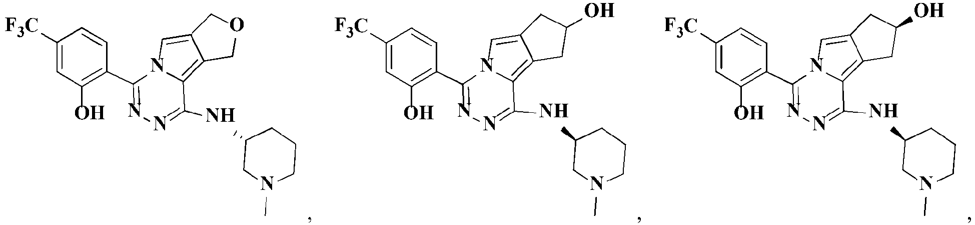

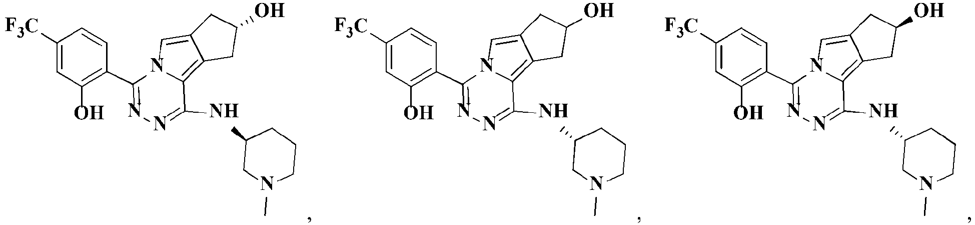

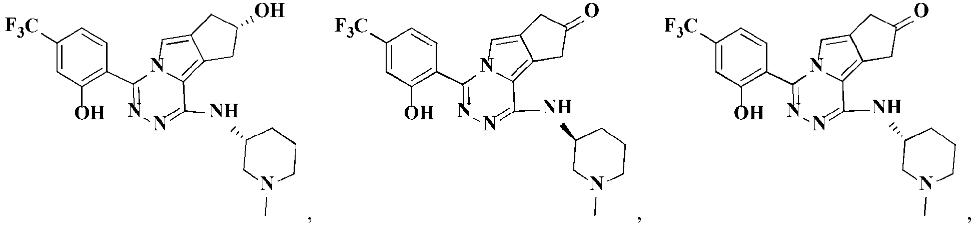

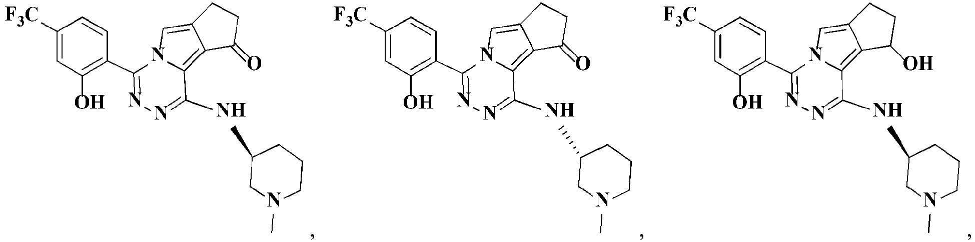

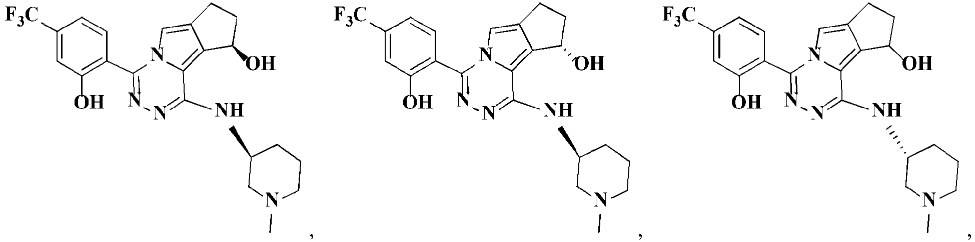

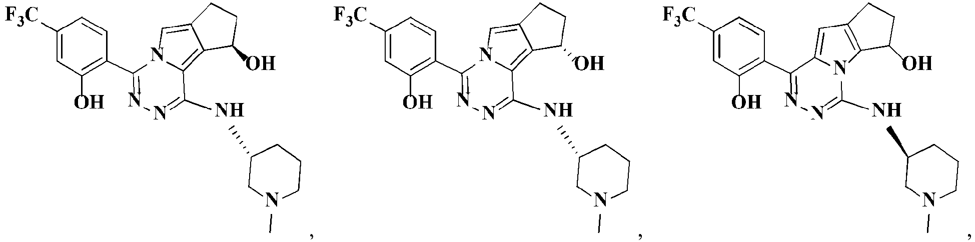

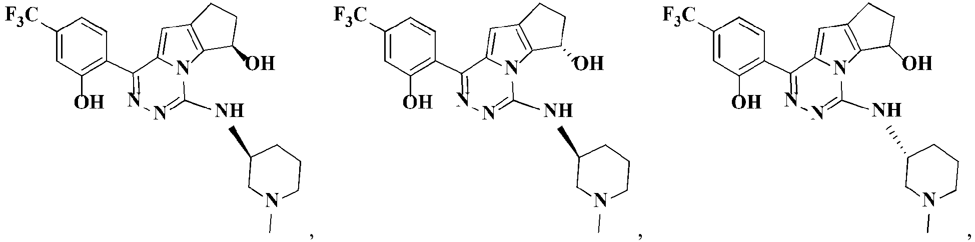

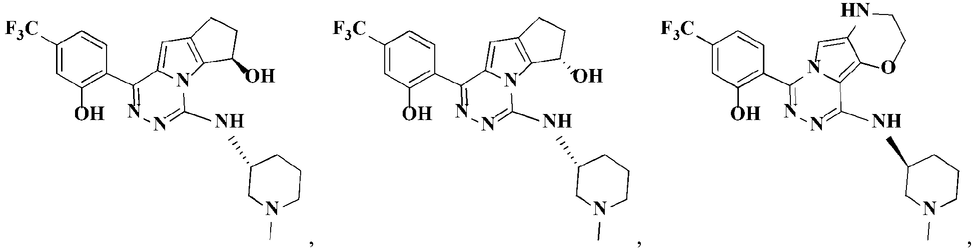

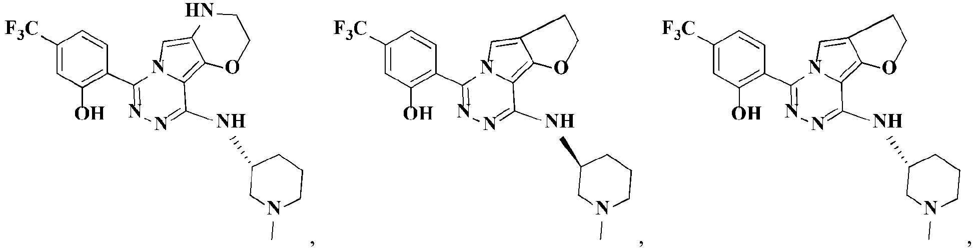

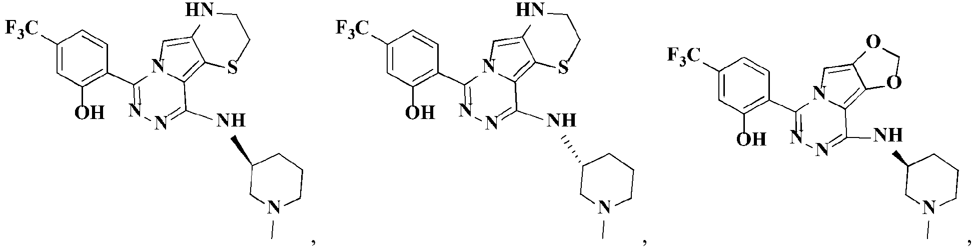

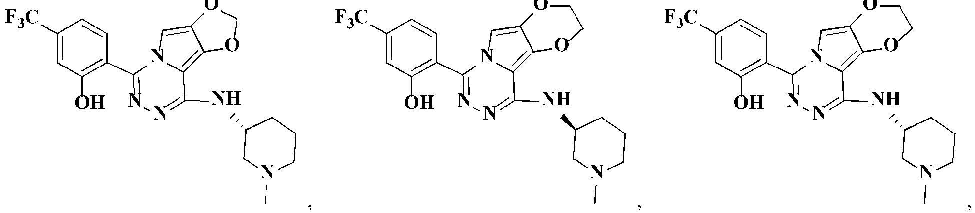

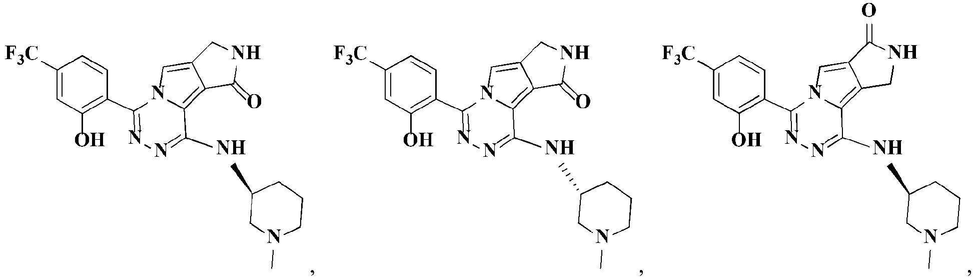

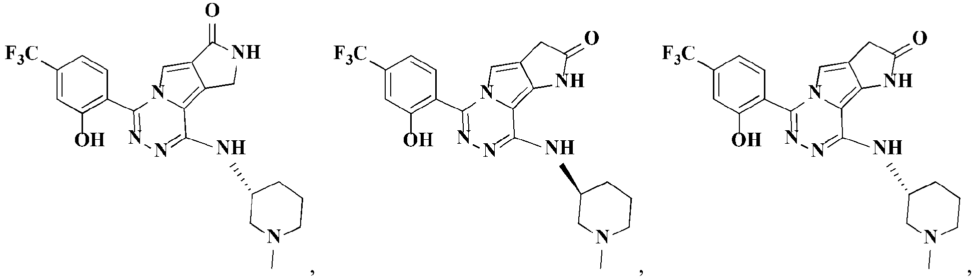

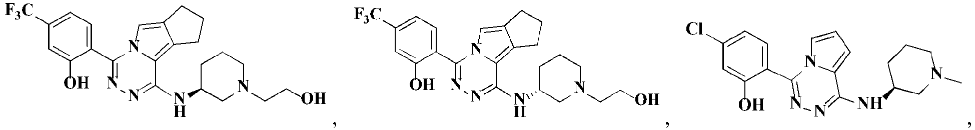

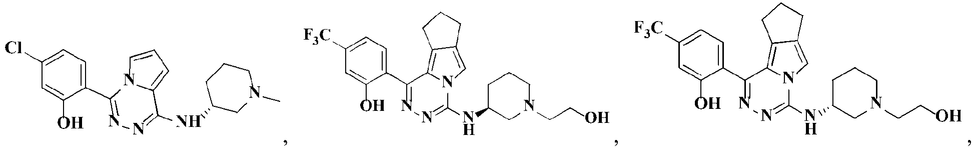

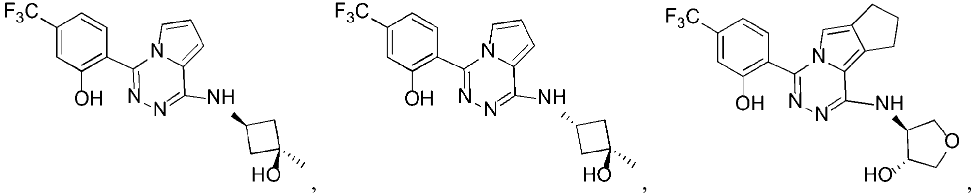

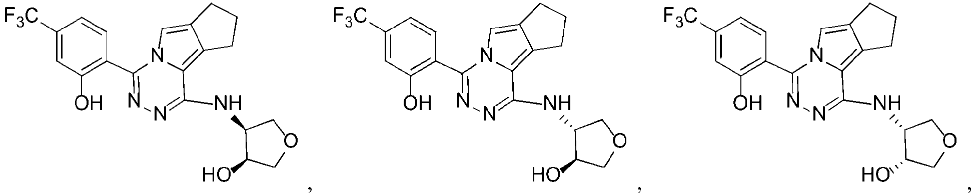

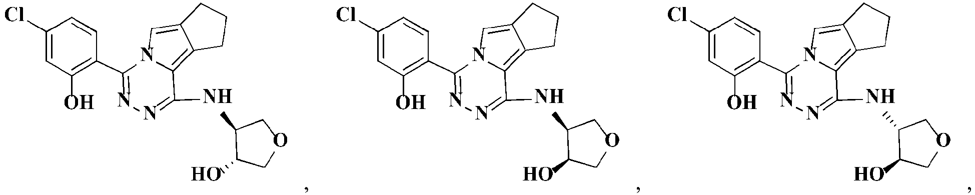

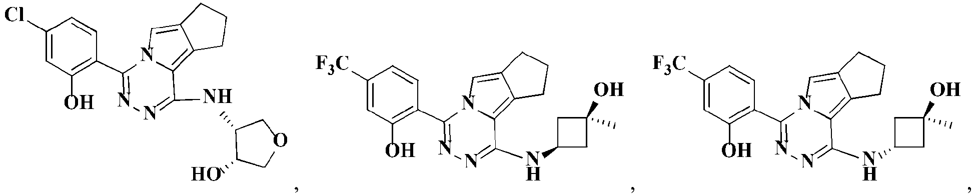

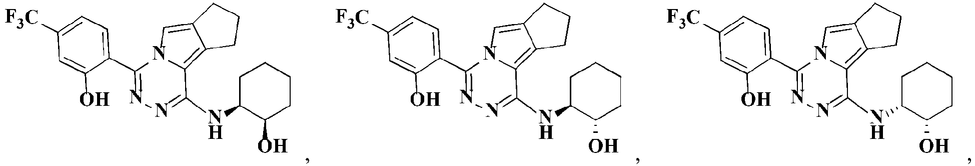

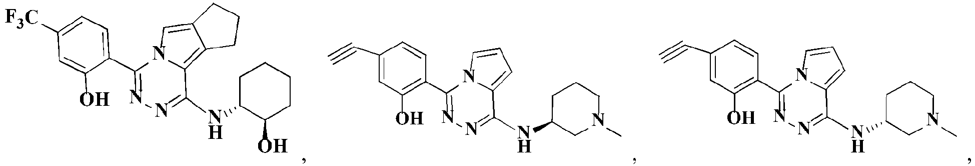

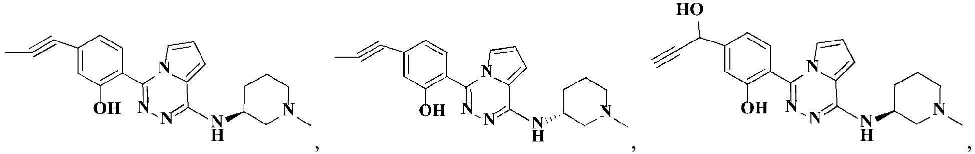

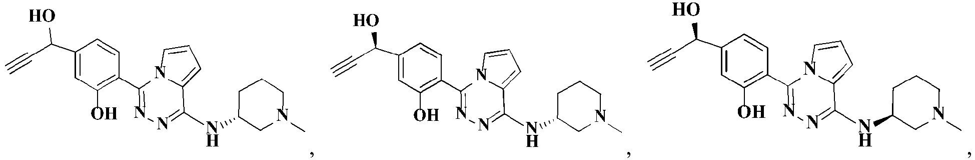

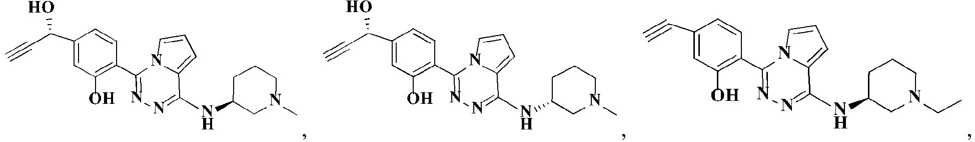

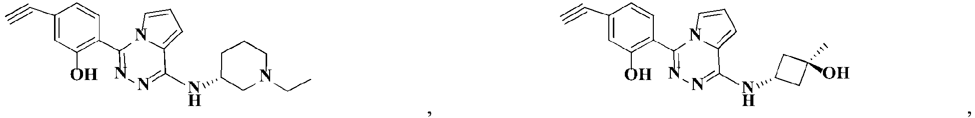

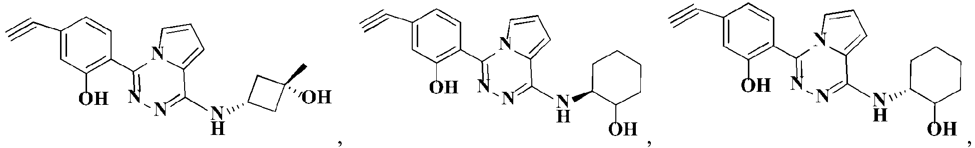

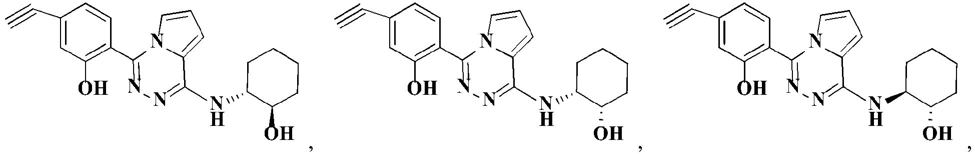

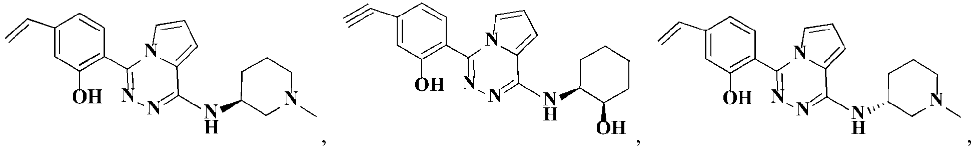

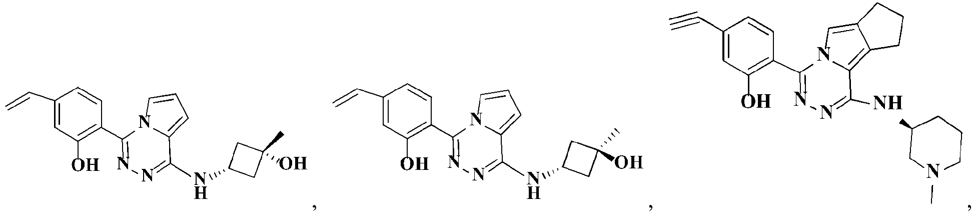

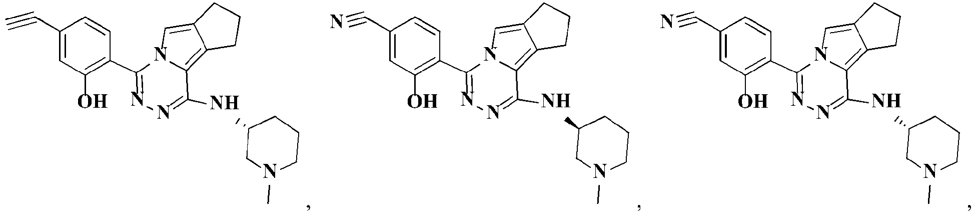

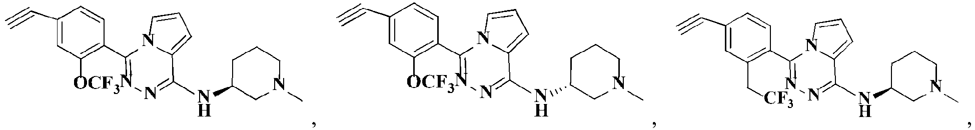

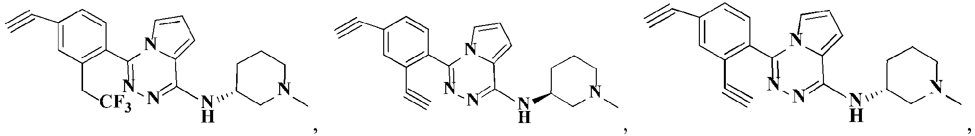

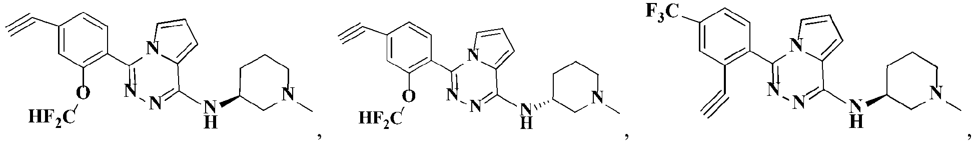

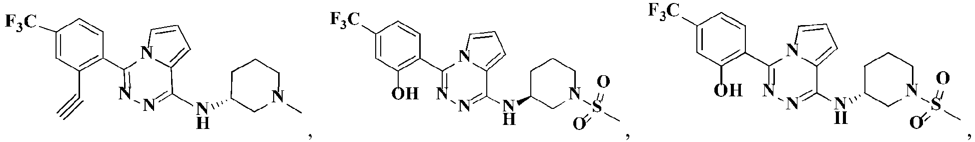

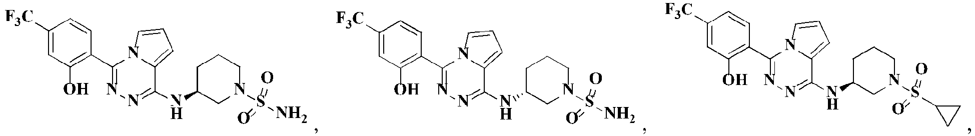

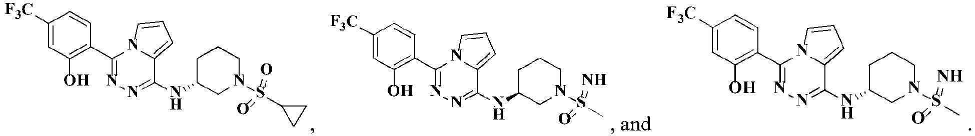

The present disclosure further provides a pharmaceutical composition, which comprises a therapeutically effective amount of the compound or the pharmaceutically acceptable salt thereof disclosed herein. In some embodiments, the pharmaceutical composition of the present disclosure further comprises a pharmaceutically acceptable carrier. The present disclosure further provides use of the compound or the pharmaceutically acceptable salt thereof disclosed herein for preparing a medicament for treating or preventing a disease (e.g., an NLRP3 inflammasome-mediated disease).

The present disclosure further provides a method for treating or preventing a disease (e.g., an NLRP3 inflammasome-mediated disease), which comprises administering to a mammal (preferably a human) in need of such treatment or prevention a therapeutically or prophylactically effective amount of the compound or the pharmaceutically acceptable salt thereof disclosed herein.

The present disclosure further provides use of the compound or the pharmaceutically acceptable salt thereof disclosed herein for treating or preventing a disease (e.g., an NLRP3 inflammasome-mediated disease).

The present disclosure further provides the compound or the pharmaceutically acceptable salt thereof disclosed herein for use in the treatment or prevention of a disease (e.g., an NLRP3 inflammasome-mediated disease).

In some embodiments of the present disclosure, the NLRP3 inflammasome-mediated disease is selected from the group consisting of: an inflammasome-related disease/disorder and an inflammatory disease.

### Technical effects

The compound in the present disclosure, as an NLRP3 inflammasome inhibitor with a novel structure, exhibits a good inhibitory effect on NLRP3 inflammasome-induced signaling, demonstrates relatively good anti-pyroptosis activity in J774A.1 cells, shows good inhibitory activity against IL-1β expression in THP-1 cells, and has relatively good *in-vivo* inhibitory activity. The compound of the present disclosure has good pharmacokinetic properties, and can be developed into a novel NLRP3 inflammasome inhibitor drug.

### Definitions

Unless otherwise stated, the following terms and phrases used herein are intended to have the following meanings. A particular term or phrase, unless otherwise specifically defined, should not be considered indefinite or unclear, but construed according to its common meaning. When referring to a trade name, it is intended to refer to its corresponding commercial product or its active ingredient.

The term "pharmaceutically acceptable" is used herein for those compounds, materials, compositions, and/or dosage forms that are, within the scope of sound medical judgment, suitable for use in contact with the tissues of humans and animals without excessive toxicity, irritation, allergic response, or other problems or complications, and commensurate with a reasonable benefit/risk ratio.

The term "pharmaceutically acceptable salt" refers to a salt of the compound of the present disclosure, which is prepared from the compound having particular substituents discovered by the present disclosure and a relatively nontoxic acid or base. When the compound of the present disclosure contains a relatively acidic functional group, a base addition salt can be obtained by allowing such a compound to be in contact with a sufficient amount of a base in a pure solution or a suitable inert solvent. When the compound of the present disclosure contains a relatively basic functional group, an acid addition salt can be obtained by allowing such a compound to be in contact with a sufficient amount of an acid in a pure solution or a suitable inert solvent. Certain particular compounds of the present disclosure contain both basic and acidic functional groups that allow the compounds to be converted into either base or acid addition salts.

The pharmaceutically acceptable salt of the present disclosure can be synthesized from the parent compound containing an acid radical or a basic group by conventional chemical methods. In general, such salts are prepared by subjecting the free acid or base form of the compound to a reaction with a stoichiometric amount of the appropriate base or acid in water or an organic solvent or a mixture thereof.

The compound of the present disclosure may exist in the form of a particular geometric isomer or stereoisomer.

All such compounds are contemplated herein, including *cis* and *trans* isomers, (-)- and (+)-enantiomers, (R)- and (S)-enantiomers, diastereoisomers, (D)-isomers, (L)-isomers, and racemic mixtures and other mixtures thereof, such as a mixture enriched in enantiomer or diastereoisomer, all of which are encompassed within the scope of the present disclosure. The substituents such as alkyl may have an additional asymmetric carbon atom. All these isomers and mixtures thereof are encompassed within the scope of the present disclosure.

Unless otherwise stated, for compounds having one or more stereoisomers, the range in which the valence bond at the chiral center is represented by a solid bond ( 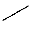 ) encompasses all compounds in a single enantiomeric form, in a form enriched in one enantiomer, or in a racemic form.

Unless otherwise stated, the absolute configuration of a stereogenic center is represented by a wedged solid bond ( 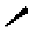 ) and a wedged dashed bond ( 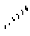 ), and the relative configuration of a stereogenic center is represented by a straight solid bond ( 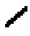 ) and a straight dashed bond ( 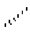 ). A wavy line ( 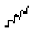 ) represents a wedged solid bond ( 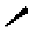 ) or a wedged dashed bond ( 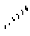 ), or a wavy line ( 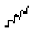 ) represents a straight solid bond ( 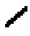 ) and a straight dashed bond ( 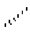 ).

Unless otherwise stated, when a double bond structure such as a carbon-carbon double bond, a carbon-nitrogen double bond, and a nitrogen-nitrogen double bond is present in the compound, and each atom on the double bond is linked to two different substituents (in the double bond including a nitrogen atom, a lone pair of electrons on the nitrogen atom is regarded as a substituent to which the nitrogen atom is linked), if the atom on the double bond of the compound and its substituents are linked using a wavy line ( 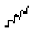 ), it means that the compound exists in the form of a (Z)-type isomer, an (E)-type isomer, or a mixture of the two isomers.

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerization and imine-enamine isomerization. A specific example of a proton tautomer is an imidazole moiety where a proton can transfer between two ring nitrogen atoms. A valence tautomer includes the interconversion via recombination of some bonding electrons.

The compound of the present disclosure may contain an unnatural proportion of atomic isotope at one or more of the atoms that constitute the compound. For example, the compound may be labeled with a radioisotope, such as tritium (³H), iodine-125 (¹²⁵I), or C-14 (¹⁴C). For another example, hydrogen can be substituted by deuterium to form a deuterated drug, and the bond formed by deuterium and carbon is firmer than that formed by common hydrogen and carbon. Compared with an un-deuterated drug, the deuterated drug has the advantages of reduced toxic and side effects, increased stability, enhanced efficacy, prolonged biological half-life, and the like. All isotopic variations of the compound of the present disclosure, whether radioactive or not, are encompassed within the scope of the present disclosure. For example, it should be understood that compounds formed by replacing one or more hydrogen atoms in the compound of formula (I) of the present disclosure with a deuterium atom still fall within the scope of the compound of formula (I) disclosed herein.

The term "optional" or "optionally" means that the subsequently described event or circumstance may, but not necessarily, occur, and the description includes instances where the event or circumstance occurs and instances where it does not.

The term "substituted" means that one or more hydrogen atoms on a specific atom are substituted by substituents, wherein the substituents may include deuterium and hydrogen variants, as long as the valence of the specific atom is normal and the compound after substitution is stable. When the substituent is oxygen (i.e., =O), it means that two hydrogen atoms are substituted. Substitution with oxygen does not occur on aromatic groups. The term "optionally substituted" means that an atom may or may not be substituted with a substituent. Unless otherwise specified, the type and number of the substituent may be arbitrary as long as being chemically achievable.

The term "substituted with one or more substituents" means that any one or more hydrogen atoms on a specific atom are substituted by substituents, wherein the substituents may include deuterium and hydrogen variants, and the number of the substituents includes 1, 2, 3, 4, 5, 6, 7, 8, 9, or 10 as long as being chemically achievable.

The "substituent" described herein includes, but is not limited to, the terms mentioned in context including "alkyl", "alkoxy", "alkylthio", "alkenyl", "alkynyl", "cycloalkyl", "partially saturated cycloalkyl", "heterocycloalkyl", "partially saturated heterocycloalkyl", "heteroaryl", "aryl", etc., and the corresponding non-limiting or exemplary groups, wherein some non-limiting examples of the "substituent" include protium, deuterium, tritium, -OH, -SH, halogen, -NH₂, nitro, nitroso, -CN, azido, a sulfoxide group, a sulfone group, a sulfonamide group, carboxyl, a carboxaldehyde group, an imine group, alkyl, halogenated alkyl, cycloalkyl, halogenated cycloalkyl, alkenyl, halogenated alkenyl, cycloalkenyl, halogenated cycloalkenyl, alkynyl, halogenated alkynyl, cycloalkynyl, halogenated cycloalkynyl, heteroalkyl, halogenated heteroalkyl, alkoxy, alkylthio, aryl, aryloxy, arylthio, aralkyl, arylalkoxy, arylalkylthio, heteroaryl, heteroaryloxy, heteroarylthio, heteroaralkyl, heteroarylalkoxy, heteroarylalkylthio, heterocyclyl, heterocyclyloxy, heterocyclylthio, heterocyclylalkyl, heterocyclylalkoxy, heterocyclylalkylthio, acyl, acyloxy, a carbamate group, an amide group, ureido, an epoxy group, an ester group, and the like, wherein the groups are optionally substituted with one or more substituents selected from the group consisting of the following substituents: oxo, hydroxy, amino, nitro, halogen, cyano, alkyl, alkenyl, alkynyl, alkoxy, halogenated alkoxy, alkylamino, dialkylamino, halogenated alkylamino, halogenated dialkylamino, carboxy, -C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkyl, cycloalkyloxy, heterocyclyl, heterocyclylalkyl, heterocyclyloxy, heterocycloalkyl, heterocycloalkylalkyl, heterocycloalkyloxy, heteroaryl, heteroarylalkyl, heteroaryloxy, aryl, arylalkyl, and aryloxy.

In some embodiments herein, the "substituent" is selected from the group consisting of deuterium, tritium, hydroxyl, sulfydryl, halogen, amino, nitro, nitroso, cyano, azido, a sulfoxide group, a sulfone group, a sulfonamide group, carboxyl, an aldehyde group, an imine group, C₁₋₁₂ alkyl, halogenated C₁₋₁₂ alkyl, 3- to 12-membered cycloalkyl, halogenated 3- to 12-membered cycloalkyl, C₂₋₁₂ alkenyl, halogenated C₂₋₁₂ alkenyl, 3-to 12-membered cycloalkenyl, halogenated 3- to 12-membered cycloalkenyl, C₂₋₁₂ alkynyl, halogenated C₂₋₁₂ alkynyl, 8- to 12-membered cycloalkynyl, halogenated 8- to 12-membered cycloalkynyl, C₁₋₁₂ heteroalkyl, halogenated C₁₋₁₂ heteroalkyl, C₁₋₁₂ alkoxy, C₁₋₁₂ alkylthio, 6- to 10-membered aryl, 6- to 10-membered aryloxy, 6- to 10-membered arylthio, 6- to 10-membered aryl C₁₋₁₂ alkylene, 6- to 10-membered aryl C₁₋₁₂ alkoxy, 6- to 10-membered aryl C_{I-12} alkylthio, 5- to 10-membered heteroaryl, 5- to 10-membered heteroaryloxy, 5- to 10-membered heteroarylthio, 5- to 10-membered heteroarylalkylene, 5- to 10-membered heteroarylalkoxy, 5- to 10-membered heteroarylalkylthio, 3- to 12-membered heterocyclyl, 3- to 12-membered heterocyclyloxy, 3- to 12-membered heterocyclylthio, 3- to 12-membered heterocyclyl C₁₋₁₂ alkylene, 3-to 12-membered heterocyclyl C₁₋₁₂ alkoxy, 3- to 12-membered heterocyclyl C_{I-12} alkylthio, C₁₋₁₂ acyl, C₁₋₁₂ acyloxy, a carbamate group, C₁₋₁₂ amido, ureido, an epoxy group, a C₂₋₁₂ ester group, and oxo, wherein these substituents are optionally substituted with one or more substituents selected from the group consisting of the following substituents: oxo, hydroxy, amino, nitro, halogen, cyano, C_{I-12} alkyl, C₂₋₁₂ alkenyl, C₂₋₁₂ alkynyl, C₁₋₁₂ alkoxy, halogenated C₁₋₁₂ alkoxy, C₁₋₁₂ alkylamino, di-C₁₋₁₂ alkylamino, halogenated C₁₋₁₂ alkylamino, halogenated di-C₁₋₁₂ alkylamino, carboxyl, -C(O)O-C₁₋₁₂ alkyl, -OC(O)-C₁₋₁₂ alkyl, -C(O)NH₂, -C(O)NH-C₁₋₁₂ alkyl, -C(O)N(C₁₋₁₂ alkyl)₂, -NHC(O)-C₁₋₁₂ alkyl, -C(O)-C₁₋₁₂ alkyl, -S(O)-C₁₋₁₂ alkyl, -S(O)₂-C₁₋₁₂ alkyl, -S(O)₂NH₂, -S(O)₂NH-C₁₋₁₂ alkyl, -S(O)₂N(C₁₋₁₂ alkyl)₂, 3- to 12-membered cycloalkyl, 3- to 12-membered cycloalkyl C₁₋₁₂ alkylene, 3- to 12-membered cycloalkyloxy, 3- to 12-membered heterocyclyl, 3- to 12-membered heterocyclyl C₁₋₁₂ alkylene, 3- to 12-membered heterocyclyloxy, 3- to 12-membered heterocycloalkyl, 3- to 12-membered heterocycloalkyl C₁₋₁₂ alkylene, 3- to 12-membered heterocycloalkyloxy, 5- to 10-membered heteroaryl, 5- to 10-membered heteroaryl C_{I-12} alkylene, 5- to 10-membered heteroaryloxy, 6- to 10-membered aryl, 6- to 10-membered aryl C₁₋₁₂ alkylene, and 6- to 10-membered aryloxy. When any variable (e.g., R) occurs more than once in the constitution or structure of a compound, the definition of the variable in each case is independent. Thus, for example, if a group is substituted with 0-2 R, the group can be optionally substituted with up to two R, and the definition of R in each case is independent. Furthermore, a combination of a substituent and/or a variant thereof is permissible only if the combination can result in a stable compound.

When one of the variables is selected from a single bond, it means that the two groups which it connects are connected directly. For example, in A-L-Z, when L represents a single bond, it means that the structure is actually A-Z.

When the direction for connection of the listed connecting group is not specified, the direction for connection is arbitrary. For example, when the connecting group L contained in 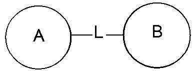 is -M-W-, -M-W- can either connect ring A and ring B in a direction same as left-to-right reading order to form 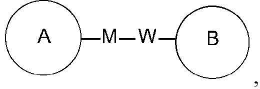 or connect ring A and ring B in a direction opposite to the left-to-right reading order to form 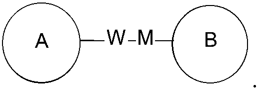 A combination of the connecting group, the substituent, and/or the variant thereof is permissible only if the combination can result in a stable compound.

When the direction for connection of the listed connecting group is not specified, the direction for connection is arbitrary. For example, when the R³ groups on Z¹ and Z², together with the atoms to which they are attached, form 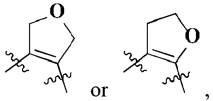 the structural unit 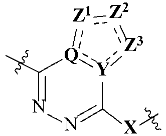 may be either

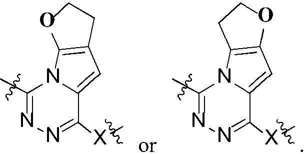

For example, when the R³ groups on Z² and Z³, together with the atoms to which they are attached, form 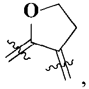 the structural unit 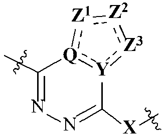 may be either

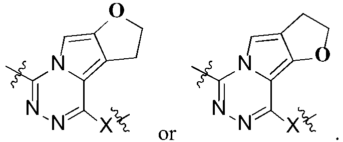

Unless otherwise specified, when a group has one or more connectable sites, any one or more of the sites of the group may be connected to other groups by chemical bonds. If there is no designated connecting mode for chemical bonds and H atoms are present at a connectable site, when the connectable site is connected to chemical bonds, the number of the H atoms at the connectable site is correspondingly reduced based on the number of the connected chemical bonds, resulting in a group with the corresponding valence. The chemical bond that connects the site and another group may be represented by a straight solid bond ( 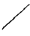 ), a straight dashed bond ( 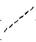 ), or a wavy line 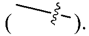 For example, the straight solid bond in -OCH₃ refers to being connected to another group via the oxygen atom in the group; the straight dashed bonds in 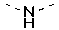 refer to being connected to other groups via two ends of the nitrogen atom in the group; the wavy lines in 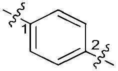 refer to being connected to other groups via the carbon atoms at positions 1 and 2 in the phenyl group; 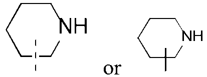 means that any connectable site on the piperidinyl can be connected to another group via 1 chemical bond in at least 4 connecting modes: 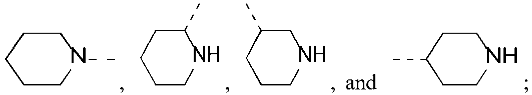 even if -N- is connected with an H atom, 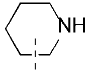 includes the connection mode of 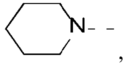 but when 1 chemical bond is connected to a site, the number of H at that site is correspondingly reduced by 1, resulting in a corresponding monovalent piperidinyl group.

Unless otherwise specified, the term "C₁₋₆ alkyl" refers to a linear or branched saturated hydrocarbon group consisting of 1 to 6 carbon atoms. The C₁₋₆ alkyl includes C₁₋₄ and C₁₋₃ alkyl, and the like, and may be monovalent (e.g., methyl), divalent (e.g., methylene), or polyvalent (e.g., methenyl). Examples of the C₁₋₃ alkyl include, but are not limited to, methyl (Me), ethyl (Et), propyl (including n-propyl and isopropyl), and the like.

Unless otherwise specified, the term "C₁₋₃ alkoxy" refers to those alkyl groups that each contain 1 to 3 carbon atoms and are linked to the rest part of the molecule via an oxygen atom. The C₁₋₃ alkoxy includes C₁₋₂, C₂₋₃, C₃ and C₂ alkoxy, and the like. Examples of the C₁₋₃ alkoxy include, but are not limited to, methoxy, ethoxy, propoxy (including n-propoxy and isopropoxy), and the like.

Unless otherwise specified, the term "C₁₋₃ alkylthio" refers to those alkyl groups that each contain 1 to 3 carbon atoms and are linked to the rest part of the molecule via a sulfur atom. The C₁₋₃ alkylthio includes C₁₋₂, C₂₋₃, C₃ and C₂ alkylthio, and the like. Examples of the C₁₋₃ alkylthio include, but are not limited to, methylthio, ethylthio, propylthio (including n-propylthio and isopropylthio), and the like.

Unless otherwise specified, the term "halogen", by itself or as part of another substituent, refers to a fluorine, chlorine, bromine, or iodine atom.

Unless otherwise specified, Cₙ₋ₙ₊ₘ or Cₙ-Cₙ₊ₘ includes any one of the specific cases of n to n+m carbon atoms.

For example, C₁₋₁₂ includes C₁, C₂, C₃, C₄, C₅, C₆, C₇, C₈, C₉, C₁₀, C₁₁, and C₁₂. Also, any range within n to n+m may be included. For example, C₁₋₁₂ includes C₁₋₃, C₁₋₆, C₁₋₉, C₃₋₆, C₃₋₉, C₃₋₁₂, C₆₋₉, C₆₋₁₂, C₉₋₁₂, and the like. Similarly, n- to n+m-membered represents that the number of atoms on the ring is n to n+m. For example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring. n- to n+m-membered also represents any range within n to n+m. For example, 3- to 12-membered ring includes 3- to 6-membered ring, 3- to 9-membered ring, 5- to 6-membered ring, 5- to 7-membered ring, 6- to 7-membered ring, 6- to 8-membered ring, 6- to 10-membered ring, and the like.

Unless otherwise specified, "C₂₋₆ alkenyl" is used to denote a linear or branched hydrocarbon group containing 2 to 6 carbon atoms and at least one carbon-carbon double bond, which may be located anywhere in the group. The C₂₋₆ alkenyl may be C₂₋₄ alkenyl or C₂₋₃ alkenyl. The C₂₋₄ alkenyl includes C₂₋₃, C₄, C₃ and C₂ alkenyl, and the like; examples of the C₂₋₄ alkenyl include, but are not limited to, ethenyl, propenyl, butenyl, butadienyl, and the like.

Unless otherwise specified, "C₂₋₆ alkynyl" is used to denote a linear or branched hydrocarbon group containing 2 to 6 carbon atoms and at least one carbon-carbon triple bond, which may be located anywhere in the group. Examples of the C₂₋₆ alkynyl include, but are not limited to, ethynyl, propynyl, and the like.

Unless otherwise specified, the term "C₃₋₈ cycloalkyl" refers to a saturated hydrocarbon cycloalkyl group consisting of 3 to 8 carbon atoms. This includes monocyclic and bicyclic systems, wherein the bicyclic system includes spiro, fused, and bridged rings. The C₃₋₈ cycloalkyl includes C₃₋₆, C₃₋₅, C₄₋₈, C₄₋₆, C₄₋₅, C₅₋₈ or C₅₋₆ cycloalkyl, and may be monovalent, divalent, or polyvalent. Examples of the C₃₋₈ cycloalkyl include, but are not limited to, cyclopropyl, cyclobutyl, cyclopentyl, cyclohexyl, cycloheptyl, norbornyl, [2.2.2]bicyclooctyl, and the like.

Unless otherwise specified, the term "C₃₋₈ partially saturated cycloalkyl" refers to a partially saturated hydrocarbon cycloalkyl group consisting of 3 to 8 carbon atoms. This includes monocyclic and bicyclic systems, wherein the bicyclic system includes spiro, fused, and bridged rings. The C₃₋₈ cycloalkyl includes C₃₋₆, C₃₋₅, C₄₋₈, C₄₋₆, C₄₋₅, C₅₋₈ or C₅₋₆ partially saturated cycloalkyl, and may be monovalent, divalent, or polyvalent.

Unless otherwise specified, the term "C₃₋₆ cycloalkyl" refers to a saturated cyclic hydrocarbon group consisting of 3 to 6 carbon atoms, including monocyclic and bicyclic systems. The C₃₋₆ cycloalkyl includes C₃₋₅, C₄₋₅ and C₅₋₆ cycloalkyl, and the like, and may be monovalent, divalent, or polyvalent. Examples of the C₃₋₆ cycloalkyl include, but are not limited to, cyclopropyl, cyclobutyl, cyclopentyl, cyclohexyl, and the like.

Unless otherwise specified, the term "3- to 8-membered heterocycloalkyl" refers to a saturated cyclic group consisting of 3 to 8 ring atoms, of which 1, 2, 3, or 4 ring atoms are heteroatoms independently selected from the group consisting of O, S, N, P, Si, and Se, with the remaining being carbon atoms, wherein the nitrogen atom is optionally quaternized, and the carbon, silicon, nitrogen, phosphorus, and sulfur heteroatoms may be optionally oxidized (i.e., Si=O, C=O, NO, P=O, and S(O)ₚ, wherein p is 1 or 2). This includes monocyclic and bicyclic systems, wherein the bicyclic system includes spiro, fused, and bridged rings. Furthermore, with respect to the "3-to 8-membered heterocycloalkyl", a heteroatom may occupy the position where the heteroalkyl ring is linked to the rest part of the molecule. The 3- to 8-membered heterocycloalkyl includes 3- to 6-membered heterocycloalkyl and the like. Examples of the 3- to 8-membered heterocycloalkyl include, but are not limited to, oxiranyl, thiiranyl, aziridinyl, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl, dioxepanyl, or the like.

Unless otherwise specified, the term "3- to 8-membered partially saturated heterocycloalkyl" means a partially saturated cyclic group consisting of 3 to 8 ring atoms, of which 1, 2, 3, or 4 ring atoms are heteroatoms independently selected from the group consisting of O, S, N, P, Si, and Se, with the remaining being carbon atoms, wherein the nitrogen atom is optionally quaternized, and the carbon, silicon, nitrogen, phosphorus, and sulfur heteroatoms may be optionally oxidized (i.e., Si=O, C=O, NO, P=O, and S(O)ₚ, wherein p is 1 or 2). This includes monocyclic and bicyclic systems, wherein the bicyclic system includes spiro, fused, and bridged rings. Furthermore, with respect to the "3- to 8-membered partially saturated heterocycloalkyl", a heteroatom may occupy the position where the heteroalkyl ring is linked to the rest part of the molecule. The 3- to 8-membered partially saturated heterocycloalkyl includes 3- to 6-membered partially saturated heterocycloalkyl, 5- to 6-membered partially saturated heterocycloalkyl, and the like.

Unless otherwise specified, the term "3- to 6-membered heterocycloalkyl", by itself or in combination with other terms, refers to a saturated cyclic group consisting of 3 to 6 ring atoms, of which 1, 2, 3, or 4 ring atoms are heteroatoms independently selected from the group consisting of O, S, N, P, Si, and Se, with the remaining being carbon atoms, wherein the nitrogen atom is optionally quaternized, and the carbon, silicon, nitrogen, phosphorus, and sulfur heteroatoms may be optionally oxidized (i.e., C=O, NO, P=O, and S(O)ₚ, wherein p is 1 or 2). It is a monocyclic system. Furthermore, with respect to the "3- to 6-membered heterocycloalkyl", a heteroatom may occupy the position where the heterocycloalkyl is linked to the rest part of the molecule. The 3- to 6-membered heterocycloalkyl includes 3-membered, 4-membered, 5-membered and 6-membered heterocycloalkyl, and the like. Examples of the 3- to 6-membered heterocycloalkyl include, but are not limited to, oxiranyl, thiiranyl, aziridinyl, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl, or the like.

Unless otherwise specified, the term "5- to 9-membered heteroaryl" refers to a cyclic group consisting of 5 to 9 ring atoms and having a conjugated π-electron system, of which 1, 2, 3, or 4 ring atoms are heteroatoms independently selected from the group consisting of O, S, and N, with the remaining being carbon atoms, wherein the nitrogen atom is optionally quaternized, and the carbon, nitrogen, and sulfur heteroatoms may be optionally oxidized (i.e., C=O, NO, and S(O)ₚ, wherein p is 1 or 2). It may be a monocyclic, fused bicyclic, or fused tricyclic system, wherein each ring is aromatic. The 5- to 9-membered heteroaryl may be linked to the rest part of the molecule via a heteroatom or a carbon atom. The 5- to 9-membered heteroaryl includes 5- to 8-membered, 5- to 7-membered, 5- to 6-membered, 5-membered, and 6-membered heteroaryl, and the like. Examples of the 5- to 9-membered heteroaryl include, but are not limited to, pyrrolyl (including N-pyrrolyl, 2-pyrrolyl, 3-pyrrolyl, etc.), pyrazolyl (including 2-pyrazolyl, 3-pyrazolyl, etc.), imidazolyl (including N-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1H-1,2,3-triazolyl, 2H-1,2,3-triazolyl, 1H-1,2,4-triazolyl, 4H-1,2,4-triazolyl, etc.), tetrazolyl, isoxazolyl (3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), thienyl (including 2-thienyl, 3-thienyl, etc.), pyridinyl (including 2-pyridinyl, 3-pyridinyl, 4-pyridinyl, etc.), pyrazinyl, pyrimidinyl (including 2-pyrimidinyl, 4-pyrimidinyl, etc.), benzothiazolyl (including 5-benzothiazolyl, etc.), purinyl, benzimidazolyl (including 2-benzimidazolyl, etc.), benzoxazolyl, indolyl (including 5-indolyl, etc.), isoquinolinyl (including 1-isoquinolinyl, 5-isoquinolinyl, etc.), quinoxalinyl (including 2-quinoxalinyl, 5-quinoxalinyl, etc.), quinolinyl (including 3-quinolinyl, 6-quinolinyl, etc.), and the like.

Unless otherwise specified, the term "C₆₋₁₀ aryl" refers to a cyclic hydrocarbon group consisting of 6 to 10 carbon atoms and having a conjugated π-electron system, which may be a monocyclic, fused bicyclic, or fused tricyclic system, wherein each ring is aromatic. It may be monovalent, divalent, or polyvalent. The C₆₋₁₀ aryl includes C₉, C₁₀ and C₆ aryl. Examples of the C₆₋₁₀ aryl include, but are not limited to, phenyl and naphthyl (including 1-naphthyl, 2-naphthyl, etc.).

The term "treat", "treating", or "treatment" means administering the compound or formulation described in the present disclosure to ameliorate or eliminate a disease or one or more symptoms related to the disease, and includes:
(i) inhibiting the disease or disease state, i.e., arresting its development; and
(ii) alleviating the disease or disease state, i.e., causing its regression.

The term "prevent", "preventing", or "prevention" means administering the compound or formulation described in the present disclosure to prevent a disease or one or more symptoms related to the disease, and includes preventing the occurrence of the disease or disease state in a mammal, particularly when such a mammal is predisposed to the disease state but has not yet been diagnosed with it.

The term "therapeutically or prophylactically effective amount" refers to an amount of the compound of the present disclosure for (i) treating a specific disease, condition, or disorder, or (ii) alleviating, ameliorating, or eliminating one or more symptoms of a specific disease, condition, or disorder, or (iii) preventing or delaying the specific disease, condition, or disorder described herein. The amount of the compound of the present disclosure composing the "therapeutically or prophylactically effective amount" varies depending on the compound, the disease state and its severity, the mode of administration, and the age of the mammal to be treated, but may be determined routinely by those skilled in the art in accordance with their knowledge and the present disclosure.

The therapeutic or prophylactic dose of the compound of the present disclosure may be determined by, for example, the specific use of a treatment or prevention, the mode of administration of the compound, the health and condition of a patient, and the judgment of a prescribing physician. The proportion or concentration of the compound of the present disclosure in a pharmaceutical composition may not be constant and depends on a variety of factors including doses, chemical properties (e.g., hydrophobicity), and routes of administration. For example, the compound of the present disclosure may be provided for parenteral administration by a physiological buffered aqueous solution containing about 0.1%-10% w/v of the compound. In certain embodiments, the dose ranges from about 0.001 mg/kg body weight/day to about 200 mg/kg body weight/day. The dose is likely to depend on variables such as the type and degree of progression of the disease or disorder, the general health condition of a specific patient, the relative biological potency of the compound selected, the excipient formulation, and its route of administration. Effective doses can be extrapolated from dose-response curves derived from *in-vitro* or animal model test systems.

The word "comprise" and variations thereof such as "comprises" or "comprising" will be understood in an open, non-exclusive sense, i.e., "including but not limited to".

"Pharmaceutical composition" refers to a composition comprising one or more of the compound, the isomer thereof, or the pharmaceutically acceptable salt thereof described in the present disclosure, as well as other components such as a physiologically/pharmaceutically acceptable carrier and excipient. The pharmaceutical composition is intended to promote the administration to an organism and facilitate the absorption of the active ingredient so that it can exert its biological activity.

The pharmaceutical composition of the present disclosure may be prepared by combining the compound of the present disclosure with a suitable pharmaceutically acceptable excipient.

The pharmaceutical composition of the present disclosure can be manufactured using methods well known in the art, such as conventional mixing, dissolving, granulating, dragee-making, levigating, emulsifying, and lyophilizing.

The compounds of the present disclosure can be prepared using a variety of synthetic methods well known to those skilled in the art, including the specific embodiments listed below, embodiments formed by combinations thereof with other chemical synthetic methods, and equivalents thereof well known to those skilled in the art. The preferred embodiments include, but are not limited to, the examples of the present disclosure.

In some embodiments, some of the compounds of the present disclosure can be prepared by those skilled in the art of organic synthesis by reference to the following routes:

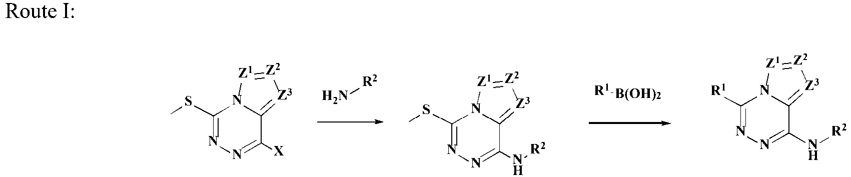

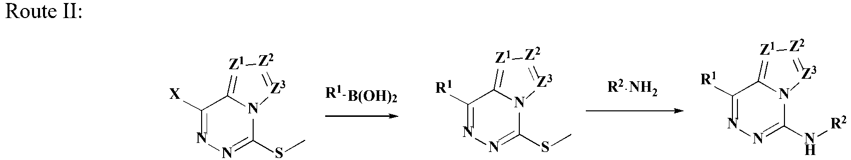

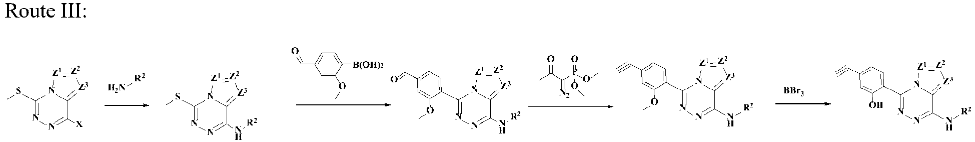

In the routes, Z¹, Z², Z³, R¹, and R² are as defined in the compound of formula (I) above.

The chemical reactions in the specific embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthetic procedure or a reaction process based on the existing embodiments.

The starting materials or intermediates used in the embodiments of the present disclosure may be obtained commercially or prepared using methods in the prior art.

An important consideration in synthetic route planning in the art is the selection of suitable protecting groups for reactive functional groups (e.g., amino in the present disclosure). For example, reference may be made to Greene's Protective Groups in Organic Synthesis (4th Ed.) Hoboken, New Jersey: John Wiley & Sons, Inc.

For clarity, the present disclosure is further described with the following examples, which are, however, not intended to limit the scope of the present disclosure.

All the reagents used in the present disclosure are commercially available and can be used without further purification.

### DETAILED DESCRIPTION

The present disclosure is described in detail below by way of examples. However, this is by no means disadvantageously limiting the scope of the present disclosure. Although the present disclosure has been described in detail herein and specific embodiments thereof have also been disclosed, it will be apparent to those skilled in the art that various changes and modifications can be made to the specific embodiments disclosed herein without departing from the spirit and scope of the present disclosure.

### Example 1

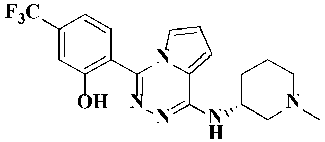

### Reaction process:

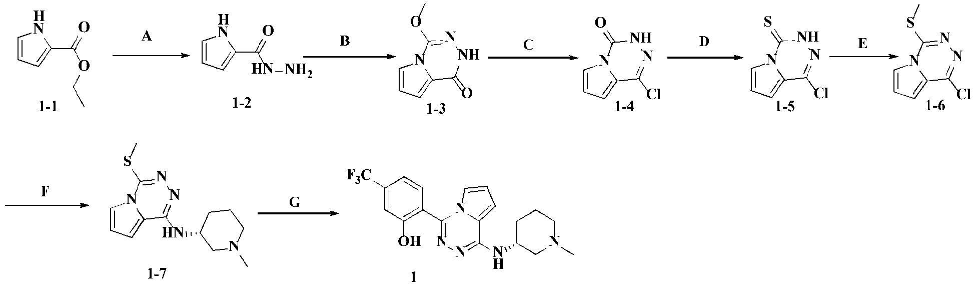

**Step A: 1-1** (8 g, 57.5 mmol) and hydrazine hydrate (71.5 g, 2.23 mol) were added to ethanol (70 mL), and the mixture was heated to 90 °C and allowed to react for 16 h. After the reaction was completed, the reaction solution was cooled to room temperature, filtered, washed with water (10 mL), and washed with ethanol (5 mL). The filter cake was collected and dried under vacuum at 40 °C to give **1-2** (6.6 g).

MS (ESI-, [M-H]⁻) m/z: 124.06.

**Step B:** Tetramethoxymethane (8.16 g, 59.9 mmol) and aluminum isopropoxide (1.632 g, 7.99 mmol) were added to a solution of 1-2 (5 g, 40 mmol) in acetonitrile (10 mL). The mixture was purged with nitrogen, then heated to 120 °C, and allowed to react for 18 h. After the reaction was completed, the reaction solution was concentrated to dryness by rotary evaporation. The resulting crude product was purified by silica gel column chromatography to give **1-3** (3.6 g). MS (ESI+, [M+H]⁺) m/z: 165.92.

¹H NMR (500 MHz, DMSO-*d₆*) δ 11.29 (s, 1H), 7.48 (dd, *J =* 2.8, 1.4 Hz, 1H), 7.03 (dd, *J =* 3.7, 1.4 Hz,1H), 6.73 (dd, *J =* 3.7, 3.0 Hz, 1H), 4.00 (s, 3H).

**Step C: 1-3** (4 g, 24.22 mmol) was dissolved in toluene (100 mL), and then N,N-diisopropylethylamine (DIPEA) (8.46 mL, 48.4 mmol), water (0.57 mL, 31.5 mmol), and phosphorus oxychloride (18.57 g, 121 mmol) were added. The mixture was purged with nitrogen, then heated to 110 °C, and allowed to react for 15 h. After the reaction was completed, the reaction solution was cooled to room temperature, and the solvent was removed by evaporation under reduced pressure. Water (50 mL) and a 1 M sodium hydroxide solution (20 mL) were then added to the residue, and the resulting mixture was extracted with ethyl acetate (3 × 150 mL). The organic phases were combined and concentrated to dryness by rotary evaporation. The resulting crude product was purified by silica gel column chromatography to give **1-4** (1.3 g). MS (ESI-, [M-H]⁻) *m*/*z:* 167.99.

¹H NMR (500 MHz, DMSO-*d6*) δ 12.53 (s, 1H), 7.87 (dd, J = 3.0, 1.5 Hz, 1H), 6.94 - 6.86 (m, 2H).

**Step D:** To a 100-mL single-necked flask were added **1-4** (1.3 g, 7.67 mmol), toluene (20 mL), and Lawesson's reagent (3.1 g, 7.67 mmol) in sequence. The mixture was heated to 110 °C and allowed to react for 18 h. After the reaction was completed, the reaction solution was cooled to room temperature. Water (100 mL) was then added, and the resulting mixture was extracted with ethyl acetate (3 × 100 mL). The organic phases were combined and concentrated to dryness by rotary evaporation. The resulting crude product was purified by silica gel column chromatography to give **1-5** (1.2 g). MS (ESI-, [M-H]⁻) *m*/*z:* 184.00.

¹H NMR (500 MHz, DMSO-*d₆*) δ 14.12 (s, 1H), 8.22 (dd, *J =* 3.0, 1.5 Hz, 1H), 7.14 (dd, *J =* 3.9, 1.5 Hz, 1H), 7.11 (dd, *J =* 3.9, 3.0 Hz, 1H).

**Step E:** To a 100-mL single-necked flask were added **1-5** (1.2 g, 6.46 mmol), tetrahydrofuran (20 mL), cesium carbonate (2.11 g, 7.54 mmol), and iodomethane (1.84 g, 12.9 mmol) in sequence. The mixture was heated to 60 °C and allowed to react for 12 h. After the reaction was completed, the reaction solution was cooled to room temperature. Water (100 mL) was then added, and the resulting mixture was extracted with ethyl acetate (3 × 100 mL). The organic phases were combined and concentrated to dryness by rotary evaporation. The resulting crude product was purified by silica gel column chromatography to give **1-6** (1.2 g). MS (ESI+, [M+H]⁺) *m*/*z:* 200.08.

**Step F:** To a 25-mL single-necked flask were added **1-6** (400 mg, 2.00 mmol) and (R)-1-methylpiperidin-3-amine (4.57 g, 40.1 mmol). The mixture was heated to 110 °C and allowed to react for 4.0 h. After the reaction was completed, the reaction solution was cooled to room temperature and purified by silica gel column chromatography to give **1-7** (380 mg). MS (ESI+, [M+H]⁺) *m*/*z:* 278.01.

**Step G:** To a 35-mL microwave tube were added **1-7** (200 mg, 0.721 mmol), 1,4-dioxane (15 mL), 2-hydroxy-4-trifluoromethylphenylboronic acid (445 mg, 2.16 mmol), copper(I) thiophene-2-carboxylate (302 mg, 1.58 mmol), and tetrakis(triphenylphosphine)palladium (83 mg, 0.072 mmol). The reaction mixture was placed in a microwave reactor and allowed to react under microwave at 110 °C for 8.0 h. After the reaction was completed, the reaction solution was cooled to room temperature and filtered under vacuum, and the filtrate was concentrated to dryness by rotary evaporation. The resulting crude product was purified by silica gel column chromatography to give 1 (55 mg).

¹H NMR (500 MHz, DMSO-*d₆*) δ 11.02(s, 1H), 7.64 (d, *J* = 7.8 Hz, 1H), 7.31 (d, *J* = 8.2 Hz, 2H), 7.06 (dd, *J* = 18.6, 3.2 Hz, 2H), 6.98 (d, *J* = 7.9 Hz, 1H), 6.72 (t, *J* = 3.3 Hz, 1H), 4.26 (d, *J* = 9.6 Hz, 1H), 3.05 - 2.99 (m, 1H), 2.72 - 2.66 (m, 1H), 2.21 (s, 3H), 1.98 - 1.86 (m, 3H), 1.74 (dt, *J =* 12.8, 3.7 Hz, 1H), 1.62 - 1.54 (m, 1H), 1.38 (td, *J* = 11.7, 4.0 Hz, 1H).

HRMS (ESI+, [M+H]⁺) m/z: 392.1695.

### Example 2

### Reaction process:

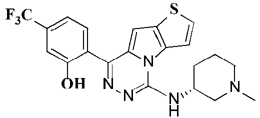

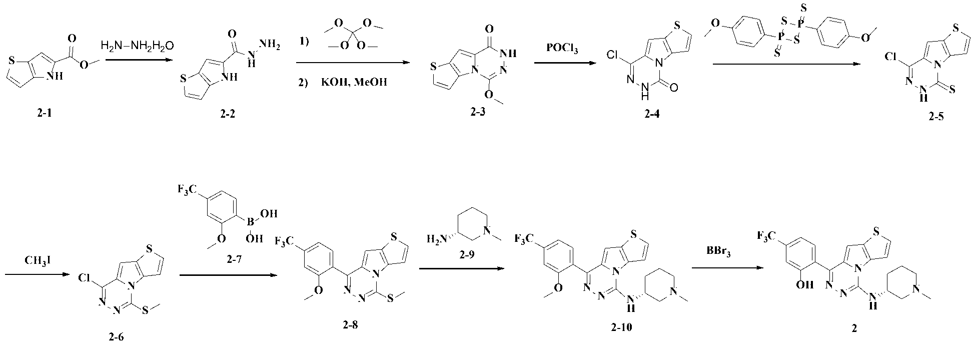

**Step A:** To a 250-mL single-necked flask were added MeOH (80 mL), **2-1** (10 g, 55.2 mmol), and hydrazine hydrate (32.5 g, 552 mmol) in sequence. Under a nitrogen atmosphere, the mixture was heated to 90 °C and allowed to react for 16 h. After the reaction was completed, the mixture was filtered under vacuum, and the filter cake was dried under vacuum to give **2-2** (9.55 g).

MS (ESI+, [M+H]⁺) *m*/*z:* 182.05.

**Step B:** To a 100-mL single-necked flask were added **2-2** (1.0 g, 5.52 mmol), acetonitrile (30 mL), acetic acid (1.0 g, 27.6 mmol), and tetramethoxymethane (1.50 g, 11.04 mmol) in sequence. Under a nitrogen atmosphere, the mixture was heated to 70 °C and allowed to react for 4 h. After the reaction was completed, the solvent was removed by evaporation under reduced pressure using a rotary evaporator to give a residue. MeOH (50 mL) and potassium hydroxide (0.62 g, 11.04 mmol) were added to the above residue, and the mixture was heated to 70 °C and allowed to react for 3 h. After the reaction was completed, the methanol was removed by evaporation under reduced pressure using a rotary evaporator. Water (40 mL) was added to the residue, and the pH was adjusted to 1 with a 6 N HCl solution (10 mL). The mixture was stirred for 1 h and filtered under vacuum, and the filter cake was dried under vacuum to give **2-3** (1.12 g).

¹H NMR (500 MHz, DMSO-*d₆*) δ 11.34 (s, 1H), 7.75 (d, *J* = 5.4 Hz, 1H), 7.45 (d, *J* = 5.3 Hz, 1H), 7.42 (s, 1H), 4.07 (s, 3H).

MS (ESI+, [M+H]⁺) *m*/*z*: 221.91.

**Step C:** To a 100-mL single-necked flask were added toluene (50 mL), **2-3** (1.12 g, 5.06 mmol), DIPEA (1.31 g, 10.13 mmol), water (0.119 g, 6.58 mmol), and phosphorus oxychloride (3.88 g, 25.3 mmol) in sequence. Under a nitrogen atmosphere, the mixture was heated to 110 °C and allowed to react for 16 h. After the reaction was completed, the solvent was removed by evaporation under reduced pressure using a rotary evaporator. Water (30 mL) was added to the residue, resulting in the precipitation of a solid. The mixture was filtered under vacuum, and the filter cake was dried under vacuum to give **2-4** (0.82 g).

MS (ESI-, [M-H]⁻) m/z: 223.96.

**Step D:** To a 50-mL single-necked flask were added **2-4** (400 mg, 1.77 mmol), xylene (12 mL), and Lawesson's reagent (574 mg, 1.41 mmol) in sequence. Under a nitrogen atmosphere, the mixture was heated to 140 °C and allowed to react for 5 h. After the reaction was completed, water (80 mL) was added, and the mixture was extracted with ethyl acetate (50 mL × 2). The organic layers were combined, washed with saturated brine (80 mL), dried over anhydrous sodium sulfate, filtered, and concentrated. The resulting crude product was purified by silica gel column chromatography (DMC:MeOH = 50:1) to give **2-5** (85 mg).

MS (ESI-, [M-H]⁻) *m*/*z:* 240.04.

**Step E:** To a 50-mL single-necked flask were added **2-5** (170 mg, 0.703 mmol), THF (8 mL), iodomethane (200 mg, 1.407 mmol), and cesium carbonate (229 g, 0.703 mmol) in sequence. Under a nitrogen atmosphere, the mixture was heated to 60 °C and allowed to react for 2 h. After the reaction was completed, ethyl acetate (50 mL) and water (50 mL) were added to the reaction solution. The phases were separated, and the organic phase was washed with saturated brine (30 mL), dried over anhydrous sodium sulfate, filtered, and concentrated under reduced pressure. The resulting crude product was purified by silica gel column chromatography (PE:EA = 20:1) to give **2-6** (72 mg).

¹H NMR (500 MHz, DMSO-*d₆*) δ 7.91 (d, *J* = 5.5 Hz, 1H), 7.77 (d, *J* = 5.5 Hz, 1H), 7.53 (s, 1H), 2.85 (s, 3H). MS (ESI+, [M+H]⁺) m/z: 255.89.

**Step F:** To a 50-mL single-necked flask were added **2-6** (50 mg, 0.196 mmol), 1,4-dioxane (10 mL), **2-7** (86 mg, 0.391 mmol), XphosPdG3 (33 mg, 0.039 mmol), potassium carbonate (81 mg, 0.587 mmol), and water (3 mL) in sequence. Under a nitrogen atmosphere, the mixture was heated to 90 °C and allowed to react for 5 h. After the reaction was completed, the solvent was removed by evaporation under reduced pressure using a rotary evaporator. The resulting crude product was purified by silica gel column chromatography (PE:EA = 5:1) to give **2-8** (52 mg).

¹H NMR (500 MHz, DMSO-*d₆*) δ 7.81 (dd, *J* = 14.8, 5.5 Hz, 2H), 7.70 (d, *J* = 7.7 Hz, 1H), 7.54 (s, 1H), 7.50 (d, *J* = 7.8 Hz, 1H), 7.02 (s, 1H), 3.85 (s, 3H), 2.90 (s, 3H).

MS (ESI+, [M+H]⁺) *m*/*z:* 396.05.

**Step G:** To a 10-mL reaction flask were added **2-8** (240 mg, 0.607 mmol) and **2-9** (1.73 g, 15.17 mmol) in sequence. Under a nitrogen atmosphere, the mixture was heated to 120 °C and allowed to react for 16 h. After the reaction was completed, the solvent was removed by evaporation under reduced pressure using a rotary evaporator. The resulting crude product was purified by silica gel column chromatography (DCM:MeOH = 25:1) to give **1-10** (130 mg).

MS (ESI+, [M+H]+) m/z: 462.12.

**Step H:** To a 25-mL single-necked flask were added **2-10** (130 mg, 0.282 mmol), dichloromethane (10 mL), and a solution of boron tribromide in dichloromethane (2 N, 1.408 mL, 2.82 mmol) in sequence. Under a nitrogen atmosphere, the mixture was allowed to react at room temperature for 1 h. After the reaction was completed, the reaction solution was poured into a saturated aqueous sodium bicarbonate solution (50 mL) and extracted with DCM (50 mL × 2). The organic phase was collected, dried over anhydrous sodium sulfate, and filtered, and the solvent was removed by evaporation under reduced pressure using a rotary evaporator. The resulting crude product was subjected to preparative liquid chromatography (CHIRAL ART Cellulose-SB chromatographic column; 0.05% aqueous phosphoric acid solution-ethanol (60:40)) to give compound 2 (20 mg).

¹H NMR (500 MHz, DMSO-*d₆*) δ 12.37 (s, 1H), 8.24 - 7.51 (m, 3H), 7.46 - 7.15 (m, 3H), 6.91 - 6.47 (m, 1H), 4.59 - 4.07 (m, 1H), 2.98 - 2.84 (m, 1H), 2.65 - 2.55 (m, 1H), 2.41 - 2.28 (m, 1H), 2.25 (s, 3H), 2.20 - 2.04 (m, 1H), 1.97 - 1.87 (m, 1H), 1.80 - 1.57 (m, 3H).

HRMS (ESI+, [M+H]+) m/z: 448.1447.

### Example 3

### Reaction process:

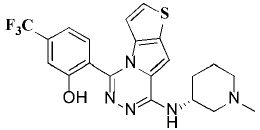

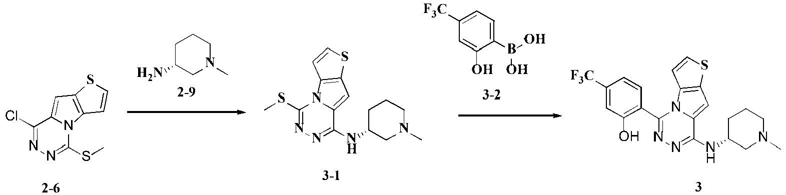

**Step A:** To a 10-mL reaction flask were added 2-6 (200 mg, 0.782 mmol) and 2-9 (1.73 g, 15.17 mmol) in sequence. Under a nitrogen atmosphere, the mixture was heated to 100 °C and allowed to react for 16 h. After the reaction was completed, the solvent was removed by evaporation under reduced pressure using a rotary evaporator. The resulting crude product was purified by silica gel column chromatography (DCM:MeOH = 25:1) to give **3-1** (76 mg).

MS (ESI+, [M+H]+) m/z: 334.05.

**Step B:** To a 35-mL microwave tube were added **3-1** (76 mg, 0.228 mmol), copper(I) thiophene-2-carboxylate (192 mg, 1.002 mmol), Pd(Ph₃P)₄ (52 mg, 0.046 mmol), **3-2** (282 mg, 1.368 mmol), and 1,4-dioxane (7 mL) in sequence. Under a nitrogen atmosphere, the mixture was heated to 120 °C under microwave and allowed to react for 4 h. After the reaction was completed, the mixture was filtered, and the solvent was removed by evaporation under reduced pressure using a rotary evaporator. The resulting crude product was subjected to preparative liquid chromatography (XB-C18 chromatographic column; water (10 mM ammonium acetate + 0.1% glacial acetic acid)-acetonitrile (62:38)) to give compound **3** (11 mg).

¹H NMR (500 MHz, DMSO-*d6*) δ 10.84 (s, 1H), 7.67 (s, 1H), 7.56 - 7.17 (m, 4H), 7.07 (s, 1H), 6.03 (s, 1H), 4.30 (s, 1H), 3.06 (s, 1H), 2.71 (s, 1H), 2.22 (s, 3H), 2.01 - 1.88 (m, 3H), 1.75 (s, 1H), 1.60 (s, 1H), 1.42 (s, 1H). HRMS (ESI+, [M+H]⁺) m/z: 448.1408.

### Example 4

### Reaction process:

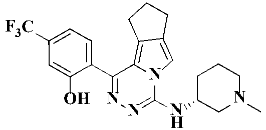

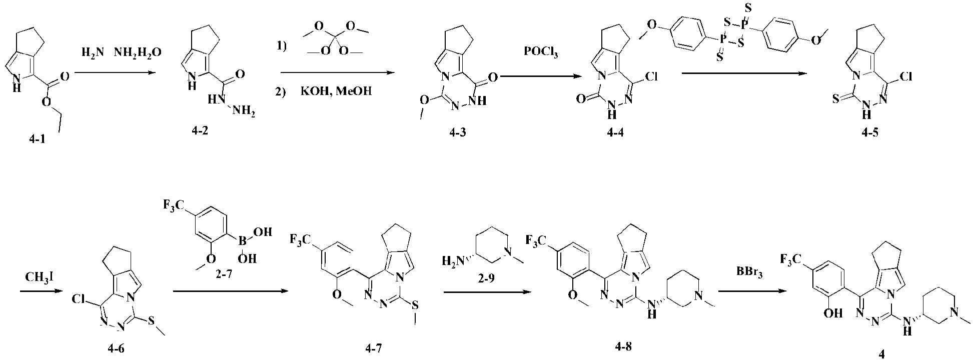

**Step A:** Referring to Example 2, **4-2** was obtained by replacing **2-1** with **4-1** in step A.

¹H NMR (500 MHz, DMSO-*d₆*) δ 10.77 (s, 1H), 8.32 (s, 1H), 6.50 (d, *J =* 2.5 Hz, 1H), 4.27 (s, 2H), 2.73 (t, *J* = 7.2 Hz, 2H), 2.52 (d, *J =* 8.4 Hz, 2H), 2.25 (dd, *J =* 14.3, 7.2 Hz, 2H).

MS (ESI+, [M+H]⁺) *m*/*z:* 165.95.

**Step B:** Referring to Example 2, **4-3** was obtained by replacing 2-2 with 4-2 in step B.

¹H NMR (500 MHz, DMSO-*d₆*) δ 11.06 (s, 1H), 7.08 (s, 1H), 3.95 (s, 3H), 2.84 (t, *J =* 7.2 Hz, 2H), 2.69 (t, *J =* 7.2 Hz, 2H), 2.35 (p, *J* = 7.3 Hz, 2H).

MS (ESI+, [M-H]⁺) *m*/*z:* 205.90.

**Step C:** Referring to Example 2, **4-4** was obtained by replacing 2-3 with 4-3 in step C.

¹H NMR (500 MHz, DMSO-*d₆*) *δ* 12.25 (s, 1H), 7.51 (s, 1H), 2.91 (t, *J =* 7.3 Hz, 2H), 2.74 (t, *J =* 7.2 Hz, 2H), 2.39 (p, *J* = 7.3 Hz, 2H).

MS (ESI-, [M-H]⁻) *m*/*z:* 207.98.

**Step D:** Referring to Example 2, **4-5** was obtained by replacing **2-4** with **4-4** in step D.

¹H NMR (500 MHz, DMSO-*d₆*) *δ* 13.80 (s, 1H), 7.92 (s, 1H), 2.99 (t, J = 7.3 Hz, 2H), 2.81 (t, *J* = 7.2 Hz, 2H), 2.41 (p, *J =* 7.3 Hz, 2H).

MS (ESI-, [M-H]⁻) m/z: 223.98.

**Step E:** Referring to Example 2, **4-6** was obtained by replacing **2-5** with **4-5** in step E.

¹H NMR (500 MHz, DMSO-*d₆*) δ 7.49 (s, 1H), 3.01 (t, *J* = 7.3 Hz, 2H), 2.84 (t, *J* = 7.3 Hz, 2H), 2.76 (s, 3H), 2.47 - 2.40 (m, 2H).

MS (ESI+, [M+H]⁺) *m*/*z:* 239.93.

**Step F:** Referring to Example 2, **4-7** was obtained by replacing **2-6** with **4-6** in step F.

MS (ESI+, [M+H]⁺) *m*/*z:* 380.08.

**Step G:** Referring to Example 2, **4-8** was obtained by replacing **2-7** with **4-7** in step G.

¹H NMR (500 MHz, DMSO-*d₆*) δ 7.60 (s, 1H), 7.50 (d, *J* = 8.0 Hz, 1H), 7.41 (s, 2H), 6.93 (d, *J* = 6.7 Hz, 1H), 4.28 (s, 1H), 3.79 (s, 3H), 3.10 (s, 1H), 2.75 (s, 3H), 2.24 (d, *J =* 9.5 Hz, 7H), 1.98 (s, 3H), 1.77 (d, *J =* 11.4 Hz, 1H), 1.60 (d, *J* = 11.9 Hz, 1H), 1.42 (d, *J =* 10.7 Hz, 1H).

MS (ESI+, [M+H]⁺) m/z: 446.19.

**Step H:** Referring to Example 2, **4** was obtained by replacing **2-8** with **4-8** in step H.

¹H NMR (500 MHz, DMSO-*d₆*) δ 11.78 (s, 1H), 7.72 (s, 1H), 7.68 (d, *J* = 7.9 Hz, 1H), 7.27 (d, *J* = 8.2 Hz, 1H), 7.25 (s, 1H), 7.14 (s, 1H), 4.31 (s, 1H), 3.12 (s, 1H), 2.80 (t, *J* = 7.1 Hz, 3H), 2.64 (t, *J* = 7.1 Hz, 2H), 2.35 - 2.21 (m, 5H), 2.00 (d, *J* = 9.1 Hz, 3H), 1.77 (s, 1H), 1.62 (d, J = 11.2 Hz, 1H), 1.46 (s, 1H).

HRMS (ESI+, [M+H]⁺) m/z: 432.2007.

### Example 5

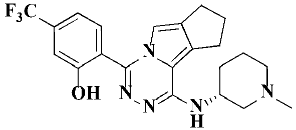

### Reaction process:

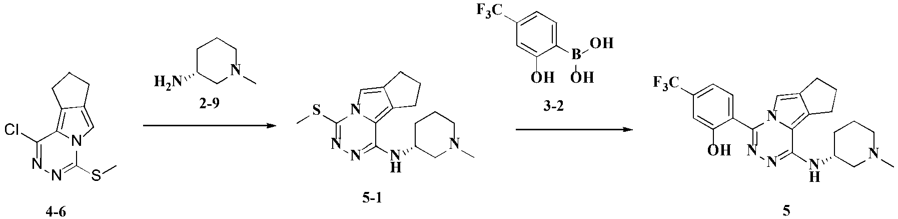

**Step** A: Referring to Example 3, **5-1** was obtained by replacing **2-6** with **4-6** in step A.

MS (ESI+, [M+H]⁺) *m*/*z:* 318.05.

**Step B:** Referring to Example 3, **5** was obtained by replacing **3-1** with **5-1** in step B.

¹H NMR (500 MHz, DMSO-*d₆*) δ 10.82 (s, 1H), 7.60 (d, *J* = 8.1 Hz, 1H), 7.30 (d, *J* = 6.7 Hz, 2H), 6.71 (s, 1H), 5.69 (d, *J* = 6.9 Hz, 1H), 4.26 (s, 1H), 3.00 (dd, *J* = 8.5, 5.4 Hz, 2H), 2.85 (s, 1H), 2.68 (t, *J* = 7.0 Hz, 2H), 2.39 (dd, *J =* 14.1, 7.0 Hz, 2H), 2.32-2.08 (m, 5H), 1.91 (s, 1H), 1.79 (s, 1H), 1.72 (s, 1H), 1.57 (s, 2H).

HRMS (ESI+, [M+H]⁺) m/z: 432.2012.

### Example 6

### Reaction process:

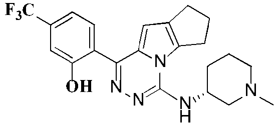

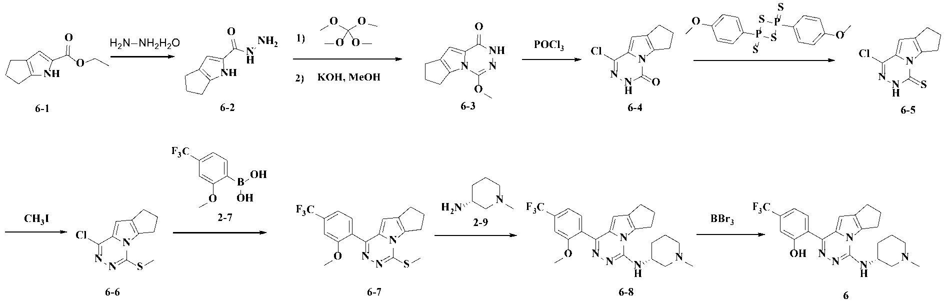

**Step A:** Referring to Example 2, **6-2** was obtained by replacing **2-1** with **6-1** in step A.

MS (ESI+, [M+H]⁺) m/z: 165.96.

**Step B:** Referring to Example 2, **6-3** was obtained by replacing **2-2** with **6-2** in step B.

¹H NMR (500 MHz, DMSO-*d₆*) δ 11.06 (s, 1H), 6.78 (s, 1H), 3.93 (s, 3H), 2.95 (t, *J =* 7.2 Hz, 2H), 2.67 - 2.63 (m, 2H), 2.45 - 2.40 (m, 2H).

MS (ESI+, [M+H]⁺) *m*/*z:* 206.00.

**Step C:** Referring to Example 2, **6-4** was obtained by replacing 2-3 with 6-3 in step C.

¹H NMR (500 MHz, DMSO-*d₆*) δ 12.25 (s, 1H), 6.63 (s, 1H), 3.10 (t, *J =* 7.2 Hz, 2H), 2.67 (t, *J =* 7.1 Hz, 2H), 2.45 (dt, *J =* 14.5, 7.3 Hz, 2H).

MS (ESI-, [M-H]⁻) *m*/*z:* 208.09.

**Step D:** Referring to Example 2, **6-5** was obtained by replacing **2-4** with **6-4** in step D.

¹H NMR (500 MHz, DMSO-*d₆*) δ 13.68 (s, 1H), 6.85 (s, 1H), 3.47 - 3.43 (m, 2H), 2.70 (t, *J =* 7.3 Hz, 2H), 2.42 (dd, *J* = 14.6, 7.3 Hz, 2H).

MS (ESI-, [M-H]⁻) m/z: 224.08.

**Step E:** Referring to Example 2, **6-6** was obtained by replacing **2-5** with **6-5** in step E.

¹H NMR (500 MHz, DMSO-*d₆*) δ 6.82 (s, 1H), 3.28 (t, *J =* 7.3 Hz, 2H), 2.77 (t, *J =* 7.2 Hz, 2H), 2.71 (s, 3H), 2.49 (s, 2H).

MS (ESI+, [M+H]⁺) m/z: 239.96.

**Step F:** Referring to Example 2, **6-7** was obtained by replacing **2-6** with **6-6** in step F.

¹H NMR (500 MHz, DMSO-*d₆*) δ 7.60 (d, *J* = 7.8 Hz, 1H), 7.48 (s, 1H), 7.45 (d, *J* = 7.8 Hz, 1H), 6.31 (s, 1H), 3.82 (s, 3H), 3.34 (d, *J* = 7.3 Hz, 2H), 2.75 (s, 3H), 2.72 (t, *J* = 7.1 Hz, 2H), 2.47 (d, *J* = 7.3 Hz, 2H).

MS (ESI+, [M+H]⁺) *m*/*z*: 380.09.

**Step G:** Referring to Example 2, **6-8** was obtained by replacing **2-8** with **6-7** in step F.

¹H NMR (500 MHz, DMSO-*d₆*) δ 7.54 (d, *J* = 7.7 Hz, 1H), 7.48 - 7.38 (m, 2H), 6.07 (s, 1H), 5.90 (d, *J* = 7.6 Hz, 1H), 4.20 (s, 1H), 3.80 (s, 3H), 3.29 (s, 2H), 2.68 (t, *J =* 7.0 Hz, 2H), 2.53 (s, 2H), 2.35 (s, 3H), 2.23 (s, 3H), 1.75 (s, 3H), 1.65 (d, *J* = 8.6 Hz, 1H), 1.56 (s, 1H).

MS (ESI+, [M+H]+) m/z: 446.41.

**Step H:** Referring to Example 2, **6** was obtained by replacing **2-10** with **6-8** in step F.

¹H NMR (500 MHz, DMSO-*d₆*) 13.58 (s, 1H), 8.08 (d, *J* = 8.3 Hz, 1H), 7.25 (d, *J* = 6.6 Hz, 2H), 6.95 (s, 1H), 6.17 (d, *J* = 7.1 Hz, 1H), 4.21 (s, 1H), 3.35 (s, 2H), 2.78 (t, *J* = 7.0 Hz, 2H), 2.67 (s, 1H), 2.60 - 2.53 (m, 2H), 2.33 (s, 3H), 2.23 (s, 3H), 1.73 (d, *J* = 13.0 Hz, 3H), 1.56 (d, *J* = 5.5 Hz, 1H).

HRMS (ESI+, [M+H]+) m/z: 432.2002.

### Example 7

### Reaction process:

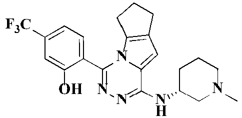

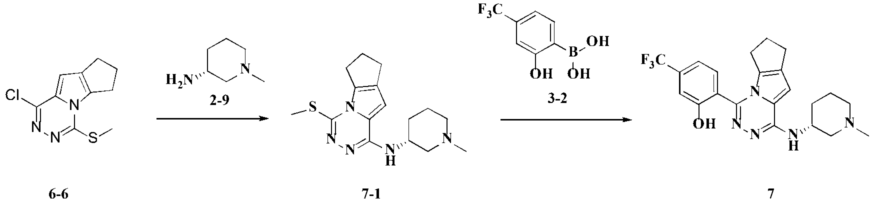

**Step A:** Referring to Example 3, 7-1 was obtained by replacing 2-6 with 6-6 in step A.

MS (ESI+, [M+H]⁺) *m*/*z:* 318.05.

**Step B:** Referring to Example 3, 7 was obtained by replacing 3-1 with 7-1 in step B.

¹H NMR (500 MHz, DMSO-*d₆*) 13.56 (s, 1H), 8.09 (d, *J* = 7.1 Hz, 1H), 7.25 (s, 2H), 6.95 (s, 1H), 6.18 (s, 1H), 4.21 (s, 1H), 3.36 (s, 2H), 2.78 (s, 2H), 2.68 (s, 1H), 2.57 (d, *J* = 23.9 Hz, 2H), 2.34 (s, 3H), 2.24 (s, 3H), 1.74 (s, 3H), 1.56 (s, 1H).

HRMS (ESI+, [M+H]⁺) m/z: 432.2012.

### Example 8

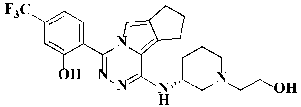

### Reaction process:

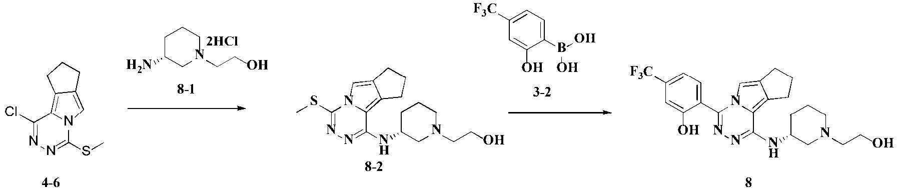

**Step A:** Referring to Example 5, **8-2** was obtained by replacing **2-9** with **8-1** in step A.

MS (ESI+, [M+H]⁺) *m*/*z:* 380.05.

**Step B:** Referring to Example 5, **8** was obtained by replacing **5-1** with **8-2** in step B.

¹H NMR (500 MHz, DMSO-*d₆*) 10.87 (s, 1H), 7.82 - 7.54 (m, 1H), 7.49 - 7.19 (m, 2H), 6.90 - 6.65 (m, 1H), 5.98 - 5.58 (m, 1H), 4.68 - 4.13 (m, 2H), 3.60 (s, 2H), 3.17 - 2.99 (m, 2H), 2.90 (dd, *J* = 29.1, 8.6 Hz, 1H), 2.74 (t, *J* = 14.5 Hz, 2H), 2.52 - 2.32 (m, 5H), 1.83 - 1.49 (m, 4H), 1.34 (d, *J* = 32.1 Hz, 2H).

HRMS (ESI+, [M+H]⁺) m/z: 462.2123.

### Example 9

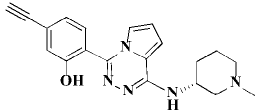

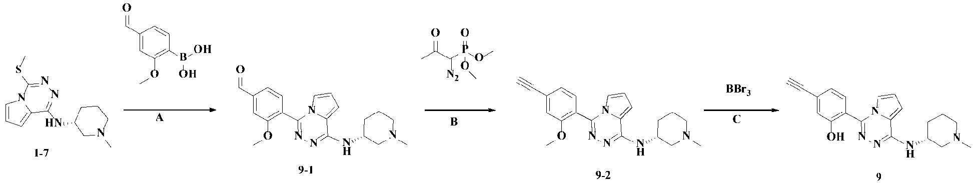

**Step A:** To a 250-mL single-necked flask were added 1,4-dioxane (100 mL), **1-7** (800 mg, 2.88 mmol), 4-formyl-2-methoxyphenylboronic acid (2.06 g, 11.54 mmol), copper(I) 3-methylsalicylate (2.46 g, 11.54 mmol), and Pd(Ph₃P)₄ (1.66 g, 1.44 mmol) in sequence. Under a nitrogen atmosphere, the mixture was heated to 100 °C and allowed to react for 7.0 h. After the reaction was completed, the solvent was removed by evaporation under reduced pressure using a rotary evaporator. The resulting crude product was purified by silica gel column chromatography (dichloromethane:methanol = 20:1) to give **9-1** (364 mg).

¹H NMR (500 MHz, DMSO-*d₆*) δ 10.12 (s, 1H), 7.71 (s, 3H), 7.14 (s, 1H), 7.10 (d, *J* = 4.0 Hz, 1H), 6.98 (d, *J* = 4.1 Hz, 1H), 6.77 - 6.68 (m, 1H), 4.35 (s, 1H), 3.83 (s, 3H), 3.17 (s, 1H), 2.89 (s, 1H), 2.41 (s, 3H), 1.98 (d, *J* = 9.8 Hz, 2H), 1.82 (s, 1H), 1.66 (d, *J* = 11.7 Hz, 2H), 1.49 (s, 1H).

MS (ESI+, [M+H]⁺) *m*/*z*: 366.28.

**Step B:** To a 50-mL single-necked flask were added methanol (15 mL), **9-1** (190 mg, 0.52 mmol), potassium carbonate (144 mg, 1.04 mmol), dimethyl (1-diazo-2-oxopropyl)phosphonate (120 mg, 0.624 mmol) in sequence. Under a nitrogen atmosphere, the mixture was allowed to react at room temperature for 16 h. After the reaction was completed, water (80 mL) was added, and the mixture was extracted with dichloromethane (50 mL × 2). The organic layers were combined, washed with saturated brine (80 mL), dried over anhydrous sodium sulfate, filtered, and concentrated. The resulting crude product was purified by silica gel column chromatography (dichloromethane:methanol = 30:1) to give **9-2** (66 mg).

MS (ESI+, [M+H]⁺) *m*/*z:* 362.30.

**Step I:** To a 50-mL single-necked flask were added dichloromethane (15 mL), **9-2** (47 mg, 0.13 mmol), and a solution of boron tribromide in dichloromethane (2 N, 0.65 mL, 1.30 mmol) in sequence. Under a nitrogen atmosphere, the mixture was allowed to react in an ice bath for 0.5 h. After the reaction was completed, the reaction solution was poured into a saturated aqueous sodium bicarbonate solution (30 mL) and extracted with dichloromethane (20 mL × 2). The organic phase was collected, dried over anhydrous sodium sulfate, and filtered, and the solvent was removed by evaporation under reduced pressure using a rotary evaporator. The resulting crude product was subjected to preparative liquid chromatography (CHIRAL ART Cellulose-SB chromatographic column; 0.05% aqueous phosphoric acid solution-ethanol (60:40)) to give compound 9 (23 mg). ¹H NMR (500 MHz, DMSO-*d₆*) δ 10.40 (s, 1H), 7.41 (d, *J* = 7.7 Hz, 1H), 7.07 (ddd, *J* = 11.2, 3.4, 1.4 Hz, 3H), 7.02 (dd, *J* = 2.8, 1.3 Hz, 1H), 6.93 (d, *J* = 8.0 Hz, 1H), 6.71 (dd, *J* = 3.8, 2.7 Hz, 1H), 4.29 (s, 1H), 4.27 - 4.19 (m, 1H), 3.12 - 2.91 (m, 1H), 2.69 (d, *J* = 11.1 Hz, 1H), 2.21 (s, 3H), 1.97 - 1.82 (m, 3H), 1.74 (dt, *J* = 11.8, 3.7 Hz, 1H), 1.58 (tdd, *J* = 15.6, 9.7, 3.9 Hz, 1H), 1.44 - 1.29 (m, 1H).

HRMS (ESI+, [M+H]⁺) m/z: 348.1818.

### Example 10

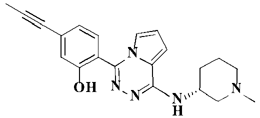

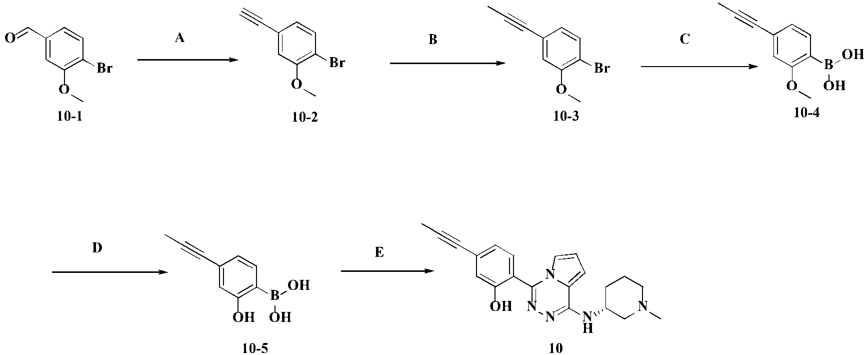

**Step A:** To a 250-mL two-necked reaction flask were added **10-1** (5 g, 23.25 mmol), methanol (100 mL), dimethyl (1-diazo-2-oxopropyl)phosphonate (6.70 g, 34.90 mmol), and K₂CO₃ (6.43 g, 46.5 mmol) in sequence. Under a nitrogen atmosphere, the mixture was allowed to react at room temperature for 5 h. After the reaction was completed, purified water (100 mL) was added to quench the reaction, and ethyl acetate (125 mL × 2) was added for extraction. The organic phases were combined, washed with saturated brine (50 mL), dried over anhydrous sodium sulfate, and filtered, and the solvent was removed by evaporation under reduced pressure using a rotary evaporator. The resulting crude product was purified by silica gel column chromatography (petroleum ether) to give **10-2** (4.39 g).

¹H NMR (500 MHz, Chloroform-*d*) δ 7.48 (d, *J* = 8.0 Hz, 1H), 6.99 (d, *J =* 1.8 Hz, 1H), 6.97 (dd, *J =* 8.1, 1.8 Hz, 1H), 3.89 (s, 3H), 3.12 (s, 1H).

**Step B:** To a 250-mL two-necked reaction flask were added **10-2** (3.6 g, 17.06 mmol) and tetrahydrofuran (60 mL) in sequence. Under a nitrogen atmosphere, the mixture was cooled to -30 °C, and then sodium hexamethyldisilazide (7.88 g, 21.49 mL, 43.0 mmol) was slowly added dropwise via a syringe. The mixture was then stirred at this temperature for 15 min, and then iodomethane (7.26 g, 3.20 mL, 51.2 mmol) was slowly added dropwise via a syringe. After the addition was completed, the mixture was slowly warmed to room temperature and allowed to react for another 3 h. After the reaction was completed, purified water (50 mL) was added to quench the reaction, and ethyl acetate (100 mL × 3) was added for extraction. The organic phases were combined, washed with saturated brine (50 mL), dried over anhydrous sodium sulfate, and filtered, and the solvent was removed by evaporation under reduced pressure using a rotary evaporator. The resulting crude product was purified by silica gel column chromatography (petroleum ether) to give **10-3** (4.14 g).

¹H NMR (500 MHz, Chloroform-*d*) δ 7.43 (d, *J* = 8.1 Hz, 1H), 6.91 (d, *J =* 1.8 Hz, 1H), 6.86 (dd, *J =* 8.1, 1.8 Hz, 1H), 3.87 (s, 3H), 2.04 (s, 3H).

**Step C:** To a 250-mL two-necked reaction flask were added **10-3** (4 g, 17.77 mmol) and tetrahydrofuran (100 mL) in sequence. Under a nitrogen atmosphere, the mixture was cooled to -78 °C and incubated for 10 min, and then *n*-butyllithium (1.8 g, 17.56 mL, 28.1 mmol) was slowly added dropwise via a syringe. After the addition was completed, the mixture was stirred at this temperature for 20 min, and then triethyl borate (7.78 g, 53.3 mmol) was slowly added dropwise via a syringe. After the addition was completed, the mixture was further stirred at this temperature for 40 min, then slowly warmed to room temperature, and stirred for another 1.5 h. After the reaction was completed, 20 mL of 4 N HCl was added to quench the reaction. The mixture was then stirred for 1 h, and ethyl acetate (150 mL) and water (50 mL) were added. The organic phase was separated, and the aqueous phase was extracted with ethyl acetate (50 mL × 2). The organic phases were combined, washed with saturated brine (50 mL), dried over anhydrous sodium sulfate, and filtered, and the solvent was removed by evaporation under reduced pressure using a rotary evaporator. The resulting crude product was purified by silica gel column chromatography (petroleum ether:ethyl acetate = 1:1) to give **10-4** (2.9 g).

**Step D:** To a 100-mL reaction flask were added **10-4** (2.9 g, 15.26 mmol), dichloromethane (50 mL), and boron tribromide (12 g, 23.95 mL, 47.9 mmol) in sequence. Under a nitrogen atmosphere, the mixture was allowed to react in an ice-water bath for 1 h. After the reaction was completed, the reaction solution was slowly poured into water (50 mL) and extracted with ethyl acetate (125 mL × 2). The organic layers were combined, washed with saturated brine (50 mL), dried over anhydrous sodium sulfate, and filtered, and the solvent was removed by evaporation under reduced pressure using a rotary evaporator. The resulting crude product was purified by silica gel column chromatography (dichloromethane:methanol = 100:1) to give **10-5** (2.96 g).

MS (ESI-, [M-H]⁻) *m*/*z:* 175.04.

**Step E:** Referring to Example **9**, **10** was obtained by replacing 4-formyl-2-methoxyphenylboronic acid with **10-5** in step G. ¹H NMR (500 MHz, DMSO-*d₆*) δ 10.12 (s, 1H), 7.26 (d, *J =* 7.9 Hz, 1H), 7.05 (d, *J* = 3.8 Hz, 1H), 7.00 (d, *J =* 2.7 Hz, 1H), 6.95 (s, 1H), 6.88 (d, *J =* 8.0 Hz, 1H), 6.83 - 6.75 (m, 1H), 6.67 (t, *J =* 3.3 Hz, 1H), 4.23 (td, *J* = 14.3, 6.1 Hz, 1H), 3.01 (d, *J =* 10.6 Hz, 1H), 2.67 (t, *J =* 6.0 Hz, 1H), 2.19 (s, 3H), 2.05 (s, 3H), 1.90-1.86 (m, 3H), 1.73-1.69 (m, 1H), 1.57 (d, *J* = 12.7 Hz, 1H), 1.39 - 1.33 (m, 1H).

HRMS (ESI+, [M+H]⁺) m/z: 362.1979.

### Example 11

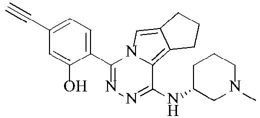

### Reaction process:

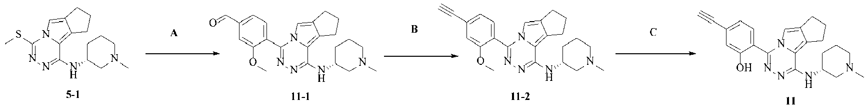

**Step A:** Referring to Example 9, **11-1** was obtained by replacing **1-7** with **5-1** in step A.

MS (ESI+, [M+H]⁺) m/z: 406.29.

**Step A:** Referring to Example 9, **11-2** was obtained by replacing **9-1** with **11-1** in step B.

MS (ESI+, [M+H]⁺) *m*/*z:* 402.14.

**Step B:** Referring to Example 9, **11** was obtained by replacing **9-2** with **11-2** in step C.

¹H NMR (500 MHz, DMSO-*d₆*) δ 10.42 (s, 1H), 7.38 (d, 1H), 7.16 - 6.97 (m, 2H), 6.70 (s, 1H), 5.62 (d, 1H), 4.28 (s, 1H), 4.26 - 4.16 (m, 1H), 3.07 - 2.92 (m, 2H), 2.78 (s, 1H), 2.68 (t, 2H), 2.46 (s, 1H), 2.38 (p, 2H), 2.21 (s, 3H), 2.15 (s, 2H), 1.77 (s, 1H), 1.69 (s, 1H), 1.61 - 1.45 (m, 2H).

HRMS (ESI+, [M+H]⁺) m/z: 388.2139.

### Example 12

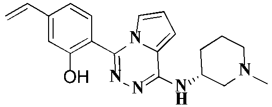

### Reaction process:

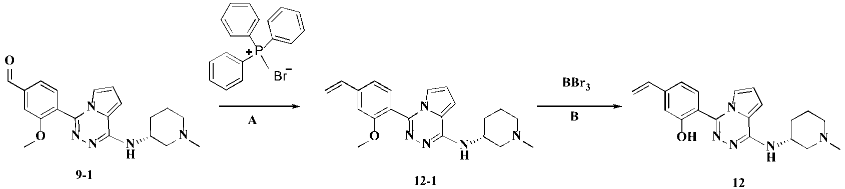

**Step** A: To a 100-mL single-necked flask were added **9-1** (500 mg, 1.36 mmol), 1,4-dioxane (50 mL), methyltriphenylphosphonium bromide (733 mg, 2.05 mmol), and potassium carbonate (567 mg, 4.10 mmol) in sequence. Under a nitrogen atmosphere, the mixture was heated to 100 °C and allowed to react for 9 h. After the reaction was completed, water (100 mL) was added, and the mixture was extracted with ethyl acetate (80 mL × 2). The organic phase was collected, dried over anhydrous sodium sulfate, and filtered, and the solvent was removed by evaporation under reduced pressure using a rotary evaporator. The resulting crude product was purified by silica gel column chromatography (dichloromethane:methanol = 20:1) to give 12-1 (64 mg).

MS (ESI+, [M+H]⁺) *m*/*z:* 364.31.

**Step B:** Referring to Example 9, **12** was obtained by replacing **9-2** with **12-1** in step C.

¹H NMR (500 MHz, DMSO-*d₆*) δ 10.21 (s, 1H), 7.39 (d, 1H), 7.18-6.97 (m, 4H), 6.91 (d, 1H), 6.81-6.59 (m, 2H), 5.85 (d, 1H), 5.35 (d, 1H), 4.32-4.18(m, 1H), 3.08-2.99 (m, 1H), 2.75-2.65 (m, 1H), 2.22 (s, 3H), 2.05-1.85 (m, 4H), 1.80-1.70 (m, 1H), 1.63-1.51 (m, 1H).

HRMS (ESI+, [M+H]⁺) m/z: 350.1980.

### Example 13

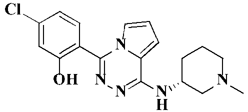

### Reaction process:

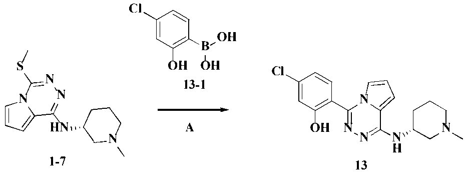

**Step A:** Referring to Example 1, **13** was obtained by replacing 2-hydroxy-4-trifluoromethylphenylboronic acid with **13-1** in step G.

¹H NMR (500 MHz, DMSO-*d₆*) δ 10.67 (s, 1H), 7.43 (d, 1H), 7.04 (d, 4H), 6.92 (d, 1H), 6.71 (s, 1H), 4.25 (s, 1H), 3.02 (d, 1H), 2.69 (d, 1H), 2.21 (s, 3H), 1.91 (s, 3H), 1.74 (d, 1H), 1.58 (d, 1H), 1.41 - 1.32 (m, 1H). HRMS (ESI+, [M+H]+) m/z: 358.1432.

### Example 14

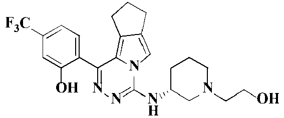

### Reaction process:

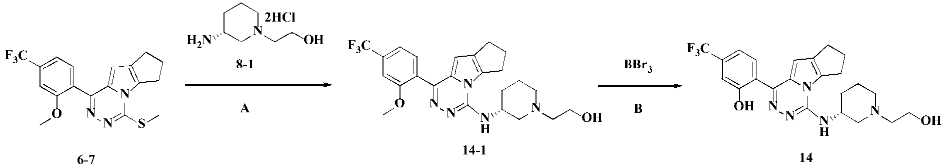

**Step A:** Referring to Example 6, **14-1** was obtained by replacing **2-9** with **8-1** in step G. MS (ESI+, [M+H]⁺) m/z: 476.21.

**Step B:** Referring to Example 6, **14** was obtained by replacing **6-8** with **14-1** in step H.

¹H NMR (500 MHz, DMSO-*d₆*) δ 11.81 (s, 1H), 7.74 - 7.65 (m, 2H), 7.26 (s, 1H), 7.25 (s, 1H), 7.07 (d, 1H), 4.50 - 4.36 (m, 1H), 4.35 - 4.17 (m, 1H), 3.60 - 3.44 (m, 2H), 3.20 - 3.03 (m, 1H), 2.80 (t, 3H), 2.65 (t, 2H), 2.49 - 2.37 (m, 2H), 2.34 - 2.24 (m, 2H), 2.19 - 1.92 (m, 3H), 1.75 (d, 1H), 1.65 - 1.52 (m, 1H), 1.51 - 1.40 (m, 1H). HRMS (ESI+, [M+H]⁺) m/z: 462.2120.

### Example 15

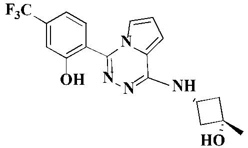

### Reaction process:

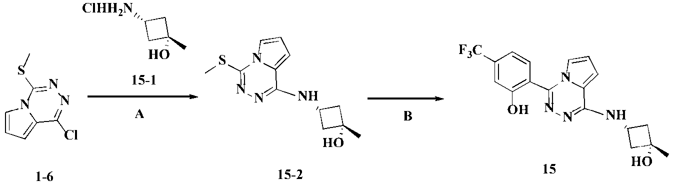

**Step A:** Referring to Example 1, **15-2** was obtained by replacing (R)-1-methylpiperidin-3-amine with **15-1** in step F.

MS (ESI+, [M+H]⁺) *m*/*z:* 265.25.

Step **B:** Referring to Example 1, **15** was obtained by replacing **1-7** with **15-2** in step G.

¹H NMR (500 MHz, DMSO-*d₆*) δ 10.90 (s, 1H), 7.64 (d, 1H), 7.43 (d, 1H), 7.30 (d, 2H), 7.05 (dd, 2H), 6.71 (s, 1H), 5.02 (s, 1H), 4.23 - 4.09 (m, 1H), 2.47 - 2.39 (m, 2H), 2.18 - 2.06 (m, 2H), 1.31 (s, 3H).

HRMS (ESI+, [M+H]⁺) m/z: 379.1386.

### Example 16

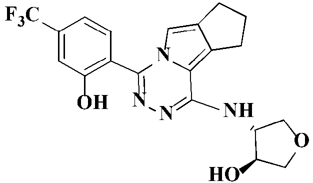

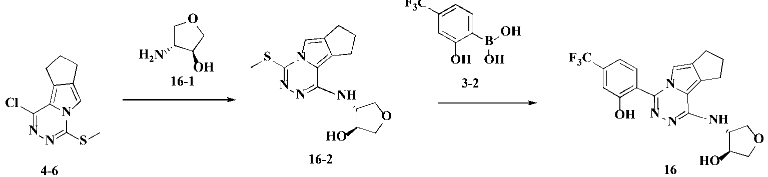

**Step A:** To a 25-mL single-necked flask were added **4-6**, **16-1**, Pd₂(dba)₃ (19 mg, 0.021 mmol), BINAP (26 mg, 0.042 mmol), Cs₂CO₃ (136 mg, 0.417 mmol), and 1,4-dioxane (2 mL) in sequence. Under an N₂ atmosphere, the mixture was heated to 90 °C and allowed to react. After the reaction was completed, the solvent was removed by evaporation under reduced pressure using a rotary evaporator. The resulting crude product was purified by silica gel column chromatography (DCM:MeOH = 50:1) to give **16-2** (36 mg).

¹H NMR (500 MHz, DMSO-*d₆*) *δ* 7.15 (s, 1H), 5.88 (d, 1H), 5.36 (d, 1H), 4.35 - 4.29 (m, 1H), 4.25 - 4.19 (m, 1H), 4.09 (dd, 1H), 3.92 (dd, 1H), 3.71 (dd, 1H), 3.54 (dd, 1H), 3.06 - 2.93 (m, 2H), 2.74 (dd, 2H), 2.66 (s, 3H), 2.40 - 2.34 (m, 2H).

MS (ESI+, [M+H]⁺) *m*/*z*: 306.98.

**Step B:** Referring to Example 3, **16** was obtained by replacing **3-1** with **16-2** in step B.

¹H NMR (500 MHz, DMSO-*d₆*) *δ* 10.84 (s, 1H), 7.63 (d, 1H), 7.31 (d, 2H), 6.77 (s, 1H), 6.24 (s, 1H), 4.43 - 4.31 (m, 2H), 4.13 (dd, 1H), 3.97 (dd, 1H), 3.76 (dd, 1H), 3.57 (dd, 1H), 3.11 - 2.96 (m, 2H), 2.69 (t, 2H), 2.40 - 2.34 (m, 2H).

HRMS (ESI+, [M+H]⁺) m/z: 421.1494.

### Example 17

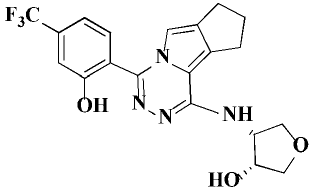

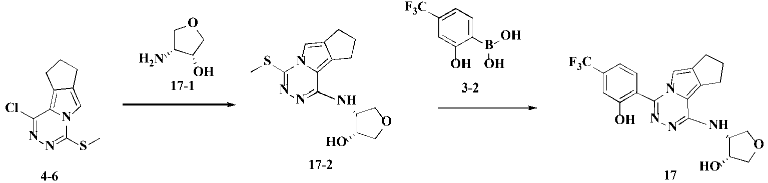

**Step A:** Referring to Example 16, **17-2** was obtained by replacing **16-1** with **17-1** in step A. MS (ESI+, [M+H]⁺) m/z: 306.98.

**Step B:** Referring to Example 3, **17** was obtained by replacing **3-1** with **17-2** in step B.

¹H NMR (500 MHz, DMSO-*d₆*) *δ* 10.85 (s, 1H), 7.66 (d, 1H), 7.31 (d, 2H), 6.75 (s, 1H), 6.24 (s, 1H), 5.61 (s, 1H), 4.48 - 4.33 (m, 2H), 4.17 (dd, 1H), 3.94 (dd, 1H), 3.76 (dd, 1H), 3.55 (dd, 1H), 3.14 - 2.92 (m, 2H), 2.72 (t, 2H), 2.42 - 2.35 (m, 2H).

HRMS (ESI+, [M+H]⁺) m/z: 421.1482.

### Example 18

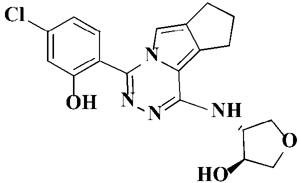

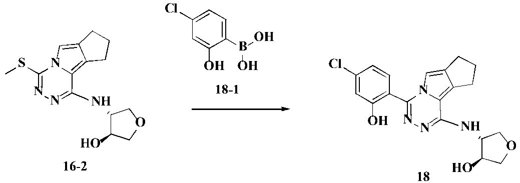

**Step A:** Referring to Example 16, **18** was obtained by replacing **3-2** with **18-1** in step B.

¹H NMR (500 MHz, DMSO-*d₆*) *δ* 10.84 (s, 1H), 7.41 (d, 1H), 7.07 - 6.98 (m, 2H), 6.72 (s, 1H), 6.02 (d, 1H), 5.44 (s, 1H), 4.42 - 4.26 (m, 2H), 4.12 (dd, 1H), 3.96 (dd, 1H), 3.75 (dd, 1H), 3.56 (dd, 1H), 3.11 - 2.95 (m, 2H), 2.68 (t, 2H), 2.40 - 2.34 (m, 2H).

HRMS (ESI+, [M+H]⁺) m/z: 387.1233.

### Example 19

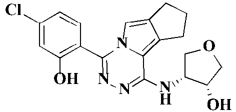

### Reaction process:

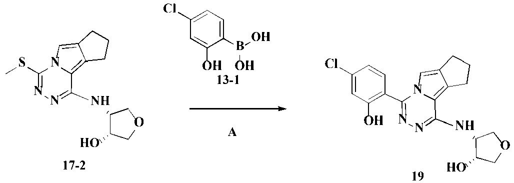

**Step A:** Referring to Example 17, **19** was obtained by replacing **3-2** with **13-1** in step B. HRMS (ESI+, [M+H]⁺) m/z: 387.1224.

### Example 20

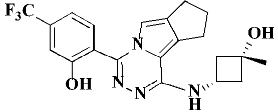

### Reaction process:

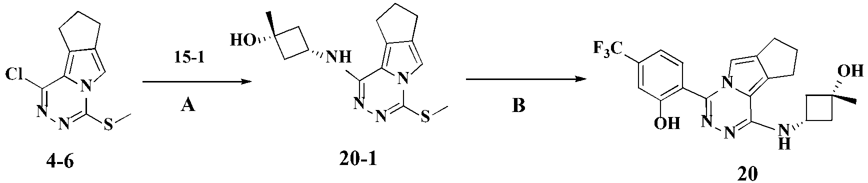

**Step A:** Referring to Example **5**, **20-1** was obtained by replacing **2-9** with **15-1** in step A.

MS (ESI+, [M+H]⁺) *m*/*z:* 305.17.

**Step B:** Referring to Example **5**, **20** was obtained by replacing **5-1** with **20-1** in step B.

¹H NMR (500 MHz, DMSO-*d₆*) δ 11.90 (s, 1H), 7.69 (d, 2H), 7.56 (s, 1H), 7.27 (dd, 1H), 7.24 (s, 1H), 5.05 (s, 1H), 4.22 - 4.11 (m, 1H), 2.81 (t, 2H), 2.66 (t, 2H), 2.47-2.44 (m, 2H), 2.29 (p, 2H), 2.18-2.15 (m, 2H), 1.33 (s, 3H).

HRMS (ESI+, [M+H]⁺) m/z: 419.1701.

### Example 21

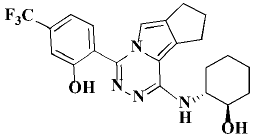

### Reaction process:

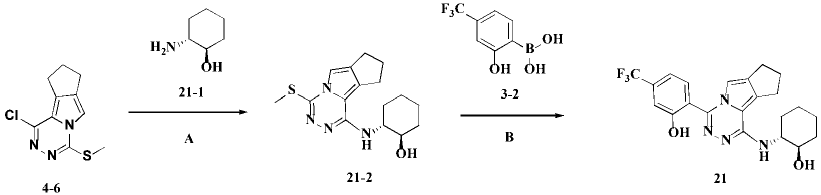

**Step A:** Referring to Example 16, **21-2** was obtained by replacing **16-1** with **21-1** in step A. MS (ESI+, [M+H]⁺) *m*/*z:* 319.19.

**Step B:** Referring to Example 16, **21** was obtained by replacing **16-2** with **21-2** in step B.

¹H NMR (500 MHz, DMSO-*d₆*) δ 10.83 (s, 1H), 7.60 (d, 1H), 7.30 (d, 2H), 6.72 (s, 1H), 5.56 (d, 1H), 5.08 (d, 1H), 3.82 (d, 1H), 3.52 (d, 1H), 3.02 (t, 2H), 2.69 (t, 2H), 2.39 (q, 2H), 2.19 (d, 1H), 1.94 (d, 1H), 1.67 (d, 2H), 1.33 - 1.26 (m, 4H).

HRMS (ESI+, [M+H]⁺) m/z: 433.1856.

### Example 22

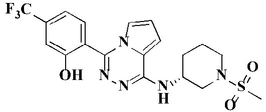

### Reaction process:

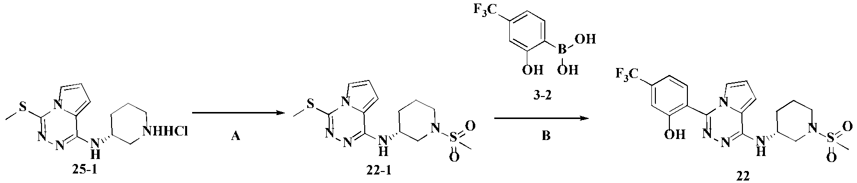

**Step A:** To a 100-mL single-necked flask were added 25-1 (300 mg, 1.0 mmol), dichloromethane (80 mL), TEA (388 mg, 3.0 mmol), and methanesulfonic anhydride (348 mg, 2.0 mmol) in sequence. The mixture was allowed to react at room temperature for 2 h. After the reaction was completed, water (80 mL) was added, and the mixture was extracted with dichloromethane (50 mL × 2). The organic layers were combined, washed with saturated sodium chloride (80 mL), dried over anhydrous sodium sulfate, and filtered under vacuum, and the filtrate was concentrated by rotary evaporation under reduced pressure to remove the solvent. The resulting crude product was purified by silica gel column chromatography (dichloromethane:methanol = 10:1) to give **22-1** (0.17 g).

MS (ESI+, [M+H]⁺) *m*/*z:* 342.02.

**Step B:** Referring to Example 21, **22** was obtained by replacing **21-2** with **22-1** in step B.

¹H NMR (500 MHz, DMSO-*d₆*) δ 10.82 (s, 1H), 7.67 (d, 1H), 7.32 (d, 2H), 7.18 (s, 1H), 7.09 (d, 2H), 6.77 - 6.73 (m, 1H), 4.30 - 4.22 (m, 1H), 3.92 (dd, 1H), 3.52 (d, 1H), 2.90 (s, 3H), 2.77 (dd, 1H), 2.66 - 2.59 (m, 1H), 2.07 - 2.01 (m, 1H), 1.92 (dd, 1H), 1.70 - 1.56 (m, 2H).

HRMS (ESI+, [M+H]⁺) m/z: 456.1321.

### Example 23

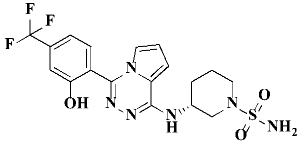

### Reaction process:

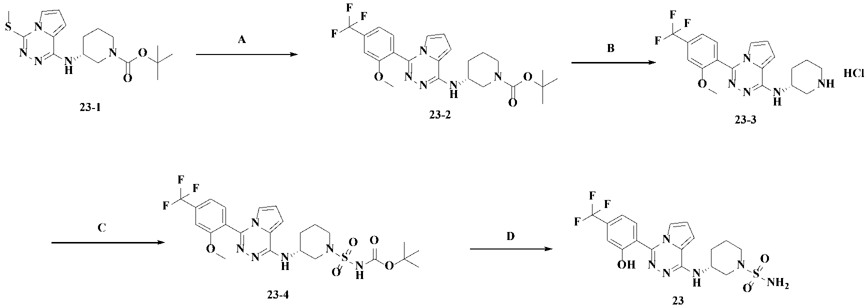

**Step A:** To a 250-mL single-necked flask were added 1,4-dioxane (20 mL), **23-1** (500 mg, 1.376 mmol), 2-7 (908 mg, 4.13 mmol), copper(I) 3-methylsalicylate (886 mg, 4.13 mmol), and Pd(Ph₃P)₄ (397 mg, 0.344 mmol) in sequence. Under a nitrogen atmosphere, the mixture was heated to 100 °C and allowed to react overnight. After the reaction was completed, the solvent was removed by evaporation under reduced pressure using a rotary evaporator. The resulting crude product was purified by silica gel column chromatography (dichloromethane:methanol = 20:1) to give **23-2** (646 mg).

MS (ESI+, [M+H]⁺) m/z: 492.19.

**Step B:** To a 100-mL single-necked flask were added **23-2** (646 mg, 1.314 mmol) and ethyl acetate (5 mL) in sequence. The mixture was cooled to 0 °C, and then 4 M HCl/dioxane (10 mL, 40.0 mmol) was slowly added dropwise via a syringe. After the addition was completed, the mixture was slowly warmed to room temperature and allowed to react overnight. After the reaction was completed, the solvent was removed by evaporation under reduced pressure using a rotary evaporator to give **23-3** (624 mg).

MS (ESI+, [M+H]⁺) m/z: 392.13.

**Step C:** To a 100-mL single-necked flask were added **23-3** (624 mg, 1.458 mmol), dichloromethane (10 mL), Et₃N (738 mg, 1.016 mL, 7.29 mmol), and *tert*-butyl chlorosulfonylcarbamate (1.887 g, 8.75 mmol) in sequence. The mixture was allowed to react at room temperature overnight. After the reaction was completed, the solvent was removed by evaporation under reduced pressure using a rotary evaporator, and then ethyl acetate (150 mL) and water (50 mL) were added. The organic phase was separated, and the aqueous phase was extracted with ethyl acetate (50 mL × 2). The organic phases were combined, washed with saturated brine (50 mL), dried over anhydrous sodium sulfate, and filtered, and the solvent was removed by evaporation under reduced pressure using a rotary evaporator. The resulting crude product was purified by silica gel column chromatography (DCM:MeOH = 20:1) to give **23-4** (320 mg).

MS (ESI+, [M+H]⁺) m/z: 571.26.

**Step D:** To a 50-mL two-necked reaction flask were added **23-4** (150 g, 0.263 mmol) and DCM (5 mL) in sequence. Under a nitrogen atmosphere, the mixture was cooled in an ice-water bath for 10 min, and then boron tribromide (330 mg, 0.659 mL, 1.317 mmol) was added. After the addition was completed, the mixture was slowly warmed to room temperature and allowed to react for 2 h. After the reaction was completed, the reaction solution was slowly poured into water (50 mL) and extracted with ethyl acetate (2 × 125 mL). The organic layers were combined, washed with saturated brine (50 mL), dried over anhydrous sodium sulfate, and filtered, and the solvent was removed by evaporation under reduced pressure using a rotary evaporator. The resulting crude product was subjected to preparative liquid chromatography (CHIRAL ART Cellulose-SB chromatographic column; water (10 mM ammonium acetate + 0.1% glacial acetic acid)-acetonitrile (62:38)) to give compound 23 (15 mg).

¹H NMR (500 MHz, DMSO-*d₆*) δ 10.82 (s, 1H), 7.66 (d, 1H), 7.40 - 7.22 (m, 2H), 7.11 (d, 1H), 7.07 (d, 2H), 6.86 - 6.62 (m, 3H), 4.32 (d, 1H), 3.79 (d, 1H), 3.41 (d, 1H), 2.61 - 2.53 (m, 1H), 2.47 - 2.39 (m, 1H), 2.00 (d, 1H), 1.90 (d, 1H), 1.63 (d, 1H), 1.52 (q, 1H).

HRMS (ESI+, [M+H]⁺) m/z: 457.1277.

### Example 24

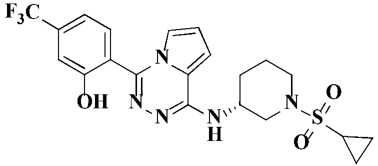

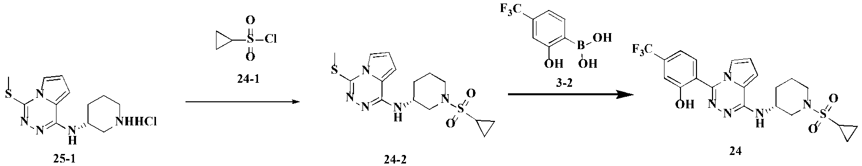

**Step A:** To a 25-mL single-necked flask were added **25-1** (200 mg, 0.67 mmol), DCM (10 mL), DIPEA (259 mg, 2.00 mmol), and **24-1** in sequence. The reaction solution was stirred at room temperature. After the reaction was completed, the solvent was removed by evaporation under reduced pressure using a rotary evaporator. The resulting crude product was purified by silica gel column chromatography (DCM:MeOH = 20:1) to give **24-2** (220 mg).

¹H NMR (500 MHz, DMSO-*d₆*) *δ* 7.46 (dd, 1H), 7.08 (dd, 1H), 6.93 (s, 1H), 6.84 (dd, 1H), 4.20 - 4.08 (m, 1H), 3.92 (dd, 1H), 3.55 (d, 1H), 3.35 - 3.31 (m, 1H), 2.93 - 2.79 (m, 1H), 2.72 - 2.66 (m, 3H), 2.63 - 2.50 (m, 1H), 2.07 - 2.00 (m, 1H), 1.94 - 1.85 (m, 1H), 1.69 - 1.52 (m, 2H), 1.01 - 0.90 (m, 4H).

MS (ESI+, [M+H]⁺) m/z: 368.09.

**Step B:** Referring to Example 3, **24** was obtained by replacing **3-1** with **24-2** in step B.

¹H NMR (500 MHz, DMSO-*d₆*) δ 11.94 (s, 1H), 10.83 (s, 1H), 7.67 (d, 1H), 7.32 (d, 2H), 7.08 (dd, 2H), 6.78 - 6.73 (m, 1H), 4.27 (d, 1H), 3.96 (dd, 1H), 3.59 (d, 1H), 2.87 (dd, 1H), 2.78 - 2.69 (m, 1H), 2.67 - 2.59 (m, 1H), 2.11 - 2.02 (m, 1H), 1.94 - 1.85 (m, 1H), 1.72 - 1.55 (m, 2H), 1.03 - 0.96 (m, 4H).

HRMS (ESI+, [M+H]⁺) m/z: 482.1483.

### Example 25

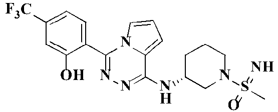

### Reaction process:

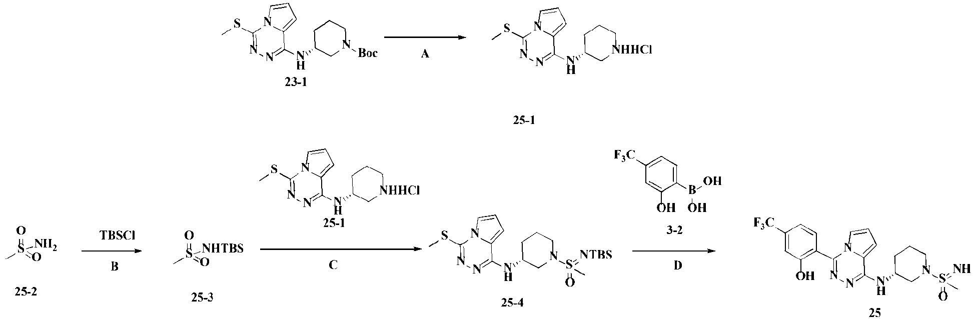

**Step** A: To a 100-mL single-necked flask were added **23-1** (1.8 g, 4.95 mmol), 1,4-dioxane (20 mL), and a solution of hydrogen chloride in 1,4-dioxane (4 N, 18.57 mL, 74.3 mmol) in sequence. The mixture was allowed to react at room temperature for 2 h. After the reaction was completed, the solvent was removed by rotary evaporation under reduced pressure. The resulting crude product was slurried with ethyl acetate (30 mL) and filtered under vacuum. The filter cake was dried to give **25-1** (1.5 g).

MS (ESI+, [M+H]⁺) *m*/*z*: 264.24.

**Step B:** To a 250-mL single-necked flask were added **25-2** (5.0 g, 52.6 mmol), dichloromethane (100 mL), TBSCl (10.3 g, 68.3 mmol), and TEA (10.64 g, 105 mmol) in sequence. The mixture was allowed to react in an ice-water bath for 4 h. After the reaction was completed, the reaction solution was concentrated by rotary evaporation under reduced pressure to remove the solvent. Water (150 mL) and ethyl acetate (150 mL) were added to the resulting crude product. The phases were separated, and the organic phase was washed with water (100 mL × 3), washed with saturated sodium chloride (100 mL), dried over anhydrous sodium sulfate, and filtered, and the solvent was removed by evaporation under reduced pressure using a rotary evaporator to give **25-3** (9.2 g).

¹H NMR (500 MHz, CDCl3) δ 4.60 (s, 1H), 3.01 (s, 3H), 0.95 (s, 9H), 0.29 (s, 6H).

**Step C:** To a 50-mL single-necked flask were added triphenylphosphine dichloride (1.07 g, 3.21 mmol), chloroform (8 mL), TEA (0.58 g, 5.73 mmol), and **25-3** (300 mg, 1.43 mmol) in sequence. The mixture was allowed to react in an ice-water bath for 0.5 h. TEA (0.29 g, 2.87 mmol) and **25-1** (344 mg, 1.146 mmol) were added, and the resulting mixture was allowed to react at room temperature for 0.5 h. After the reaction was completed, the solvent was removed by rotary evaporation under reduced pressure. The resulting crude product was purified by silica gel column chromatography (petroleum ether:ethyl acetate = 2:3) to give **25-4** (0.92 g).

MS (ESI+, [M+H]⁺) *m*/*z:* 455.21.

**Step D:** To a 100-mL single-necked flask were added **25-4** (900 mg, 3.21 mmol), 1,4-dioxane (60 mL), 3-2 (815 mg, 3.96 mmol), copper(I) 3-methylsalicylate (850 mg, 3.96 mmol), and tetrakis(triphenylphosphine)palladium (572 mg, 0.49 mmol) in sequence. The mixture was heated to 100 °C and allowed to react for 16 h. After the reaction was completed, the mixture was filtered, and the solvent was removed by evaporation under reduced pressure using a rotary evaporator. The resulting crude product was subjected to preparative liquid chromatography (XB-C18 chromatographic column; water (10 mM ammonium acetate + 0.1% glacial acetic acid)-acetonitrile (65:35)) to give compound **25** (8 mg).

¹H NMR (500 MHz, DMSO-*d₆*) δ 7.52 (d, 1H), 7.24 - 7.11 (m, 1H), 7.10 - 6.92 (m, 4H), 6.77 - 6.58 (m, 1H), 4.39 - 4.21 (m, 1H), 4.05 - 3.95 (m, 1H), 3.62-3.53 (m, 3H), 2.75 (s, 3H), 2.70 - 2.61 (m, 1H), 2.08 - 1.86 (m, 2H), 1.68 - 1.44 (m, 2H).

HRMS (ESI+, [M+H]+) m/z: 455.1490.

### Example 26

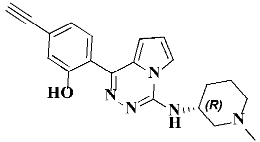

### Reaction process:

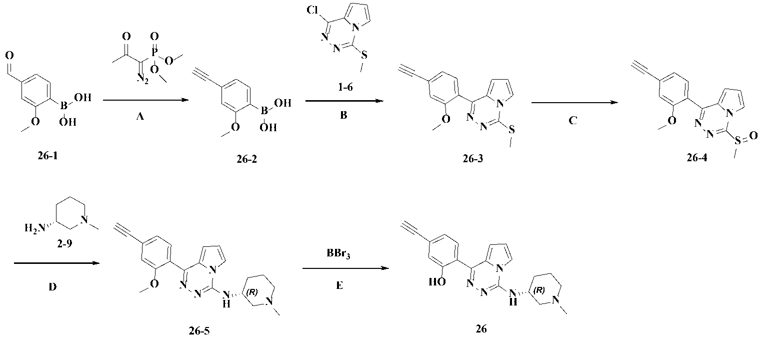

**Step A:** To a 100-mL single-necked flask were added **26-1** (500 mg, 2.78 mmol), methanol (50 mL), sodium carbonate (883 mg, 8.33 mmol), and dimethyl (1-diazo-2-oxopropyl)phosphonate (1.60 g, 8.33 mmol) in sequence. Under a nitrogen atmosphere, the mixture was allowed to react at room temperature for 1.0 h. After the reaction was completed, water (80 mL) was added, and the mixture was extracted with dichloromethane (50 mL × 2). The organic layers were combined, washed with saturated brine (50 mL), dried over anhydrous sodium sulfate, filtered, and concentrated. The resulting crude product was purified by silica gel column chromatography (PE:EA= 5:1) to give **26-2** (260 mg).

¹H NMR (500 MHz, DMSO-*d₆*) *δ* 7.79 (s, 2H), 7.50 (d, 1H), 7.07 - 7.01 (m, 2H), 4.23 (s, 1H), 3.81 (s, 3H).

MS (ESI-, [M-H]⁻) *m*/*z:* 175.19.

**Step B:** To a 100-mL single-necked flask were added **1-6** (500 mg, 2.50 mmol), 1,4-dioxane (45 mL), **26-2** (881 mg, 5.01 mmol), XphosPdG3 (318 mg, 0.376 mmol), potassium carbonate (1.04 g, 7.51 mmol), and water (15 mL) in sequence. Under a nitrogen atmosphere, the mixture was heated to 90 °C and allowed to react for 5 h. After the reaction was completed, the solvent was removed by evaporation under reduced pressure. The resulting crude product was purified by silica gel column chromatography (PE:EA = 3:1) to give **26-3** (445 mg).

¹H NMR (500 MHz, DMSO-*d₆*) δ 7.70 (dd, 1H), 7.44 (d, 1H), 7.30 (d, 1H), 7.25 - 7.20 (m, 1H), 7.06 (dd, 1H), 6.61 (d, 1H), 4.35 (s, 1H), 3.76 (s, 3H), 2.84 (s, 3H).

MS (ESI+, [M+H]⁺) m/z: 296.04.

**Step C:** To a 100-mL single-necked flask were added **26-3** (520 mg, 1.76 mmol), dichloromethane (30 mL), and m-chloroperoxybenzoic acid (357 mg, 1.76 mmol) in sequence. Under a nitrogen atmosphere, the mixture was allowed to react at room temperature for 1.0 h. After the reaction was completed, dichloromethane (30 mL) was added, and the mixture was washed with a saturated aqueous sodium bicarbonate solution (50 mL), washed with saturated brine (50 mL), dried over anhydrous sodium sulfate, filtered, and concentrated. The resulting crude product was purified by silica gel column chromatography (PE:EA = 1:1) to give **26-4** (190 mg).

¹H NMR (500 MHz, DMSO-*d₆*) δ 8.38 (dd, 1H), 7.47 (d, 1H), 7.34 (d, 1H), 7.25 (dd, 1H), 7.19 (dd, 1H), 6.81 (dd, 1H), 4.39 (s, 1H), 3.78 (s, 3H), 3.28 (s, 3H).

MS (ESI+, [M+H]⁺) *m*/*z*: 312.05.

**Step D:** To a 10-mL reaction flask were added **26-4** (160 mg, 0.514 mmol) and **2-9** (1.17 g, 10.28 mmol) in sequence. Under a nitrogen atmosphere, the mixture was heated to 100 °C and allowed to react for 2.0 h. After the reaction was completed, the solvent was removed by evaporation under reduced pressure. The resulting crude product was purified by silica gel column chromatography (DCM:MeOH = 30:1) to give **26-5** (100 mg).

¹H NMR (500 MHz, DMSO-*d₆*) δ 7.91 (dd, 1H), 7.36 (d, 1H), 7.24 (d, 1H), 7.21 - 7.11 (m, 2H), 6.88 (dd, 1H), 6.33 (d, 1H), 4.36 - 4.20 (m, 2H), 3.73 (s, 3H), 3.07 (d, 1H), 2.71 (d, 1H), 2.22 (s, 3H), 2.07 - 1.85 (m, 3H), 1.83 - 1.71 (m, 1H), 1.66 - 1.55 (m, 1H), 1.48 - 1.37 (m, 1H).

MS (ESI+, [M+H]+) m/z: 362.26.

**Step E:** Referring to Example 2, **26** was obtained by replacing **2-10** with **26-5** in step H.

¹H NMR (500 MHz, DMSO-*d₆*) δ 13.41 (s, 1H), 8.16 (s, 1H), 7.97 (d, 1H), 7.64 (s, 1H), 7.22 (d, 1H), 7.13 - 7.02 (m, 3H), 4.44-4.24 (m, 2H), 3.17 (d, 1H), 2.89 - 2.74 (m, 1H), 2.32 (s, 3H), 2.18 - 1.92 (m, 3H), 1.87-1.77 (m, 1H), 1.70-1.60 (m, 1H), 1.57-1.43 (m, 1H).

HRMS (ESI+, [M+H]⁺) m/z: 348.1811.

### Experimental Example 1: In-Vitro Anti-Pyroptosis Activity Assay in Cells

### 1.1 Anti-pyroptosis activity assay in J774A.1 cells

J774A.1 cells in good growth status were collected, washed with PBS, and digested with trypsin. Then, the digestion was terminated with a complete medium. The cells were collected into a centrifuge tube, and the cell density was adjusted to 8 × 10⁵ cells/mL. The cells were then seeded into a 96-well plate (100 µL/well), and the plate was incubated at 37 °C with 5% CO₂ overnight. The supernatant was then discarded, and serum lipase (manufacturer: Sigma; 50 µL/well) was added at a final concentration of 200 ng/mL. The plate was incubated at 37 °C with 5% CO₂ for 3 h. Compounds were added using a nanoliter pipettor to achieve final compound concentrations of 10000 nM-4.57 nM, with 2 replicate wells set for each concentration. The plate was incubated at 37 °C with 5% CO₂ for 1 h. Nigericin (manufacturer: Millipore; 50 µL/well) was added at a final concentration of 10 µM, and the plate was incubated at 37 °C with 5% CO₂ for 4 h. The detection reagent CCK-8 (manufacturer: Dojindo Laboratories, Beijing: 10 µL/well) was then added, and the plate was incubated at 37 °C with 5% CO₂ for 1 h. Absorbance values were measured at 450 nm using a PerkinElmer Envision microplate reader. Data were analyzed using a four-parameter model to fit the dose-response curve, and then the anti-pyroptosis activity (EC₅₀) was calculated. In the table below, A represents EC₅₀ ≤ 100 nM, B represents 100 nM < EC₅₀ ≤ 1000 nM, and C represents 1000 nM < EC₅₀.

The test results are shown in Table 1.

**Table 1**

| Example | EC₅₀ nM |
|---|---|
| 1 | A |
| 2 | A |
| 3 | B |
| 4 | B |
| 5 | A |
| 6 | B |
| 9 | A |
| 13 | A |
| 15 | B |
| 16 | B |
| 17 | B |
| 18 | B |
| 21 | A |
| 22 | B |
| 23 | A |
| 24 | B |
| 25 | B |

### Experimental Example 2: In-Vitro Cell (IL-1β) Expression Assay

### 2.1. In-vitro THP-1 cell (IL-1β) expression assay

THP-1 cells in good growth status were collected into a centrifuge tube, and the cell density was adjusted to 2 × 10⁶ cells/mL. The cells were then seeded into a 96-well plate (50 µL/well), and meanwhile, LPS (manufacturer: Sigma; 50 µL/well) was added at a final concentration of 100 ng/mL. The plate was then incubated at 37 °C with 5% CO₂ for 3 h. Compounds were added to achieve final compound concentrations of 1000 nM, 100 nM, and 10 nM (50 µL/well, with 2 replicate wells set for each concentration). The plate was incubated at 37 °C with 5% CO₂ for 1 h. Nigericin (manufacturer: Millipore; 50 µL/well) was added at a final concentration of 10 µM, and the plate was incubated at 37 °C with 5% CO₂ for 1 h and then centrifuged at 1500 rpm for 3 min. The supernatant was collected and stored at -20 °C for subsequent detection of human IL-1β.

Human IL-1β detection (manufacturer: R&D): Human IL-1β Capture Antibody (coating antibody) was added to a high-binding 96-well plate at a final concentration of 4 µg/mL (100 µL/well), and the plate was incubated at 4 °C overnight. The supernatant was discarded, and the plate was washed with PBST (300 µL/well, 3 times in total). A 1% BSA-PBS blocking buffer was added (200 µL/well), and the plate was blocked at 25 °C for 2 h. The supernatant was discarded, and the plate was washed with PBST (300 µL/well, 3 times in total). A standard curve sample and the diluted samples to be tested were then added (100 µL/well), and the plate was incubated at 25 °C for 2 h. The supernatant was discarded, and the plate was washed with PBST (300 µL/well, 3 times total). Human IL-1β Detection Antibody (detection secondary antibody) was added at a final concentration of 200 ng/mL (100 µL/well), and the plate was incubated at 25 °C for 2 h. The supernatant was discarded, and the plate was washed with PBST (300 µL/well, 3 times in total). Streptavidin-HRP (detection tertiary antibody) was then added (100 µL/well), and the plate was incubated at 25 °C for 20 min. The supernatant was discarded, and the plate was washed with PBST (300 µL/well, 3 times in total). TMB was added for color development (100 µL/well), and the plate was incubated at 25 °C for 5 min. Finally, 1 M H₂SO₄ was added to terminate the reaction (50 µL/well). Absorbance values were measured at 450/570 nm using a PerkinElmer Envision microplate reader. Data were analyzed using a four-parameter model to calculate the IL-1β expression amount and inhibition rate. The test results are shown in Table 2, where A represents % inhibition ≥ 50, B represents 10 ≤ % inhibition < 50, and C represents % inhibition < 10.

**Table 2**

| Example | 100 nM (% inhibition) | 10 nM (% inhibition) | 1 nM (% inhibition) |
|---|---|---|---|
| 1 | -- | A | A |
| 2 | A | B | B |
| 3 | A | B | B |
| 4 | A | B | B |
| 5 | A | A | B |
| 6 | A | B | B |
| 9 | A | A | A |
| 12 | A | A | A |

### Experimental Example 3: Evaluation on In-Vitro Stability in Liver Microsomes

Liver microsome incubation samples were prepared by mixing a PBS buffer (pH 7.4), a liver microsome solution (0.5 mg/mL), a test compound, and an NADPH + MgCl₂ solution and incubated at 37 °C and 300 rpm for 1 h. Zero-hour samples were prepared by mixing a PBS buffer (pH 7.4), a liver microsome solution (0.5 mg/mL), and a test compound. An acetonitrile solution containing an internal standard was added to the samples, and supernatants were prepared by protein precipitation, diluted, and then assayed by LC/MS/MS. The experimental results are shown in Table 3, indicating that the compounds of the present disclosure exhibited relatively good stability in human liver microsomes.

**Table 3**

| Example No. | Human |
|---|---|
| | Remaining amount at 60 min (%) |
| 1 | 95.57% |
| 2 | 80.23% |
| 5 | 72.41% |
| 7 | 90.01% |
| 8 | 79.13% |
| 9 | 98.65% |
| 10 | 99.00% |
| 11 | 75.56% |
| 12 | 100.53% |
| 13 | 102.63% |
| 14 | 93.13% |
| 23 | 93.93% |
| 25 | 98.06% |

### Experimental Example 4: Pharmacokinetic Evaluation in Mice

ICR mice weighing 18-22 g were randomized into groups of 9 after 3-5 days of acclimatization. The intragastric administration group was intragastrically given a test compound solution at a dose of 10 mg/kg.

After administration, plasma samples to be tested were prepared by taking blood from the orbit at time points of 15 min, 0.5 h, 1 h, 2 h, 4 h, 6 h, 8 h, 10 h, and 24 h.

30 µL of each of the plasma samples to be tested and a standard curve sample were taken, and an acetonitrile solution containing an internal standard was added. Supernatants were obtained by protein precipitation, diluted, and then assayed by LC/MS/MS.

Pharmacokinetic parameters were fitted using a non-compartmental model.

The experimental results indicated that the compound of the present disclosure demonstrated relatively good pharmacokinetic properties and exhibited high bioavailability.

### Experimental Example 5: Pharmacokinetic Evaluation in Rats

SD rats weighing 180-220 g were randomized into groups of 3 after 3-5 days of acclimatization. The intragastric administration group was intragastrically given a test compound solution at a dose of 10 mg/kg, and the intravenous injection group was intravenously injected with a test compound solution at a dose of 1 mg/kg.

In the intragastric administration group, the blood sampling time points were 0.25 h, 0.5 h, 1 h, 2 h, 4 h, 6 h, 8 h, 10 h, 24 h, 30 h, and 48 h.

In the intravenous injection administration group, the blood sampling time points were 0.083 h, 0.25 h, 0.5 h, 1 h, 2 h, 4 h, 6 h, 8 h, 10 h, 24 h, 30 h, and 48 h.

Plasma samples to be tested were prepared by taking blood from the orbit.

30 µL of each of the plasma samples to be tested and a standard curve sample were taken, and an acetonitrile solution containing an internal standard was added. Supernatants were obtained by protein precipitation, diluted, and then assayed by LC/MS/MS.

The experimental results indicated that the compound of the present disclosure demonstrated relatively good pharmacokinetic properties and exhibited high bioavailability.

### Experimental Example 6: Pharmacodynamic Evaluation of Compound in Lipopolysaccharide (LPS)-Induced Peritonitis Mouse Model

Male C57BL/6 mice (source: Shanghai Lingchang Biotechnology Co., Ltd.) were randomized into groups of 6 based on body weight. On experimental days 1 and 2, the mice were intragastrically given the drug once daily. On day 3, the mice were intragastrically given the drug for pre-treatment (0 h), and 1 h later, the modeling agent lipopolysaccharide (LPS) was intraperitoneally injected (15 mg/kg). The LPS was dissolved and prepared in PBS immediately before use. At 3 h, animal serum was collected for detection of IL-1β.

The experimental results indicated that the compound of the present disclosure demonstrated relatively good *in-vivo* anti-inflammatory efficacy, with a dose-response relationship in its efficacy.

## Claims

1. A compound of formula (I) or a pharmaceutically acceptable salt thereof: 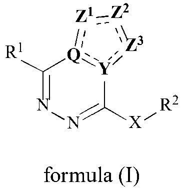 wherein
the structural unit 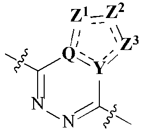 comprises fused bicyclic heteroaryl;
Q and Y are each independently selected from the group consisting of C, Si, and N, and one of Q and Y is N;
Z¹, Z², and Z³ are each independently selected from the group consisting of C(R³), Si(R³), and N;
X is selected from the group consisting of -N(R⁴)-(C(R⁵)(R⁶))ₘ-, -O-(C(R⁵)(R⁶))ₘ-, -S-(C(R⁵)(R⁶))ₘ-, and -(C(R⁵)(R⁶))ₘ-;
R¹ is selected from the group consisting of C₃₋₈ cycloalkyl, 3- to 8-membered heterocycloalkyl, C₆₋₁₀ aryl, and 5-to 9-membered heteroaryl, the C₃₋₈ cycloalkyl, 3- to 8-membered heterocycloalkyl, C₆₋₁₀ aryl, or 5- to 9-membered heteroaryl being optionally and independently substituted with 1, 2, or 3 R^{a};
R² is selected from the group consisting of C₁₋₆ alkyl, C₂₋₆ alkenyl, C₂₋₆ alkynyl, -(CH₂)ᵢC₃₋₈ cycloalkyl, -(CH₂)ᵢ-(3- to 8-membered heterocycloalkyl), -(CH₂)ᵢ-(C₆₋₁₀ aryl), and -(CH₂)ᵢ-(5- to 9-membered heteroaryl), the C₁₋₆ alkyl, C₂₋₆ alkenyl, or C₂₋₆ alkynyl being optionally and independently substituted with 1, 2, or 3 halogens, OH, NH₂, or CN, and the C₃₋₈ cycloalkyl, 3- to 8-membered heterocycloalkyl, C₆₋₁₀ aryl, or 5- to 9-membered heteroaryl being optionally and independently substituted with 1, 2, or 3 R^{b};
each R³ is independently selected from the group consisting of H, OH, CN, NH₂, halogen, C₁₋₃ alkyl, C₁₋₃ alkoxy, C₁₋₃ alkylthio, -NH(C₁₋₃ alkyl), -N(C₁₋₃ alkyl)₂, C₃₋₆ cycloalkyl, and 3- to 6-membered heterocycloalkyl, the C₁₋₃ alkyl, C₁₋₃ alkoxy, C₁₋₃ alkylthio, -NH(C₁₋₃ alkyl), or -N(C₁₋₃ alkyl)₂ being optionally and independently substituted with 1, 2, or 3 halogens, OH, NH₂, or CN, and the C₃₋₆ cycloalkyl or 3- to 6-membered heterocycloalkyl being optionally and independently substituted with 1, 2, or 3 R^{c};
or, Z¹ and Z² are each independently selected from the group consisting of C(R³) and Si(R³), and the R³ groups on Z¹ and Z², together with the atoms to which they are attached, form C₃₋₈ partially saturated cycloalkyl, 3- to 8-membered partially saturated heterocycloalkyl, C₆₋₁₀ aryl, or 5- to 9-membered heteroaryl, the C₃₋₈ partially saturated cycloalkyl, 3- to 8-membered partially saturated heterocycloalkyl, C₆₋₁₀ aryl, or 5- to 9-membered heteroaryl being optionally and independently substituted with 1, 2, or 3 R^{a};
or, Z² and Z³ are each independently selected from the group consisting of C(R³) and Si(R³), and the R³ groups on Z² and Z³, together with the atoms to which they are attached, form C₃₋₈ partially saturated cycloalkyl, 3- to 8-membered partially saturated heterocycloalkyl, C₆₋₁₀ aryl, or 5- to 9-membered heteroaryl, the C₃₋₈ partially saturated cycloalkyl, 3- to 8-membered partially saturated heterocycloalkyl, C₆₋₁₀ aryl, or 5- to 9-membered heteroaryl being optionally and independently substituted with 1, 2, or 3 R^{a};
R⁴ is selected from the group consisting of H, C₁₋₃ alkyl, halogenated C₁₋₃ alkyl, C₃₋₆ cycloalkyl, and 3- to 6-membered heterocycloalkyl, wherein the C₃₋₆ cycloalkyl or 3- to 6-membered heterocycloalkyl may be optionally and independently substituted with 1, 2, or 3 halogens, OH, NH₂, or CN;
R⁵ and R⁶ are each independently selected from the group consisting of H, OH, NH₂, CN, C₁₋₃ alkyl, and halogenated C₁₋₃ alkyl;
each R^{a} is independently selected from the group consisting of OH, CN, NH₂, halogen, =O, C₁₋₃ alkyl, C₁₋₃ alkoxy, C₁₋₃ alkylthio, -NH(C₁₋₃ alkyl), -N(C₁₋₃ alkyl)₂, C₂₋₆ alkenyl, C₂₋₆ alkynyl, -(CH₂)ᵢC₂₋₆ alkenyl, -(CH₂)ᵢC₂₋₆ alkynyl, C₃₋₆ cycloalkyl, and 3- to 6-membered heterocycloalkyl, the C₁₋₃ alkyl, C₁₋₃ alkoxy, C₁₋₃ alkylthio, -NH(C₁₋₃ alkyl), -N(C₁₋₃ alkyl)₂, C₂₋₆ alkenyl, C₂₋₆ alkynyl, -(CH₂)ᵢC₂₋₆ alkenyl, or -(CH₂)ᵢC₂₋₆ alkynyl being optionally and independently substituted with 1, 2, or 3 halogens, OH, NH₂, or CN, and the C₃₋₆ cycloalkyl or 3- to 6-membered heterocycloalkyl being optionally and independently substituted with 1, 2, or 3 R^{d};
each R^{b} is independently selected from the group consisting of OH, CN, NH₂, halogen, =O, C₁₋₃ alkyl, C₁₋₃ alkoxy, C₁₋₃ alkylthio, -NH(C₁₋₃ alkyl), -N(C₁₋₃ alkyl)₂, -S(=O)₂-C₁₋₃ alkyl, -S(=O)₂-NH₂, -S(=O)₂-NH-C₁₋₃ alkyl, -S(=O)₂-C₃₋₆ cycloalkyl, -S(=O)₂-NH-C₃₋₆ cycloalkyl, -S(=O)(NH)-C₁₋₃ alkyl, -S(=O)(NH)-C₃₋₆ cycloalkyl, C₃₋₆ cycloalkyl, and 3- to 6-membered heterocycloalkyl, the C₁₋₃ alkyl, C₁₋₃ alkoxy, C₁₋₃ alkylthio, -NH(C₁₋₃ alkyl), -N(C₁₋₃ alkyl)₂, -S(=O)₂-C₁₋₃ alkyl, -S(=O)₂-NH-C₁₋₃ alkyl, or -S(=O)(NH)-C₁₋₃ alkyl being optionally and independently substituted with 1, 2, or 3 halogens, OH, NH₂, or CN, and the -S(=O)₂-C₃₋₆ cycloalkyl, -S(=O)₂-NH-C₃₋₆ cycloalkyl, -S(=O)(NH)-C₃₋₆ cycloalkyl, C₃₋₆ cycloalkyl, or 3- to 6-membered heterocycloalkyl being optionally and independently substituted with 1, 2, or 3 R^{d};
each R^{c} is independently selected from the group consisting of halogen, OH, CN, NH₂, C₁₋₃ alkyl, and C₁₋₃ halogenated alkyl;
each R^{d} is independently selected from the group consisting of halogen, OH, CN, NH₂, C₁₋₃ alkyl, and C₁₋₃ halogenated alkyl;
each R^{e} is independently selected from the group consisting of halogen, OH, CN, NH₂, C₁₋₃ alkyl, and C₁₋₃ halogenated alkyl;
m is selected from the group consisting of 0, 1, 2, and 3;
i is selected from the group consisting of 0, 1, and 2;
each R⁴, R⁵, R⁶, R^{c}, R^{d}, or R^{e} is optionally and independently substituted with one or more substituents; optionally, each R¹, R², R³, R^{a}, or R^{b} is independently substituted with one or more additional substituents.

2. The compound of formula (I) or the pharmaceutically acceptable salt thereof according to claim 1, wherein Q and Y are each independently selected from the group consisting of C and N, and one of Q and Y is N; Z¹, Z², and Z³ are each independently selected from the group consisting of C(R³) and N.

3. The compound of formula (I) or the pharmaceutically acceptable salt thereof according to claim 2, wherein Z¹ is selected from N, and Z² and Z³ are selected from C(R³); or Z¹ and Z² are selected from N, and Z³ is selected from C(R³); or Z¹ and Z³ are selected from N, and Z² is selected from C(R³); or Z² is selected from N, and Z¹ and Z³ are selected from C(R³); or Z² and Z³ are selected from N, and Z¹ is selected from C(R³); or Z³ is selected from N, and Z¹ and Z² are selected from C(R³); or Z¹, Z², and Z³ are all selected from C(R³); or Z¹, Z², and Z³ are all selected from N.

4. The compound of formula (I) or the pharmaceutically acceptable salt thereof according to claim 1, wherein each R³ is independently selected from the group consisting of H, OH, CN, NH₂, halogen, methyl, ethyl, isopropyl, methoxy, ethoxy, methylthio, ethylthio, -NHCH₃, -N(CH₃)₂, cyclopropyl, cyclobutyl, oxiranyl, thiiranyl, aziridinyl, oxetanyl, thietanyl, and azetidinyl, the methyl, ethyl, isopropyl, methoxy, ethoxy, methylthio, ethylthio, -NHCH₃, or -N(CH₃)₂ being optionally and independently substituted with 1, 2, or 3 halogens, OH, NH₂, or CN, and the cyclopropyl, cyclobutyl, oxiranyl, thiiranyl, aziridinyl, oxetanyl, thietanyl, or azetidinyl being optionally and independently substituted with 1, 2, or 3 R^{c}; preferably, each R³ is independently selected from the group consisting of H, OH, CN, NH₂, F, Cl, Br, I, methyl, ethyl, isopropyl, difluoromethyl, trifluoromethyl, methoxy, ethoxy, difluoromethoxy, and trifluoromethoxy; further preferably, each R³ is independently selected from H.

5. The compound of formula (I) or the pharmaceutically acceptable salt thereof according to claim 1, wherein Z¹ and Z² are each independently selected from C(R³), and the R³ groups on Z¹ and Z², together with the atoms to which they are attached, form C₃₋₈ partially saturated cycloalkyl, 3- to 8-membered partially saturated heterocycloalkyl, C₆₋₁₀ aryl, or 5- to 9-membered heteroaryl, the C₃₋₈ partially saturated cycloalkyl, 3- to 8-membered partially saturated heterocycloalkyl, C₆₋₁₀ aryl, or 5- to 9-membered heteroaryl being optionally and independently substituted with 1, 2, or 3 R^{a}; preferably, Z¹ and Z² are each independently selected from C(R³), and the R³ groups on Z¹ and Z², together with the atoms to which they are attached, form C₅₋₆ partially saturated cycloalkyl, 5- to 6-membered partially saturated heterocycloalkyl containing 1 or 2 ring heteroatoms selected from the group consisting of N, O, and S, or 5-membered heteroaryl containing 1 or 2 ring heteroatoms selected from the group consisting of N, O, and S, the C₅₋₆ partially saturated cycloalkyl, 5- to 6-membered partially saturated heterocycloalkyl, or 5-membered heteroaryl being optionally and independently substituted with 1, 2, or 3 R^{a}; more preferably, Z¹ and Z² are each independently selected from C(R³), and the R³ groups on Z¹ and Z², together with the atoms to which they are attached, form 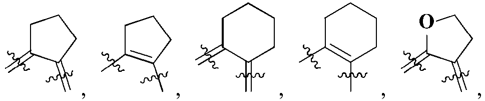 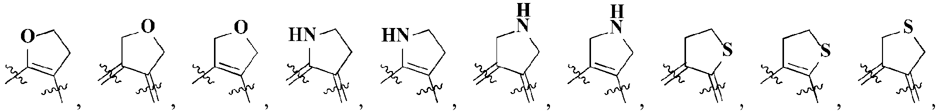 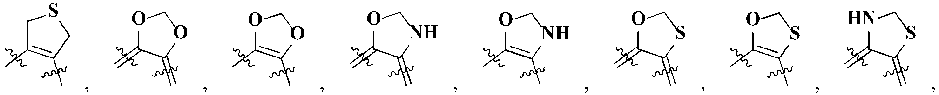 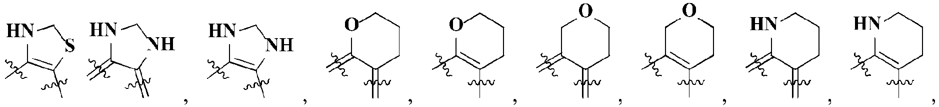 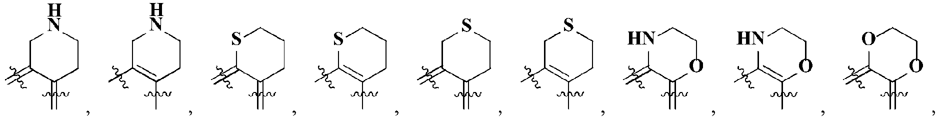 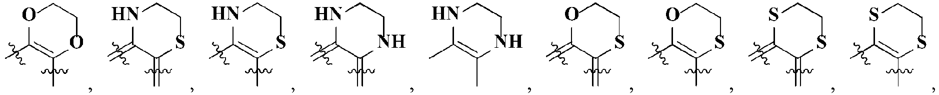 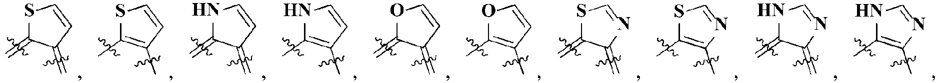 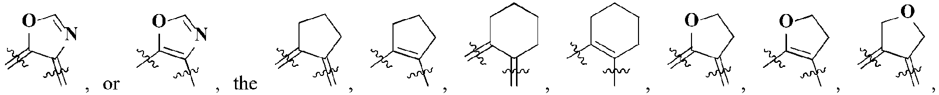 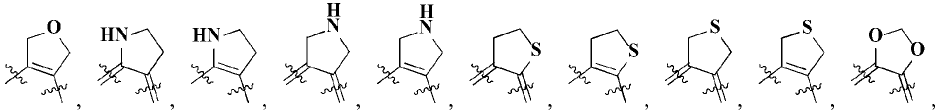 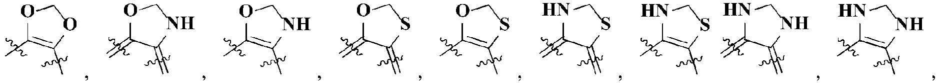 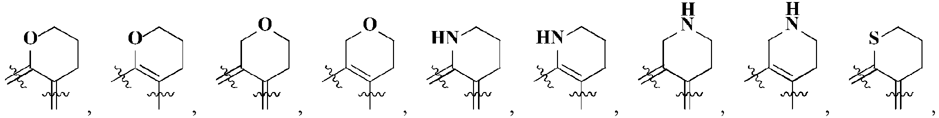 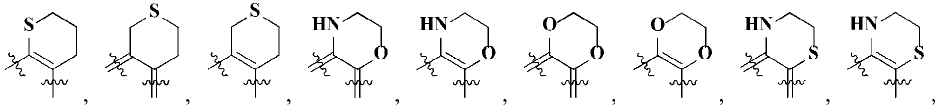 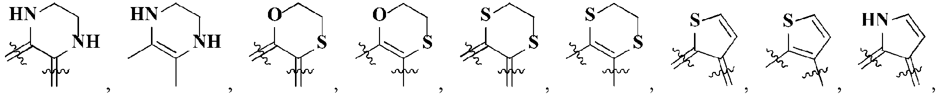 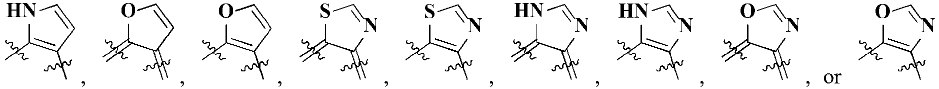 being optionally and independently substituted with 1, 2, or 3 R^{a}.

6. The compound of formula (I) or the pharmaceutically acceptable salt thereof according to claim 1, wherein Z² and Z³ are each independently selected from C(R³), and the R³ groups on Z² and Z³, together with the atoms to which they are attached, form C₃₋₈ partially saturated cycloalkyl, 3- to 8-membered partially saturated heterocycloalkyl, C₆₋₁₀ aryl, or 5- to 9-membered heteroaryl, the C₃₋₈ partially saturated cycloalkyl, 3- to 8-membered partially saturated heterocycloalkyl, C₆₋₁₀ aryl, or 5- to 9-membered heteroaryl being optionally and independently substituted with 1, 2, or 3 R^{a}; preferably, Z² and Z³ are each independently selected from C(R³), and the R³ groups on Z² and Z³, together with the atoms to which they are attached, form C₅₋₆ partially saturated cycloalkyl, 5- to 6-membered partially saturated heterocycloalkyl containing 1 or 2 ring heteroatoms selected from the group consisting of N, O, and S, or 5-membered heteroaryl containing 1 or 2 heteroatoms selected from the group consisting of N, O, and S, the C₅₋₆ partially saturated cycloalkyl, 5- to 6-membered partially saturated heterocycloalkyl, or 5-membered heteroaryl being optionally and independently substituted with 1, 2, or 3 R^{a}; more preferably, Z² and Z³ are each independently selected from C(R³), and the R³ groups on Z² and Z³, together with the atoms to which they are attached, form 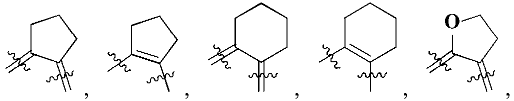 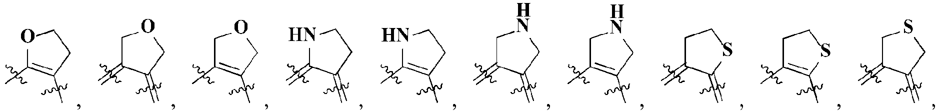 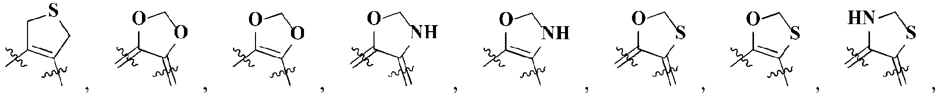 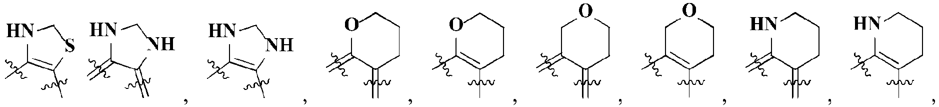 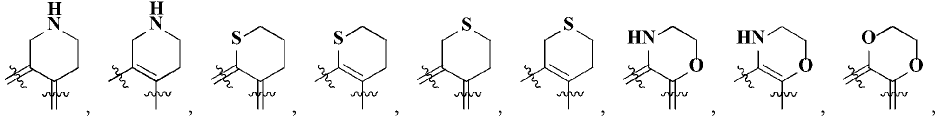 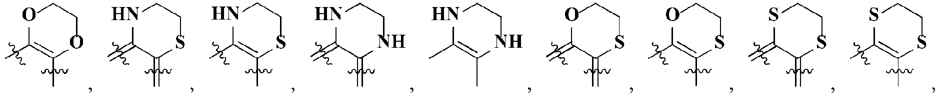 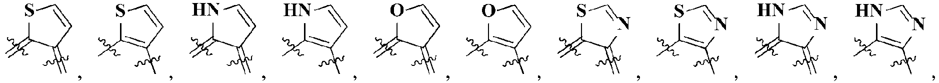 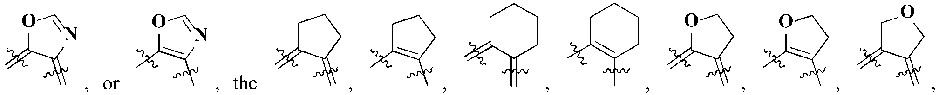 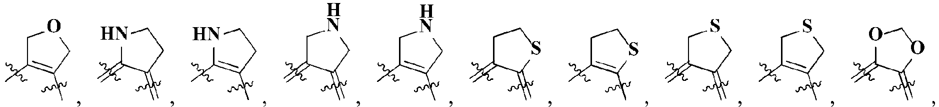 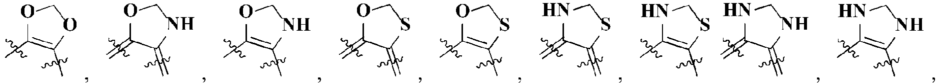 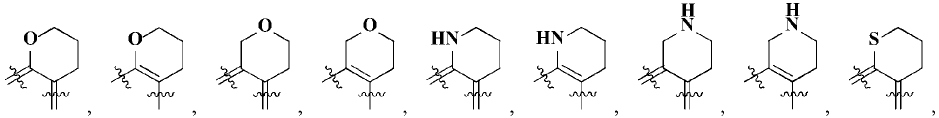 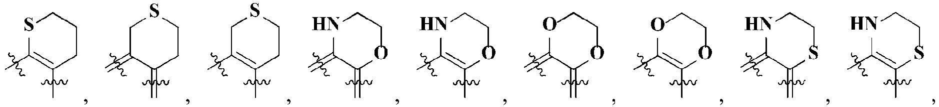 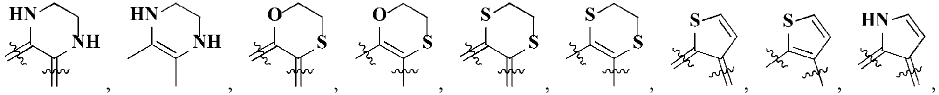 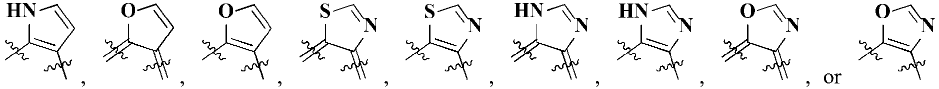 being optionally and independently substituted with 1, 2, or 3 R^{a}.

7. The compound of formula (I) or the pharmaceutically acceptable salt thereof according to claim 4, wherein each R^{c} is independently selected from the group consisting of F, Cl, OH, methyl, and trifluoromethyl; preferably, each R^{c} is independently selected from the group consisting of OH and methyl.

8. The compound of formula (I) or the pharmaceutically acceptable salt thereof according to claim 1, wherein the structural unit 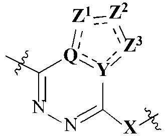 is selected from the group consisting of 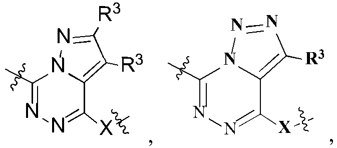 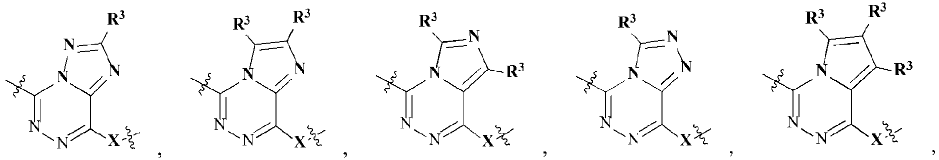 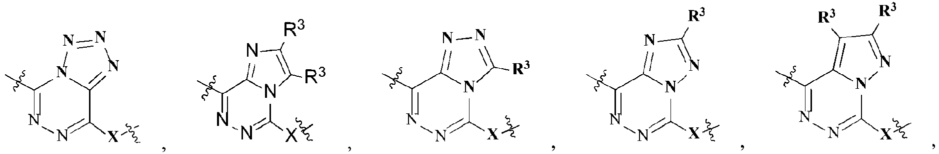 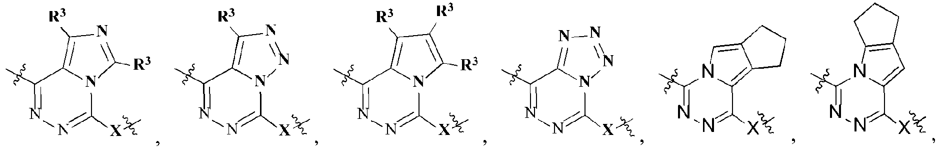 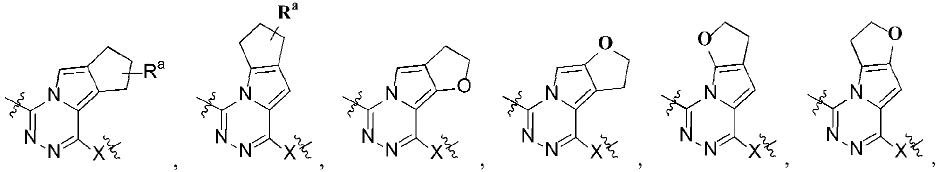 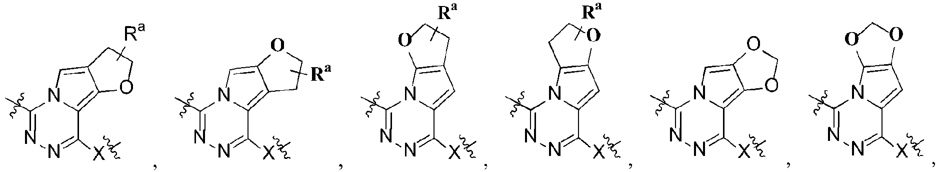 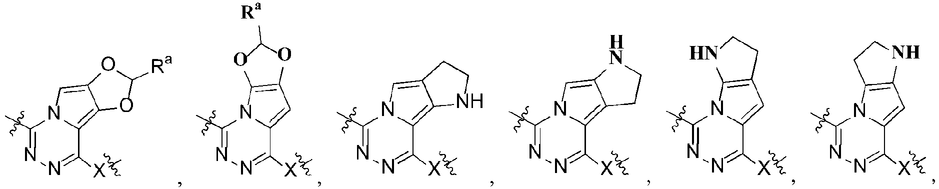 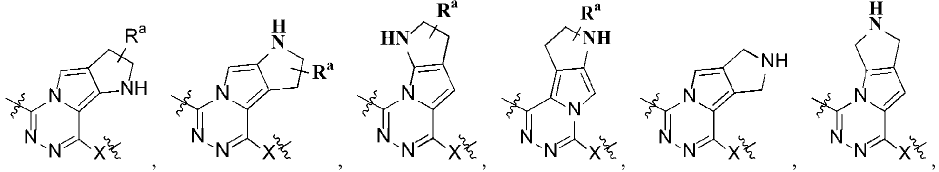 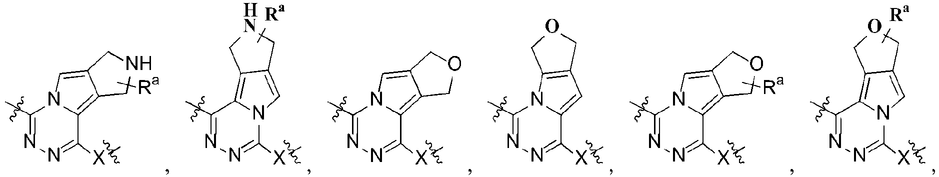 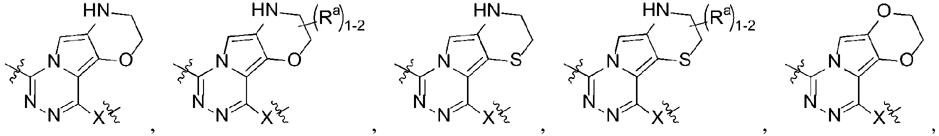 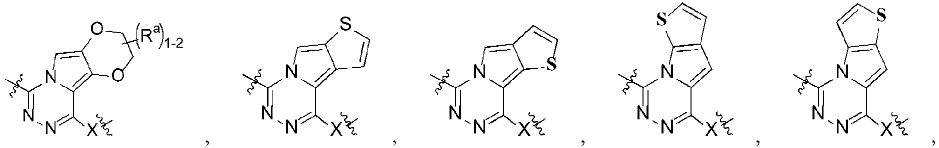 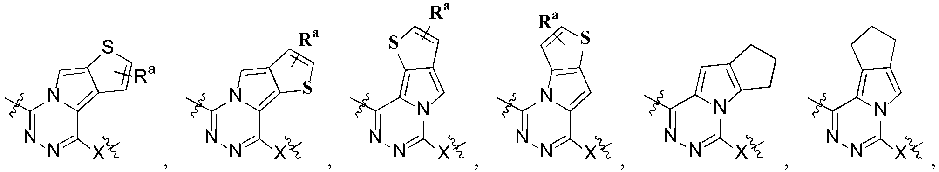 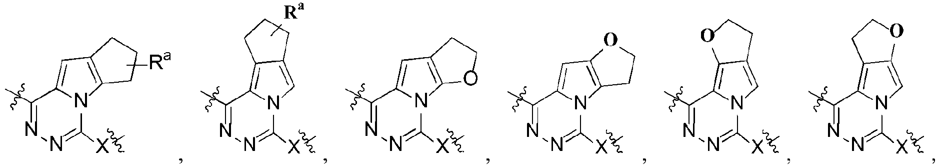 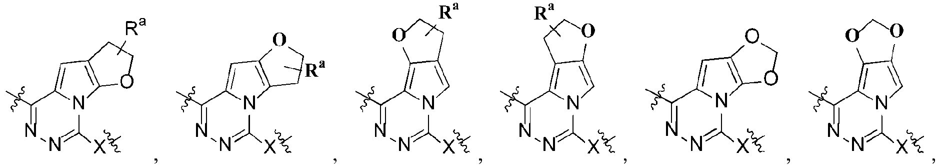 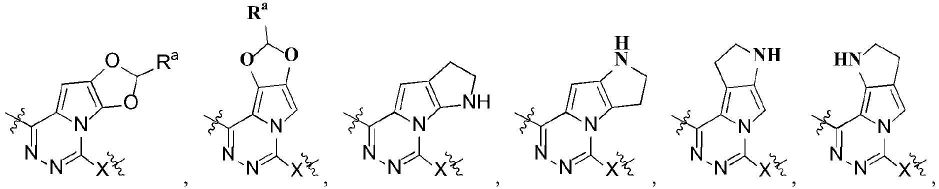 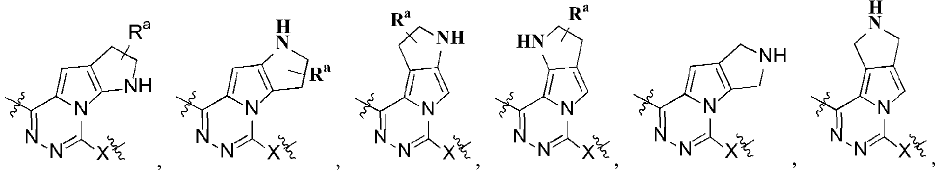 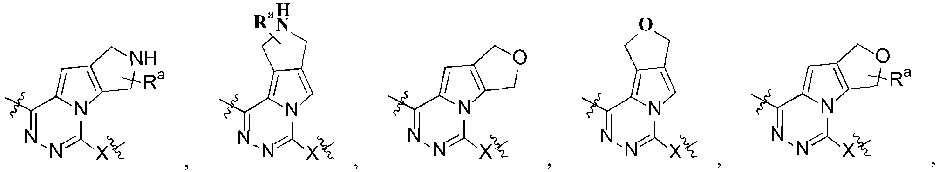 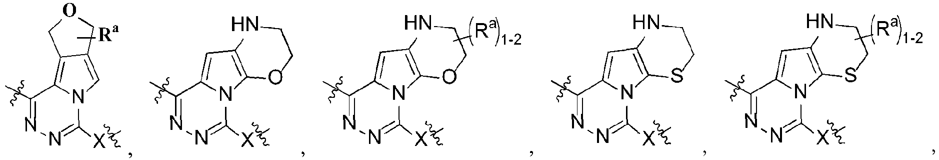 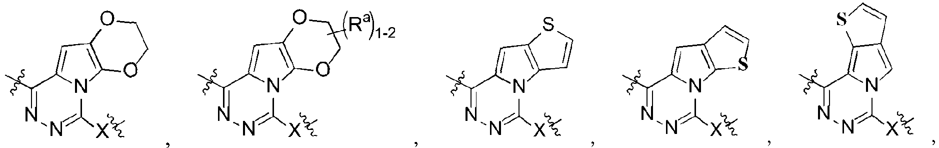 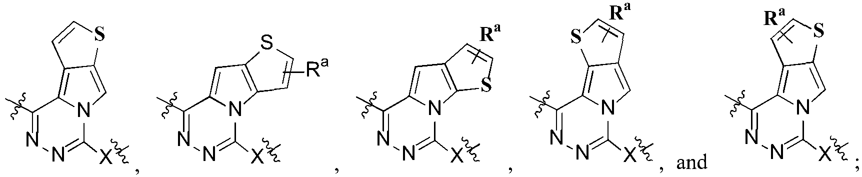 preferably, the structural unit 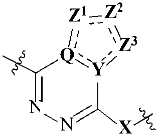 is selected from the group consisting of 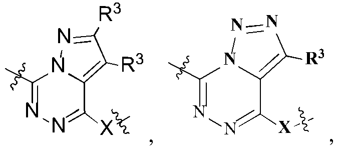 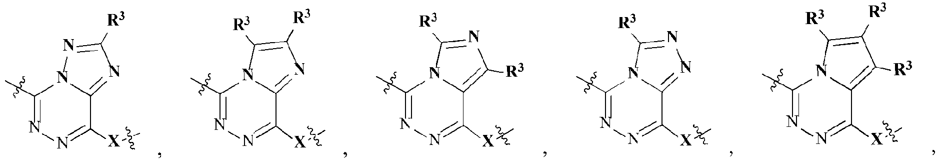 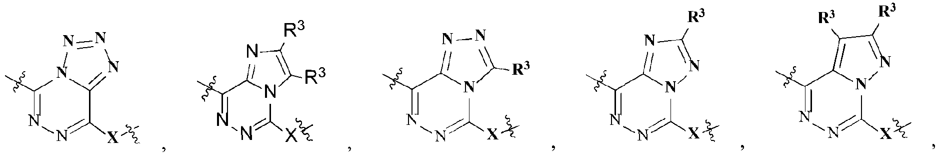 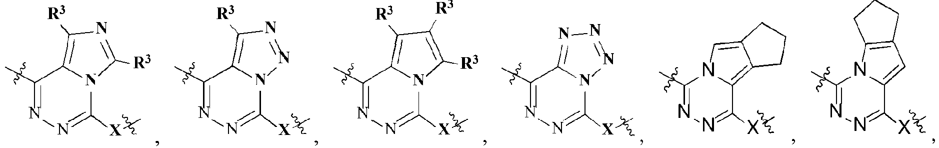 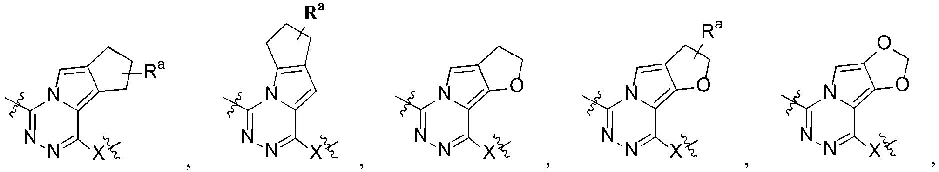 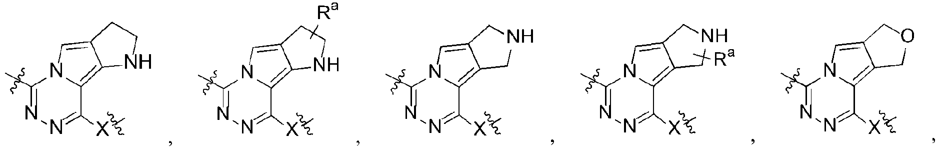 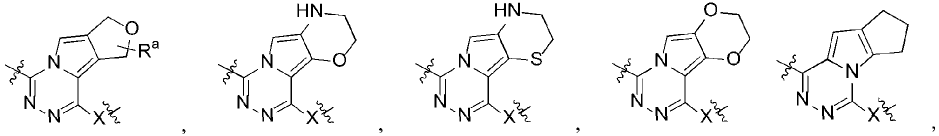 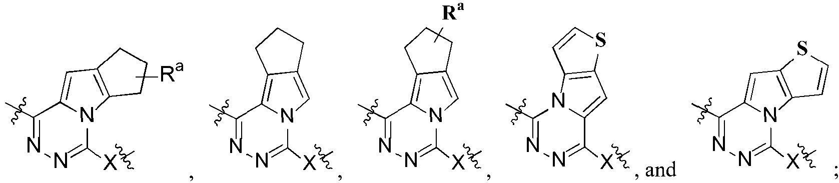 further preferably, the structural unit 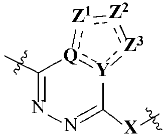 is selected from the group consisting of 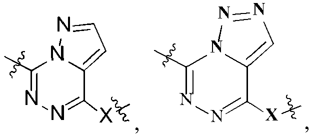 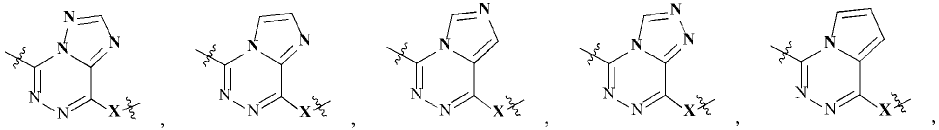 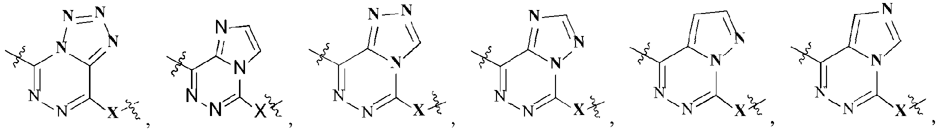 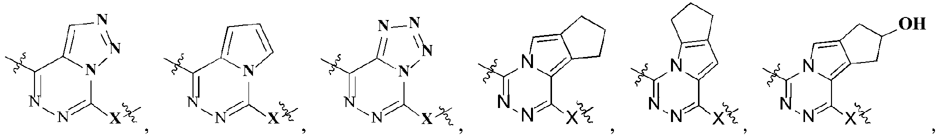 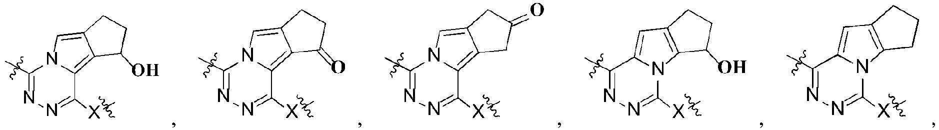 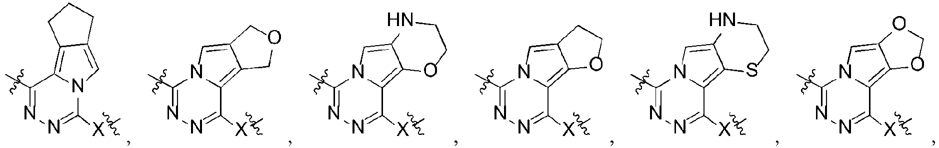 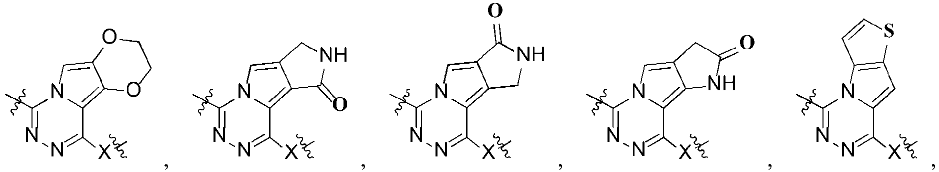 and 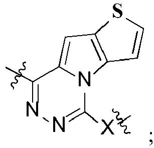 further preferably, the structural unit 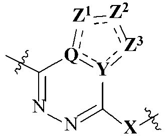 is selected from the group consisting of

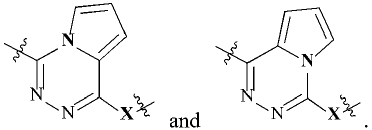

9. The compound of formula (I) or the pharmaceutically acceptable salt thereof according to claim 1, wherein X is selected from the group consisting of -N(R⁴)-, -O-, -S-, and -(C(R⁵)(R⁶))ₘ-.

10. The compound of formula (I) or the pharmaceutically acceptable salt thereof according to claim 1, wherein R¹ is selected from the group consisting of C₃₋₆ cycloalkyl, 3- to 6-membered heterocycloalkyl, phenyl, and 5- to 6-membered heteroaryl, the C₃₋₆ cycloalkyl, 3- to 6-membered heterocycloalkyl, phenyl, or 5- to 6-membered heteroaryl being optionally and independently substituted with 1, 2, or 3 R^{a}; preferably, R¹ is selected from the group consisting of phenyl, pyrrolyl, pyrazolyl, imidazolyl, oxazolyl, isoxazolyl, thiazole ring, isothiazolyl, triazolyl, furanyl, thienyl, pyridinyl, pyrazinyl, pyrimidinyl, and pyridazinyl, the phenyl, pyrrolyl, pyrazolyl, imidazolyl, oxazolyl, isoxazolyl, thiazole ring, isothiazolyl, triazolyl, furanyl, thienyl, pyridinyl, pyrazinyl, pyrimidinyl, or pyridazinyl being optionally and independently substituted with 1, 2, or 3 R^{a}; further preferably, R¹ is selected from phenyl, the phenyl being optionally and independently substituted with 1, 2, or 3 R^{a}.

11. The compound of formula (I) or the pharmaceutically acceptable salt thereof according to claim 8, wherein each R^{a} is independently selected from the group consisting of OH, CN, halogen, C₁₋₃ alkyl, C₁₋₃ alkoxy, C₂₋₄ alkenyl, C₂₋₄ alkynyl, and -(CH₂)ᵢC₂₋₄ alkynyl, the C₁₋₃ alkyl, C₁₋₃ alkoxy, C₂₋₄ alkenyl, C₂₋₄ alkynyl, or -(CH₂)ᵢC₂₋₄ alkynyl being optionally and independently substituted with 1, 2, or 3 halogens, OH, NH₂, or CN; preferably, each R^{a} is independently selected from the group consisting of F, Cl, =O, OH, trifluoromethyl, trifluoromethoxy, difluoromethoxy, CF₃CH₂-, CN,

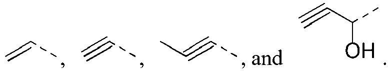

12. The compound of formula (I) or the pharmaceutically acceptable salt thereof according to claim 1, wherein R² is selected from the group consisting of C₁₋₄ alkyl, C₂₋₄ alkenyl, C₂₋₄ alkynyl, -(CH₂)₁C₃₋₆ cycloalkyl, -(CH₂)₁-(3- to 6-membered heterocycloalkyl), -(CH₂)ᵢ-phenyl, and -(CH₂)ᵢ-(5- to 6-membered heteroaryl), the C₁₋₄ alkyl, C₂₋₄ alkenyl, C₂₋₄ alkynyl, C₃₋₆ cycloalkyl, 3- to 6-membered heterocycloalkyl, phenyl, or 5- to 6-membered heteroaryl being optionally and independently substituted with 1, 2, or 3 R^{b}; preferably, R² is selected from the group consisting of methyl, ethyl, n-propyl, isopropyl, n-butyl, isobutyl, *sec*-butyl, *tert*-butyl, cyclopropyl, cyclobutyl, cyclopentyl, cyclohexyl, oxiranyl, thiiranyl, aziridinyl, oxetanyl, thietanyl, azetidinyl, pyrrolidinyl, pyrazolidinyl, imidazolidinyl, tetrahydrothienyl, tetrahydrofuranyl, tetrahydropyranyl, thiazolidinyl, isothiazolidinyl, oxazolidinyl, isoxazolidinyl, piperidinyl, piperazinyl, morpholinyl, phenyl, pyrrolyl, pyrazolyl, imidazolyl, oxazolyl, isoxazolyl, thiazole ring, isothiazolyl, triazolyl, furanyl, thienyl, pyridinyl, pyrazinyl, pyrimidinyl, and pyridazinyl, and R² is optionally and independently substituted with 1, 2, or 3 R^{b}; further preferably, R² is selected from piperidinyl, the piperidinyl being optionally and independently substituted with 1, 2, or 3 R^{b}.

13. The compound of formula (I) or the pharmaceutically acceptable salt thereof according to claim 10, wherein each R^{b} is independently selected from the group consisting of OH, CN, NH₂, halogen, =O, methyl, ethyl, isopropyl, methoxy, ethoxy, methylthio, ethylthio, -NHCH₃, -N(CH₃)₂, cyclopropyl, cyclobutyl, oxiranyl, thiiranyl, aziridinyl, oxetanyl, thietanyl, azetidinyl, -S(=O)₂-CH₃, -S(=O)₂-CH₂CH₃, -S(=O)₂-NH₂, -S(=O)₂-NH-CH₃, -S(=O)₂-NH-CH₂CH₃, -S(=O)₂-cyclopropyl, -S(=O)₂-NH-cyclopropyl, -S(=O)(NH)-CH₃, -S(=O)(NH)-CH₂CH₃, and -S(=O)(NH)-cyclopropyl, the methyl, ethyl, isopropyl, methoxy, ethoxy, methylthio, ethylthio, -NHCH₃, -N(CH₃)₂, -S(=O)₂-CH₃, -S(=O)₂-CH₂CH₃, -S(=O)₂-NH-CH₃, -S(=O)₂-NH-CH₂CH₃, -S(=O)(NH)-CH₃, or -S(=O)(NH)-CH₂CH₃ being optionally and independently substituted with 1, 2, or 3 halogens, OH, NH₂, or CN, and the cyclopropyl, cyclobutyl, oxiranyl, thiiranyl, aziridinyl, oxetanyl, thietanyl, azetidinyl, -S(=O)₂-cyclopropyl, -S(=O)₂-NH-cyclopropyl, or -S(=O)(NH)-cyclopropyl being optionally and independently substituted with 1, 2, or 3 R^{e}; preferably, each R^{b} is independently selected from the group consisting of F, Cl, Br, I, OH, CN, NH₂, methyl, trifluoromethyl, ethyl, 2-hydroxyethyl, -S(=O)₂-CH₃, -S(=O)₂-NH₂, -S(=O)₂-cyclopropyl, and -S(=O)(NH)-CH₃.

14. The compound of formula (I) or the pharmaceutically acceptable salt thereof according to claim 1, selected from the group consisting of a compound of formula (I-1a) and a compound of formula (I-1b), and pharmaceutically acceptable salts thereof: 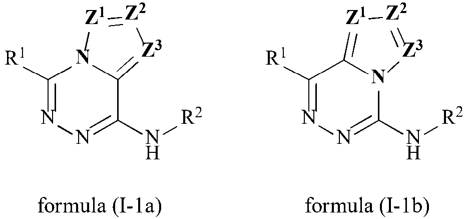 wherein
R¹, R², Z¹, Z², and Z³ are as defined in claim 1.

15. A compound of the following formula or a pharmaceutically acceptable salt thereof:

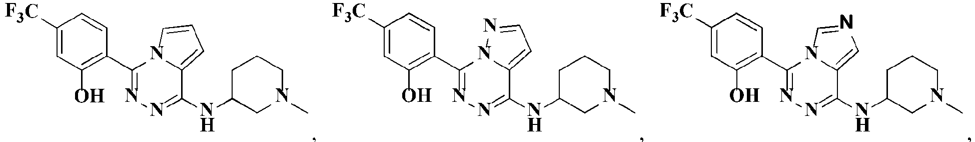

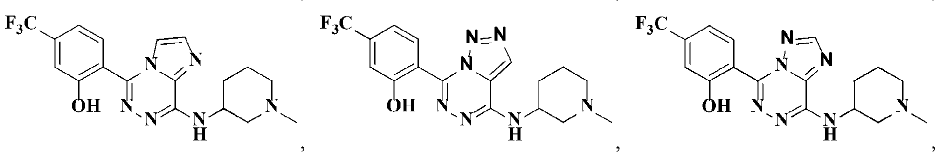

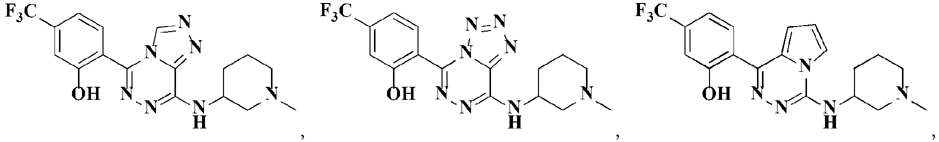

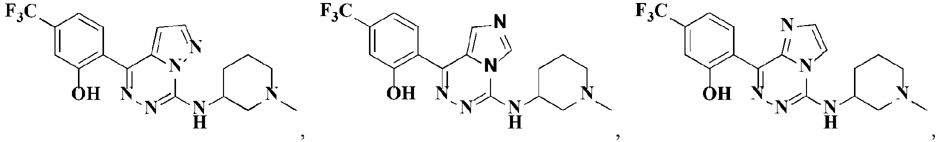

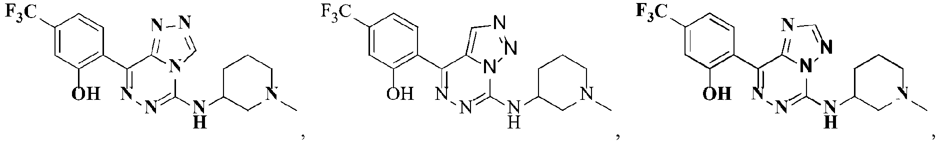

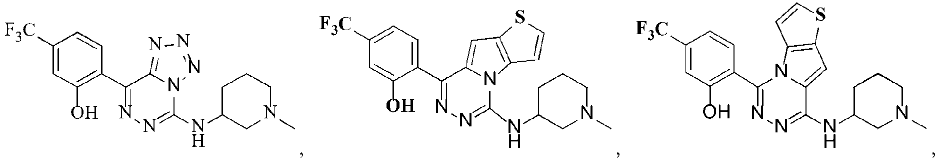

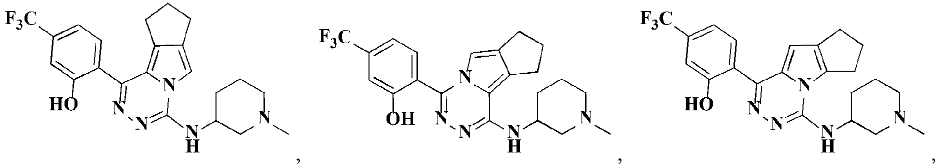

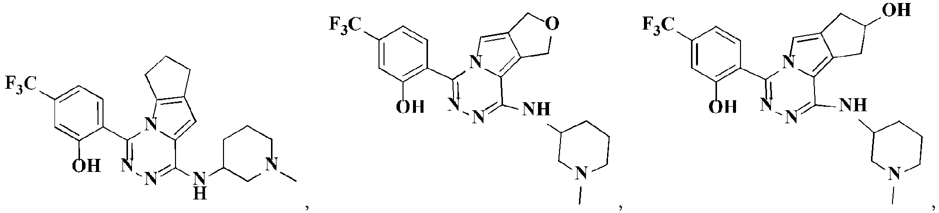

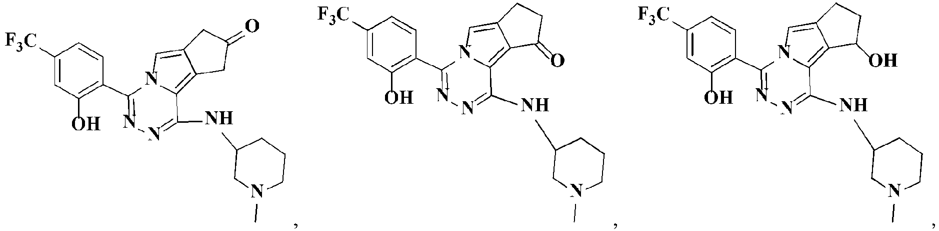

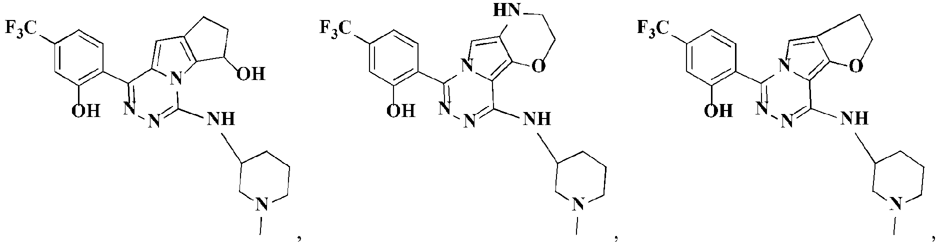

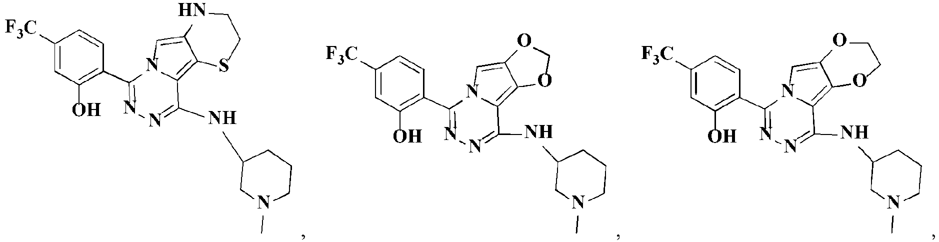

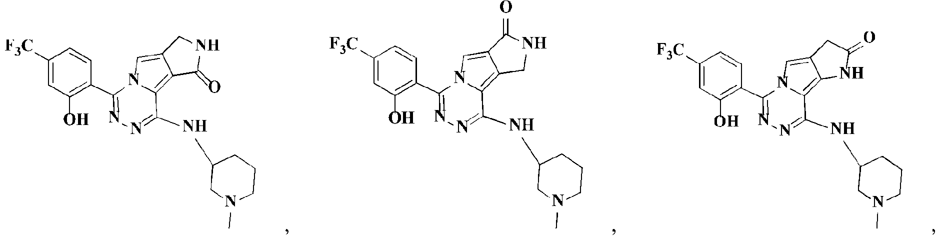

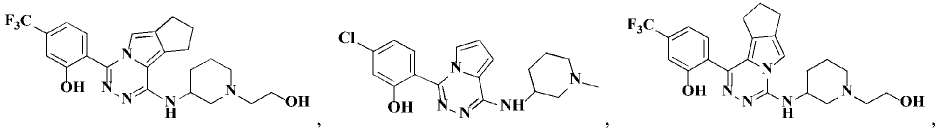

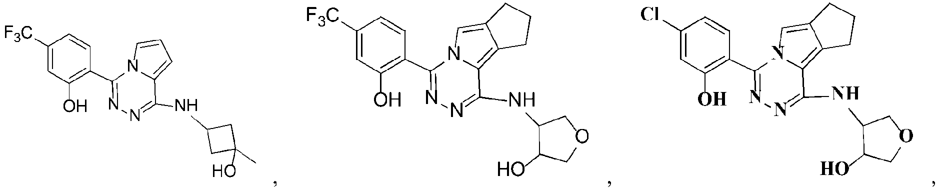

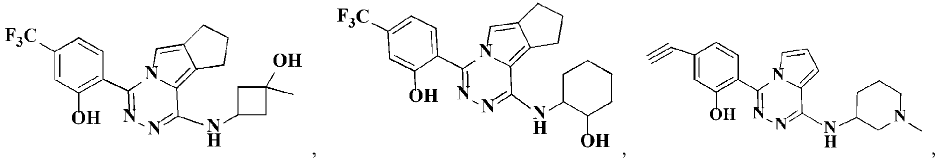

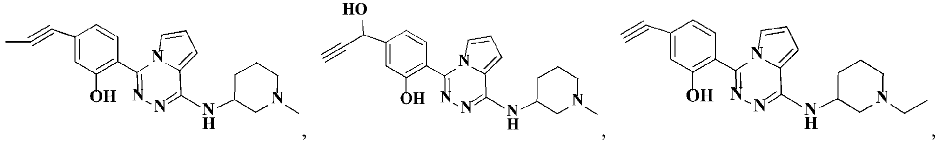

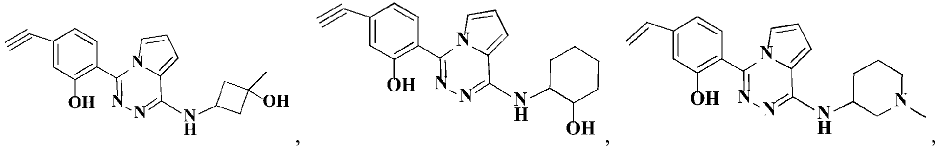

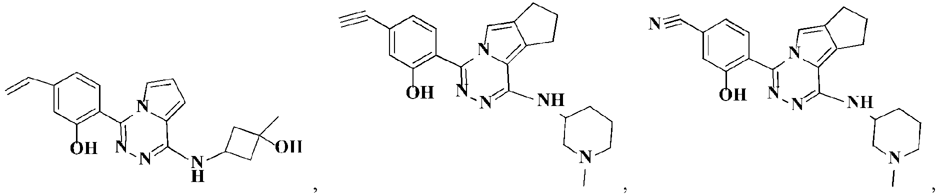

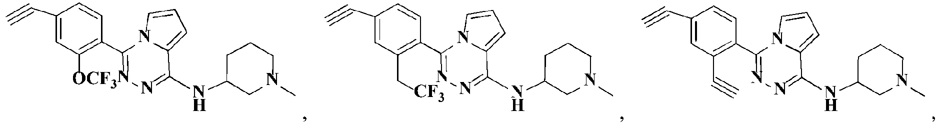

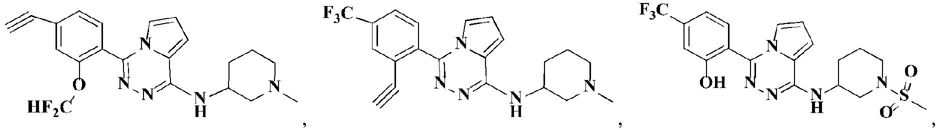

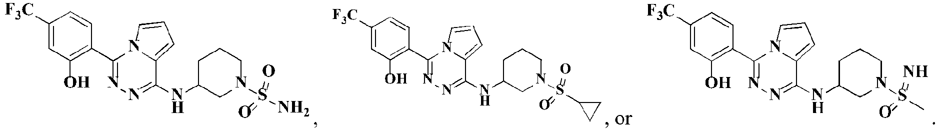

16. A pharmaceutical composition, comprising a therapeutically effective amount of the compound or the pharmaceutically acceptable salt thereof according to claims 1-15.

17. Use of the compound or the pharmaceutically acceptable salt thereof according to claims 1-15 or the pharmaceutical composition according to claim 16 for preparing a medicament for treating or preventing an NLRP3 inflammasome-mediated disease.
